(19) Europäisches Patentamt
European Patent Office
Office européen des brevets

(11) EP 4 196 792 B1

(12) **EUROPEAN PATENT SPECIFICATION**

(45) Date of publication and mention of the grant of the patent:
**01.07.2026 Bulletin 2026/27**

(21) Application number: **21766420.0**

(22) Date of filing: **17.08.2021**

(51) International Patent Classification (IPC):
***G01N 33/575*** *(2026.01)*

(52) Cooperative Patent Classification (CPC):
**G01N 33/57557; C12Q 1/6886;** C12Q 2600/106; C12Q 2600/156; C12Q 2600/158; G01N 2800/52

(86) International application number:
**PCT/EP2021/072866**

(87) International publication number:
**WO 2022/038158 (24.02.2022 Gazette 2022/08)**

(54) **SELECTING A PATIENT HAVING AN ELEVATED NECTIN-4**

AUSWÄHLEN EINES PATIENTEN MIT ERHÖHTEM NECTIN-4

SÉLECTIONNER UN PATIENT AYANT UN TAUX ÉLEVÉ DE NECTINE-4

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2020 US 202063066434 P**
**06.01.2021 US 202163134202 P**
**07.04.2021 US 202163171654 P**

(43) Date of publication of application:
**21.06.2023 Bulletin 2023/25**

(60) Divisional application:
**26180678.0**

(73) Proprietor: **BicycleTX Limited**
**Cambridge, Cambridgeshire CB21 6GS (GB)**

(72) Inventors:
- **BLAKEMORE, Stephen J.**
  **Cambridge CB21 6GS (GB)**
- **GELB, Tara**
  **Cambridge CB21 6GS (GB)**
- **SANTOS, Sean M.**
  **Cambridge CB21 6GS (GB)**
- **COHEN, Heather B.**
  **Cambridge CB21 6GS (GB)**
- **CAMPBELL, Carly**
  **Cambridge CB21 6GS (GB)**

(74) Representative: **J A Kemp LLP**
**80 Turnmill Street**
**London EC1M 5QU (GB)**

(56) References cited:
**US-A1- 2019 389 906**

- **SATOSHI NISHIWADA ET AL: "Nectin-4 expression contributes to tumor proliferation, angiogenesis and patient prognosis in human pancreatic cancer", JOURNAL OF EXPERIMENTAL & CLINICAL CANCER RESEARCH, BIOMED CENTRAL LTD, LONDON UK, vol. 34, no. 1, 28 March 2015 (2015-03-28), pages 30, XP021215383, ISSN: 1756-9966, DOI: 10.1186/S13046-015-0144-7**
- **PAVLOVA N N ET AL: "A role for PVRL4-driven cell-cell interactions in tumorigenesis", ELIFE, ELIFE SCIENCES PUBLICATIONS LTD, GB, vol. 2, 30 April 2013 (2013-04-30), pages e00358 - 1, XP002754175, ISSN: 2050-084X, [retrieved on 20130430], DOI: 10.7554/ELIFE.00358**
- **SHAO XIN ET AL: "Copy number variation is highly correlated with differential gene expression: a pancancer study", BMC MEDICAL GENETICS, vol. 20, no. 1, 1 December 2019 (2019-12-01), XP055850764, Retrieved from the Internet <URL:https://bmcmedgenet.biomedcentral.com/track/pdf/10.1186/s12881-019-0909-5.pdf> DOI: 10.1186/s12881-019-0909-5**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **GELB T ET AL: "Abstract 391: Molecular-based enrichment strategy for Nectin-4 targeted Bicycle toxin conjugate BT8009 | Cancer Research", 1 July 2021 (2021-07-01), XP055850795, Retrieved from the Internet <URL:https://cancerres.aacrjournals.org/content/81/13_Supplement/391> [retrieved on 20211013]**


Remarks:

The complete document including Reference Table(s) and the Sequence Listing(s) can be downloaded from the EPO website

## Description

### TECHNICAL FIELD OF THE INVENTION

The present invention relates to methods of identifying or selecting patients having an elevated Nectin-4 protein and/or RNA expression level in a tumor tissue and to Bicycle toxin conjugates or Bicycle tumor targeted immune agonists (TICAs) specific for Nectin-4, or pharmaceutically acceptable salts thereof, or pharmaceutical compositions thereof for use in methods of treating a cancer in a patient having an elevated Nectin-4 protein and/or RNA expression level in tumor tissue.

### BACKGROUND OF THE INVENTION

The phenomena of the presence of tumor gene amplifications being associated with the up regulation of the associated encoded protein and the use of tests that determine the presence or absence of gene amplifications as a predictor of response to therapy is well established in the treatment of solid tumors. Amplifications of ERBB2 (HER2), MET and FGFR have all been demonstrated to be predictive of response to therapeutics targeting these specific proteins {Arnoud et al, Clinical Cancer Research 2007; Cui JJ, J of Med Chem, 2014; Pearson et al, Cancer Discovery 2016}. Over-expression of Nectin-4 has been reported in multiple tumor types {Challita-Eid et al, Cancer Research, 2016} and has been associated with underlying Nectin-4 gene amplification in breast cancer {N Pavlova et al, Elife, 2013}. Analyses of TCGA data indicated that amplification of Nectin-4 occurs in additional indications and therefore may represent a way of identifying tumors with high Nectin-4 expression across indications that may respond to Nectin-4 targeted therapeutics such as BT8009.

Cyclic peptides are able to bind with high affinity and target specificity to protein targets and hence are an attractive molecule class for the development of therapeutics. In fact, several cyclic peptides are already successfully used in the clinic, as for example the antibacterial peptide vancomycin, the immunosuppressant drug cyclosporine or the anti-cancer drug octreotide (Driggers et al. (2008), Nat Rev Drug Discov 7 (7), 608-24). Good binding properties result from a relatively large interaction surface formed between the peptide and the target as well as the reduced conformational flexibility of the cyclic structures. Typically, macrocycles bind to surfaces of several hundred square angstrom, as for example the cyclic peptide CXCR4 antagonist CVX15 (400 Å$^2$; Wu et al. (2007), Science 330, 1066-71), a cyclic peptide with the Arg-Gly-Asp motif binding to integrin $\alpha$Vb3 (355 Å$^2$) (Xiong et al. (2002), Science 296 (5565), 151-5) or the cyclic peptide inhibitor upain-1 binding to urokinase-type plasminogen activator (603 Å$^2$; Zhao et al. (2007), J Struct Biol 160 (1), 1-10).

Due to their cyclic configuration, peptide macrocycles are less flexible than linear peptides, leading to a smaller loss of entropy upon binding to targets and resulting in a higher binding affinity. The reduced flexibility also leads to locking target-specific conformations, increasing binding specificity compared to linear peptides. This effect has been exemplified by a potent and selective inhibitor of matrix metalloproteinase 8, (MMP-8) which lost its selectivity over other MMPs when its ring was opened (Cherney et al. (1998), J Med Chem 41 (11), 1749-51). The favorable binding properties achieved through macrocyclization are even more pronounced in multicyclic peptides having more than one peptide ring as for example in vancomycin, nisin and actinomycin.

Different research teams have previously tethered polypeptides with cysteine residues to a synthetic molecular structure (Kemp and McNamara (1985), J. Org. Chem; Timmerman et al. (2005), ChemBioChem). Meloen and co-workers had used tris(bromomethyl)benzene and related molecules for rapid and quantitative cyclisation of multiple peptide loops onto synthetic scaffolds for structural mimicry of protein surfaces (Timmerman et al. (2005), ChemBioChem). Methods for the generation of candidate drug compounds wherein said compounds are generated by linking cysteine containing polypeptides to a molecular scaffold as for example TATA (1,1',1"-(1,3,5-triazinane-1,3,5-triyl)triprop-2-en-1-one, Heinis et al. Angew Chem, Int Ed. 2014; 53:1602-1606).

Phage display-based combinatorial approaches have been developed to generate and screen large libraries of bicyclic peptides to targets of interest (Heinis et al. (2009), Nat Chem Biol 5 (7), 502-7 and WO 2009/098450). Briefly, combinatorial libraries of linear peptides containing three cysteine residues and two regions of six random amino acids (Cys-$(Xaa)_6$-Cys-$(Xaa)_6$-Cys) were displayed on phage and cyclised by covalently linking the cysteine side chains to a small molecule scaffold.

US 2019/0389906 relates to peptide ligands specific for Nectin-4 which comprise polypeptides which are covalently bound to molecular scaffolds such that two or more peptide loops are subtended between attachment points to the scaffold, and the use of drug conjugates of said polypeptides in preventing, suppressing or treating a disease or disorder mediated by Nectin-4, such as cancer.

Nishiwada et al. 2015. Journal of Experimental & Clinical Cancer Research 34:30 mentions that Nectin-4 expression contributes to tumor proliferation, angiogenesis and patient prognosis in human pancreatic cancer.

## BRIEF DESCRIPTION OF THE DRAWINGS

**FIG. 1** shows Nectin-4 gains and amplifications are statistically significantly associated with increases in gene expression in various cancers such as: Sarcoma, Uterine Corpus, Endometrial Carcinoma, Pancreas, Lung adeno, Breast, Lung squamous, Head & Neck, Cervical, and Bladder.

**FIG. 2A** shows genes flanking Nectin-4 on the MSK Impact Panel and the Foundation Medicine Panel. **FIG. 2B** shows the gene name, description, genomic location and distance to Nectin-4 for SDHC and DDR2.

**FIG. 3A** shows the strong association between Nectin-4 DNA copy number and SDHC DNA copy number (CN) in publicly available TCGA data. The association between SDHC CN status and Nectin-4 CN status is shown for individual tumors, separately by tumor cohort.

**FIG. 3B** shows the strong association between Nectin-4 DNA copy number and DDR2 DNA copy number in publicly available TCGA data. The association between DDR2 CN status and Nectin-4 CN status is shown for individual tumors, separately by tumor cohort.

**FIG. 4** shows the identified candidate surrogate marker for Nectin-4 protein expression = SDHC (or DDR2) amplification. SDHC amplifications can be used as a surrogate marker to identify a high Nectin-4 expressing tumor which is useful for predicting a response to a Nectin-4 Bicycle Toxin Conjugate (BTC).

**FIG. 5A** shows the analysis of Nectin-4 DNA copy number versus protein expression in the membrane in TNBC samples. **FIG. 5B** shows the analysis of Nectin-4 DNA copy number versus protein expression in the cytoplasm in TNBC samples. **FIG. 5C** shows the analysis of Nectin-4 DNA copy number versus protein expression in the membrane and cytoplasm in TNBC samples. Note that for a Log2(CN ratio) ≥ about 0.6, all tumor cores have a combined H-score ≥ 100. **FIG. 5D** shows the 100% positive predictive value when using CN ≥ 3 to determine tumor membrane + cytoplasmic H-score ≥ 100. The X-axis in each of **FIG. 5A, FIG. 5B,** and **FIG. 5C** is Nectin-4 Log2(CN ratio) and the dashed lines in the two plots show quartiles for H-scores and Nectin-4 DNA copy number.

**FIG. 6A** shows that Nectin-4 DNA copy number gains are associated with rightward shift in combined membrane and cytoplasmic H-scores. **FIG. 6B** shows the relationship between H-score cutoff and copy number calls as determined by the GATK pipeline (https://gatk.broadinstitute.org/hc/en-us). (H-score boundaries are inclusive).

**FIG. 7A** (membrane + cytoplasmic H-scores), **FIG. 7B** (membrane H-score), and **FIG. 7C** (cytoplasmic H-score) show the number of samples that meet an H-score cutoff as Nectin-4 log2 copy number cutoffs increase. Both H-score and log2 CN cutoffs are inclusive. **FIG. 7D** shows the % of TNBC samples that pass distinct membrane + cytoplasmic H-score cutoffs (x-axis) in the context of different Nectin-4 log2 CN ratio thresholds.

**FIG. 8A** shows that SDHC can be used as a surrogate for Nectin-4 DNA copy number (and potentially Nectin-4 protein expression).

**FIG. 8B** shows that DDR2 can be used as a surrogate for Nectin-4 DNA copy number (and potentially Nectin-4 protein expression).

**FIG. 9** depicts a model of the effect of using a Nectin-4 CN cutoff of log2 CN ratio ≥ 0.5 to enrich for a patient population with higher membrane + cytoplasmic H-scores. Methods: resampling of the TNBC TMA membrane + cytoplasmic H-scores was performed without replacement using either no log2 CN ratio cutoff, log2 CN ratio ≥ 0, or log2 CN ratio ≥ 0.5, with an expected cohort size of 16, and 10,000 permutations. H-score bins are inclusive on the left and exclusive on the right, except the highest bin is inclusive of both sides.

**FIG. 10A** depicts that Nectin-4-expressing tumor cells and CD137-expressing immune cells co-localize in human cancers. Transcript co-expression across tumor types in TCGA.

**FIG. 10B** depicts MultiOmyx™ imaging that allows for simultaneous interrogation of immune infiltrate and spatial proteomic profiling within human tumors. A single ROI from a representative HNSCC sample is shown. T cells (CD3+, red), macrophages (CD68+, blue), NK cells (CD56+, green), and tumor cells (PanCK+, cyan) detected throughout tumor (top left). Examples of CD137+ CD4+ and CD137+ CD8+ T cells are shown and represented by white and gray arrows respectively (top right). Co-expression of Nectin-4 (red) and PanCK (blue) on tumor cells (bottom left). Tumor and stroma regions were identified using a PanCK and DAPI mask respectively (bottom right, in red and blue respectively).

**FIG. 10C** depicts tumor Nectin-4 expression where total Nectin-4+PanCK+ cells are normalized to total cells (left) and CD137+ immune infiltrate where total CD137+ cells detected are normalized to total cells (right). Within each box, the horizontal line represents the mean of 5 samples shown.

**FIG. 10D** depicts subset analysis of CD137+ immune infiltrate within stroma (left) and tumor (right) regions across samples and included T cells (CD3+CD4+ and CD3+CD8+), macrophages (CD68+), NK cells (CD56+), and B cells (CD19+). Data are total cells per phenotype normalized to total CD137+ cells detected across samples within each indication. Within each box, the horizontal line represents the mean of 5 samples shown.

## SUMMARY OF THE INVENTION

As described herein, the inventors have discovered a correlation between levels of Nectin-4 protein expression in a diseased tissue and Nectin-4 DNA copy numbers. Nectin-4 is overexpressed in many difficult to treat tumors, such as NSCLC, TNBC, pancreatic, ovarian, gastric/upper GI, and urothelial cancers. Nectin-4 is expressed at relatively low levels in normal adult tissues. Without wishing to be bound by any particular theory or mechanism, a tumor having an elevated Nectin-4 protein expression and/or an elevated Nectin-4 DNA copy number in a diseased tissue may be more likely to benefit from treatment with a Bicycle toxin conjugate specific for Nectin-4. In some embodiments the Bicycle toxin conjugate specific for Nectin-4 is BT8009.

Further, without wishing to be bound by any particular theory or mechanism, a tumor having an elevated Nectin-4 RNA expression and/or an elevated Nectin-4 DNA copy number in a diseased tissue may be more likely to benefit from treatment with a Bicycle toxin conjugate specific for Nectin-4. In some embodiments the Bicycle toxin conjugate specific for Nectin-4 is BT8009.

Additionally, without wishing to be bound by any particular theory or mechanism, a tumor having an elevated Nectin-4 protein expression and/or an elevated Nectin-4 RNA expression and/or an elevated Nectin-4 DNA copy number in a diseased tissue co-localized with CD137-expressing immune cells may be more likely to benefit from treatment with a Bicycle tumor targeted immune agonist specific for Nectin-4. In some embodiments the Bicycle toxin conjugate specific for Nectin-4 is BT7480.

Nectin-4 is also known by the following aliases, each of which is equivalent to Nectin-4: Nectin Cell Adhesion Molecule 4, Nectin-4, LNIR, PRR4, Poliovirus Receptor-Related Protein 4, Poliovirus Receptor-Related 4, Ig Superfamily Receptor LNIR, PVRL4, Nectin 4, NECTIN4, and EDSS1.

In one aspect, the invention provides a method of identifying or selecting a patient having an elevated Nectin-4 protein and/or RNA expression level in a tumor tissue, comprising measuring the Succinate Dehydrogenase Complex Subunit C (SDHC) DNA copy number in tumor tissue or in circulating tumor DNA (ctDNA) of a patient, and selecting a patient having an elevated SDHC DNA copy number.

In another aspect, the present invention provides a bicyclic peptide complex toxin conjugate or heterotandem bicyclic peptide complex specific for Nectin-4, or a pharmaceutically acceptable salt thereof, or a pharmaceutical composition thereof, for use in a method of treating a cancer in a patient having an elevated Nectin-4 protein and/or RNA expression level in tumor tissue, said method comprising selecting a patient having an SDHC Log2(copy number ratio) of 0.1 or more, 0.2 or more, 0.3 or more, 0.4 or more, 0.5 or more, 0.6 or more, 0.7 or more, 0.8 or more, 0.9 or more, or 1.0 or more in tumor tissue or in circulating tumor DNA (ctDNA) as determined by Next-generation sequencing (NGS) technologies or an array-based sequence capture, and administering to the patient a bicyclic peptide complex toxin conjugate or heterotandem bicyclic peptide complex specific for Nectin-4, the pharmaceutically acceptable salt thereof, or the pharmaceutical composition thereof.

## DETAILED DESCRIPTION OF CERTAIN EMBODIMENTS

### 1. General Description of Certain Embodiments of the Invention:

Recently it has become common practice either at initial disease diagnosis or first relapse for solid tumor patients to submit tumor tissue for genetic and genomic characterization using gene panels developed locally at academic research centers {Cheng et al, J Molecular Diagnostics, 2015; Rothwell et al, Nature Medicine 2019} or commercially {Miller et al, JCO, 2013; Lanman et al, PLoS One 2015}. These panels are typically configured to identify specific somatic mutations, gene fusions and gene amplification events. For panels that contain Nectin-4, patients can be identified with amplifications of Nectin-4 that may indicate that their tumors are predisposed to express high levels of Nectin-4 and therefore may be likely to respond to BT8009. In addition, some panels include genes which are sufficiently close to Nectin-4 on chromosome 1 that they are typically amplified together and therefore detection of SDHC (or DDR2) amplifications may act as a surrogate of Nectin-4 amplification regardless of whether Nectin-4 is included in the panel. Therefore the intention of the invention here is to use patient's tumor molecular genetic data on SDHC status to enrich for patients with high Nectin-4 expression determined by IHC who may respond to BT8009.

SDHC is also known by the following aliases, each of which is equivalent to SDHC: Succinate Dehydrogenase Complex Subunit C; CYB560; Succinate Dehydrogenase Complex, Subunit C, Integral Membrane Protein, 15kD; Succinate Dehydrogenase Cytochrome B560 Subunit, Mitochondrial; Succinate-Ubiquinone Oxidoreductase Cytochrome B Large Subunit; Succinate Dehydrogenase Cytochrome B; Large Subunit Of Cytochrome B; CYBL; SDH3; Succinate Dehydrogenase Complex, Subunit C, Integral Membrane Protein, 15kDa; Succinate Dehydrogenase Complex Subunit C Integral Membrane Protein 15kDa; Succinate Dehydrogenase Integral Membrane Subunit; Succinate Dehydrogenase Cytochrome B560 Subunit; Cytochrome B Large Subunit Of Complex II; Integral Membrane Protein CII-3b; Integral Membrane Protein CII-3; QPs-1; PGL3; QPS1; SDHC; CybL; and QPs1.

DDR2 is also known by the following aliases, each of which is equivalent to DDR2: Discoidin Domain Receptor Tyrosine Kinase 2; TKT; Discoidin Domain-Containing Receptor Tyrosine Kinase 2; Discoidin Domain Receptor Family, Member 2; Discoidin Domain-Containing Receptor 2; Receptor Protein-Tyrosine Kinase TKT; CD167 Antigen-Like Family Member B; Tyrosine-Protein Kinase TYRO10; Discoidin Domain Receptor 2; EC 2.7.10.1; NTRKR3; TYRO10; Neurotrophic Tyrosine Kinase, Receptor-Related 3; Neurotrophic Tyrosine Kinase Receptor Related 3; Cell Migration-Inducing Protein 20; Migration-Inducing Gene 16 Protein; Hydroxyaryl-Protein Kinase; CD167b Antigen; EC 2.7.10; MIG20a; WRCN; and DDR2.

Next-generation sequencing (NGS) technologies, also known as high-throughput sequencing technologies, allow for sequencing of DNA and RNA much more quickly and cheaply than the previously used Sanger sequencing. An exemplary NGS technology is Illumina (Solexa) sequencing, which simultaneously identifies DNA bases, as each base emits a unique fluorescent signal, and adds them to a nucleic acid chain. Another exemplary NGS technology is Roche 454 sequencing, which is based on pyrosequencing, a technique which detects pyrophosphate release, again using fluorescence, after nucleotides are incorporated by polymerase to a new strand of DNA. A further exemplary NGS technology is Ion Torrent: Proton / PGM sequencing, which measures the direct release of H+ (protons) from the incorporation of individual bases by DNA polymerase.

Nectin-4, SDHC, and DDR2 DNA copy numbers can be measured by a NGS technology as described herein. Nectin-4, SDHC, and/or DDR2 DNA copy numbers may be measured by sequencing the whole genome. Nectin-4, SDHC, and/or DDR2 DNA copy numbers may be measured by sequencing the whole exome.

Array-based sequence capture can also be used to sequence large targeted DNA regions in a less laborious and time-consuming approach. An array-based approach may be employed to measure Nectin-4, SDHC, and/or DDR2 DNA copy numbers. Nectin-4, SDHC, and/or DDR2 DNA copy numbers may be measured using an array-based approach. The array-based approach may be sequence based. The array-based approach may be non-sequence based.

Circulating tumor DNA (ctDNA) is found in the bloodstream and refers to DNA that comes from cancerous cells and tumors. Most DNA is inside a cell's nucleus. As a tumor grows, cells die and are replaced by new ones. The dead cells get broken down and their contents, including DNA, are released into the bloodstream. ctDNA are small pieces of DNA, usually comprising fewer than 200 building blocks (nucleotides) in length. Detection of ctDNA can be useful for detecting and diagnosing a tumor; guiding tumor-specific treatment, monitoring treatment, and monitoring periods with no symptoms (remission of cancer).

Nectin-4 DNA amplifications in tumor tissues have been measured by whole exome sequencing. It has been found that the Nectin-4 DNA amplifications are associated with higher levels of Nectin-4 protein expression, and correspondingly may be indicative of tumors having an elevated expression of Nectin-4. Without wishing to be bound by any particular theory or mechanism, the inventors have determined that a tumor having an elevated Nectin-4 DNA copy number in a diseased tissue may be more likely to benefit from a treatment with a Bicycle toxin conjugate specific for Nectin-4.

Additionally, without wishing to be bound by any particular theory or mechanism, the inventors have determined that a tumor having an elevated Nectin-4 DNA copy number in a diseased tissue may be more likely to benefit from a treatment with a Bicycle tumor targeted immune agonist specific for Nectin-4.

SDHC or DDR2 DNA amplifications in tumor tissues have been measured by whole exome sequencing. It has been found that the SDHC or DDR2 DNA amplifications may act as a surrogate of Nectin-4 expression and correspondingly may be indicative of tumors having an elevated expression of Nectin-4. Without wishing to be bound by any particular theory or mechanism, a tumor having an elevated SDHC or DDR2 DNA copy number in a diseased tissue may be more likely to benefit from treatment with a Bicycle toxin conjugate specific for Nectin-4. An exemplary Bicycle toxin conjugate specific for Nectin-4 is BT8009.

Additionally, without wishing to be bound by any particular theory or mechanism, a tumor having an elevated SDHC or DDR2 DNA copy number in a diseased tissue may be more likely to benefit from treatment with a Bicycle tumor targeted immune agonist specific for Nectin-4. An exemplary Bicycle tumor targeted immune agonists tumor targeted immune agonist specific for Nectin-4 is BT7480.

For each tissue microarray (TMA) core analyzed, the membrane H-score (maximum of 300) is added to the cytoplasmic H-score (maximum of 300), resulting in a maximum combined total H-score of 600. This approach differs from the standard approach of calculating an H-score for the whole sample (TMA core) that includes both the membrane and cytoplasmic staining resulting in a maximum H-score of 300. As the H-score is calculated by % positivity X intensity, the cytoplasm is weighted higher than the membrane given its larger surface area using the standard approach. Without being bound by any particular theory, one benefit of addition of the membrane and cytoplasmic H-scores is that this gives equal weight to the membrane and cytoplasmic staining (even though the membrane surface area is smaller than cytoplasmic surface area) which provides a more relevant score for Nectin-4 BTCs such as BT8009 which are expected to bind membrane Nectin-4. **FIG. 5A** shows the analysis comparing membrane H-scores in isolation versus Nectin-4 DNA copy number and **FIG. 5B** shows the analysis for cytoplasmic H-scores in isolation versus Nectin-4 DNA copy number. **FIG. 5C** shows the analysis of Nectin-4 DNA copy number versus protein expression in the membrane and cytoplasm in TNBC

samples. **FIG. 5D** shows the 100% positive predictive value when using CN ≥ 3 to determine tumor membrane + cytoplasmic H-score ≥ 100. As depicted in **FIG. 5D,** utilizing a cutoff of CN ≥ 3 gives the following values for sensitivity, specificity, positive predictive value, and negative predictive value:

- **Sensitivity = 28.21%** (22/78)
- **Specificity = 100%** (22/22)
- **Positive Predictive Value = 100%** (22/22)
- **Negative Predictive Value = 28.21%** (22/78)

Additionally, **FIG. 7B** (membrane H-scores in isolation) and **FIG. 7C** (cytoplasmic H-scores in isolation) show the number of samples that meet an H-score cutoff as Nectin-4 log2 copy number cutoffs increase.

The invention provides a method of identifying or selecting a patient having an elevated Nectin-4 protein level in a diseased tissue, comprising measuring SDHC DNA copy number in a tumor tissue or in ctDNA of a patient, and selecting a patient having an elevated SDHC DNA copy number. In some embodiments, the methods of the invention further comprise measuring the Nectin-4 DNA copy number in tumor tissue or in ctDNA and selecting a patient having an elevated SDHC DNA copy number and an elevated Nectin-4 DNA copy number. In some embodiments, the methods of the invention further comprise measuring the Nectin-4 DNA copy number in tumor tissue or in ctDNA and selecting a patient having an elevated SDHC DNA copy number and an elevated DDR2 DNA copy number.

In some embodiments an elevated level of SDHC DNA copy number means the copy number is 2 or more. In some embodiments an elevated level of SDHC DNA copy number means the copy number is 3 or more. In some embodiments an elevated level of SDHC DNA copy number means the copy number is 4 or more. In some embodiments an elevated level of SDHC DNA copy number means the copy number is 5 or more. In some embodiments an elevated level of SDHC DNA copy number means the copy number is 6 or more. In some embodiments an elevated level of SDHC DNA copy number means the copy number is 7 or more. In some embodiments an elevated level of SDHC DNA copy number means the copy number is 8 or more. In some embodiments an elevated level of SDHC DNA copy number means the copy number is 9 or more. In some embodiments an elevated level of SDHC DNA copy number means the copy number is 10 or more.

In some embodiments a SDHC DNA copy number of 2 or more correlates to an H-score of 100 or more in a SDHC IHC staining assay. In some embodiments a SDHC DNA copy number of 3 or more correlates to an H-score of 100 or more in a SDHC IHC staining assay. In some embodiments a SDHC DNA copy number of 4 or more correlates to an H-score of 100 or more in a SDHC IHC staining assay. In some embodiments a SDHC DNA copy number of 5 or more correlates to an H-score of 100 or more in a SDHC IHC staining assay. In some embodiments a SDHC DNA copy number of 6 or more correlates to an H-score of 100 or more in a SDHC IHC staining assay. In some embodiments a SDHC DNA copy number of 7 or more correlates to an H-score of 100 or more in a SDHC IHC staining assay. In some embodiments a SDHC DNA copy number of 8 or more correlates to an H-score of 100 or more in a SDHC IHC staining assay. In some embodiments a SDHC DNA copy number of 9 or more correlates to an H-score of 100 or more in a SDHC IHC staining assay. In some embodiments a SDHC DNA copy number of 10 or more correlates to an H-score of 100 or more in a SDHC IHC staining assay.

In some embodiments an elevated level of Nectin-4 DNA copy number means the copy number is 2 or more. In some embodiments an elevated level of Nectin-4 DNA copy number means the copy number is 3 or more. In some embodiments an elevated level of Nectin-4 DNA copy number means the copy number is 4 or more. In some embodiments an elevated level of Nectin-4 DNA copy number means the copy number is 5 or more. In some embodiments an elevated level of Nectin-4 DNA copy number means the copy number is 6 or more. In some embodiments an elevated level of Nectin-4 DNA copy number means the copy number is 7 or more. In some embodiments an elevated level of Nectin-4 DNA copy number means the copy number is 8 or more. In some embodiments an elevated level of Nectin-4 DNA copy number means the copy number is 9 or more. In some embodiments an elevated level of Nectin-4 DNA copy number means the copy number is 10 or more.

In some embodiments a Nectin-4 DNA copy number of 2 or more correlates to an H-score of 100 or more in a Nectin-4 IHC staining assay. In some embodiments a Nectin-4 DNA copy number of 3 or more correlates to an H-score of 100 or more in a Nectin-4 IHC staining assay. In some embodiments a Nectin-4 DNA copy number of 4 or more correlates to an H-score of 100 or more in a Nectin-4 IHC staining assay. In some embodiments a Nectin-4 DNA copy number of 5 or more correlates to an H-score of 100 or more in a Nectin-4 IHC staining assay. In some embodiments a Nectin-4 DNA copy number of 6 or more correlates to an H-score of 100 or more in a Nectin-4 IHC staining assay. In some embodiments a Nectin-4 DNA copy number of 7 or more correlates to an H-score of 100 or more in a Nectin-4 IHC staining assay. In some embodiments a Nectin-4 DNA copy number of 8 or more correlates to an H-score of 100 or more in a Nectin-4 IHC staining assay. In some embodiments a Nectin-4 DNA copy number of 9 or more correlates to an H-score of 100 or more in a Nectin-4 IHC staining assay. In some embodiments a Nectin-4 DNA copy number of 10 or more correlates to an H-score of 100 or more in a Nectin-4 IHC staining assay.

In some embodiments an elevated level of DDR2 DNA copy number means the copy number is 2 or more. In some embodiments an elevated level of DDR2 DNA copy number means the copy number is 3 or more. In some embodiments an elevated level of DDR2 DNA copy number means the copy number is 4 or more. In some embodiments an elevated level of DDR2 DNA copy number means the copy number is 5 or more. In some embodiments an elevated level of DDR2 DNA copy number means the copy number is 6 or more. In some embodiments an elevated level of DDR2 DNA copy number means the copy number is 7 or more. In some embodiments an elevated level of DDR2 DNA copy number means the copy number is 8 or more. In some embodiments an elevated level of DDR2 DNA copy number means the copy number is 9 or more. In some embodiments an elevated level of DDR2 DNA copy number means the copy number is 10 or more.

In some embodiments a DDR2 DNA copy number of 2 or more correlates to an H-score of 100 or more in a DDR2 IHC staining assay. In some embodiments a DDR2 DNA copy number of 3 or more correlates to an H-score of 100 or more in a DDR2 IHC staining assay. In some embodiments a DDR2 DNA copy number of 4 or more correlates to an H-score of 100 or more in a DDR2 IHC staining assay. In some embodiments a DDR2 DNA copy number of 5 or more correlates to an H-score of 100 or more in a DDR2 IHC staining assay. In some embodiments a DDR2 DNA copy number of 6 or more correlates to an H-score of 100 or more in a DDR2 IHC staining assay. In some embodiments a DDR2 DNA copy number of 7 or more correlates to an H-score of 100 or more in a DDR2 IHC staining assay. In some embodiments a DDR2 DNA copy number of 8 or more correlates to an H-score of 100 or more in a DDR2 IHC staining assay. In some embodiments a DDR2 DNA copy number of 9 or more correlates to an H-score of 100 or more in a DDR2 IHC staining assay. In some embodiments a DDR2 DNA copy number of 10 or more correlates to an H-score of 100 or more in a DDR2 IHC staining assay.

In another aspect, the present invention provides a bicyclic peptide complex toxin conjugate or heterotandem bicyclic peptide complex specific for Nectin-4, or a pharmaceutically acceptable salt thereof, or a pharmaceutical composition thereof for use in a method of treating a cancer in a patient having an elevated Nectin-4 protein and/or RNA expression level in tumor tissue, said method comprising selecting a patient having a Log2 of the SDHC copy number ratio, in tumor a diseased tissue of 0.1 or more, 0.2 or more, 0.3 or more, 0.4 or more, 0.5 or more, 0.6 or more, 0.7 or more, 0.8 or more, 0.9 or more, or 1.0 or more in tumor tissue or in circulating tumor DNA (ctDNA) as determined by Next-generation sequencing (NGS) technologies or an array-based sequence capture, and administering to the patient the bicyclic peptide complex toxin conjugate or heterotandem bicyclic peptide complex specific for Nectin-4, or the pharmaceutically acceptable salt thereof, or the pharmaceutical composition thereof.

An elevated Nectin-4, SDHC, or DDR2 DNA copy number may be used to identify patients likely to have tumors with higher Nectin-4 protein expression (as measured by IHC). FIG. 7D shows a rightward shift of the red curve (log2 CN ratio $\geq$ 0.6) compared to the blue curve (no copy number cutoff). This demonstrates that Nectin-4/SDHC/DDR2 amplifications can be used to identify and enrich for patients with higher Nectin-4 H-scores. Without being bound by any particular theory, such identification would enrich for a higher percentage of patients with higher Nectin-4 protein expression (as determined by IHC). This would have the potential to reduce the screen fail frequency as defined by (number of patients that don't meet the required H-score cutoff)/(total number of patients screened).

This concept is also shown in the modeling depicted in **FIG. 9**- depicting enrichment for tumors with higher Nectin-4 protein expression by using a log2 CN ratio $\geq$ 0.5 compared to not using this log2 CN ratio cutoff (shown as the Allcomers plot). This shift is seen when comparing the leftmost H-score bin of 0-100 between the Allcomers plot (left) and the log2 CN ratio $\geq$ 0.5 plot (right).

CD137 (4-1BB) is an immune costimulatory receptor with high therapeutic potential in cancer. After disappointing initial clinical outcomes with agonistic anti-CD137 antibodies, a new generation of systemic and targeted CD137 agonists is entering clinical development. A tumor target dependent CD137 agonist using a novel chemical approach based on the fully synthetic constrained bicyclic peptide technology has been developed. Nectin-4 is a cell adhesion protein that is overexpressed in multiple human cancers that may benefit from CD137 agonism. BT7480 is a novel, first-in-class, Nectin-4/CD137 tumor-targeted immune cell agonist (TICA).

It is well known that several major solid tumor types express Nectin-4 and may be infiltrated with immune cells to varying degrees. A compound that activates CD137 only when co-ligated with Nectin-4 requires the relevant immune tumor cells to come into proximity. Analysis of RNA expression data from human tumor samples within The Cancer Genome Atlas (TCGA) indicated co-expression of Nectin-4 and CD137 across several tumor types, including lung, breast, esophageal, stomach, ovarian, head and neck, pancreatic, and bladder (see **FIG. 10A).** More than half of the tumor types examined had a substantive proportion of tumors that expressed high levels of both Nectin-4 and CD137 (see **FIG. 10A).** Based on these data, three major tumor types, namely NSCLC, HNSCC and bladder cancers were selected to further examine Nectin-4 and CD137 in human tumors via spatial proteomic profiling and image analysis (see Example 3).

The Nectin-4 protein levels in tumor tissues have been measured by a multiplexed immunofluorescence (mIF) assay. It has been found that the Nectin-4 protein levels on tumor cell membrane and in tumor cell cytoplasm are indicative of tumor responsiveness to treatment with a Bicycle tumor targeted immune agonist (TICA) specific for Nectin-4. Without wishing to be bound by any particular theory or mechanism, the inventors have discovered that a tumor having an elevated Nectin-4 protein level in a diseased tissue is more likely to benefit from a treatment with a Bicycle tumor targeted immune

agonist (TICA) specific for Nectin-4. It has also been found that a tumor having an elevated Nectin-4 protein level on tumor cell membrane is more likely to benefit from a treatment with BT7480.

**2. Compounds and Definitions:**

As used herein, the term "a Bicycle toxin conjugate specific for Nectin-4" refers to a Bicycle toxin conjugate that binds specifically to Nectin-4. Various Bicycle toxin conjugates specific for Nectin-4 have been described previously, for example, in US 2019/03889906, WO 2019/243832, and WO 2019/243833. BT8009 is referred to as BCY8245 in US 2019/03889906, WO 2019/243832, and WO 2019/243833.

The term "BT8009," as used herein, is a Bicycle toxin conjugate having a structure as shown below, or a pharmaceutically acceptable salt thereof, wherein the molecular scaffold is 1,1',1"-(1,3,5-triazinane-1,3,5-triyl)triprop-2-en-1-one (TATA), and the peptide ligand comprises the amino acid sequence:

($\beta$-Ala)-$Sar_{10}$-$C_i$P[1Nal][dD]$C_{ii}$M[HArg]DWSTP[HyP]W$C_{iii}$ (SEQ ID NO: 1)

wherein Sar is sarcosine, 1Nal represents 1-naphthylalanine, HArg represents homoarginine, HyP represents hydroxyproline and $C_i$, $C_{ii}$ and $C_{iii}$ represent first, second and third cysteine residues.

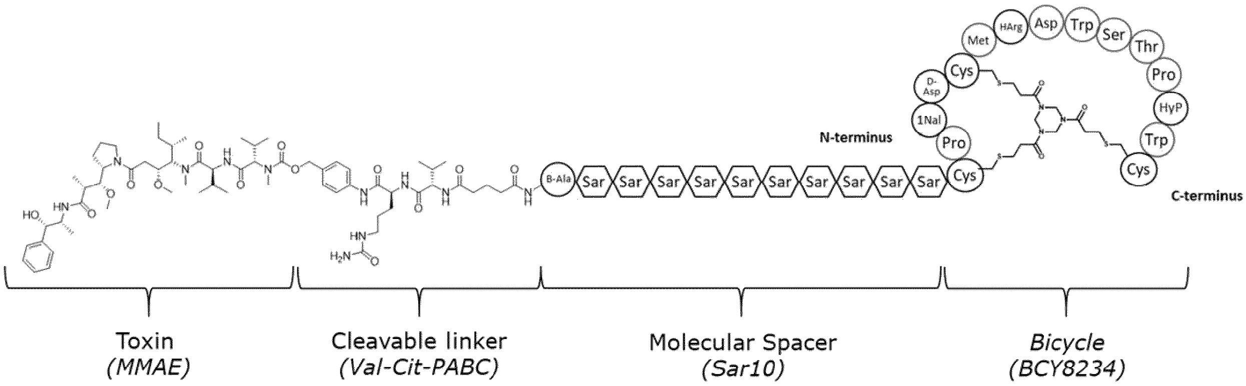


**BT8009**

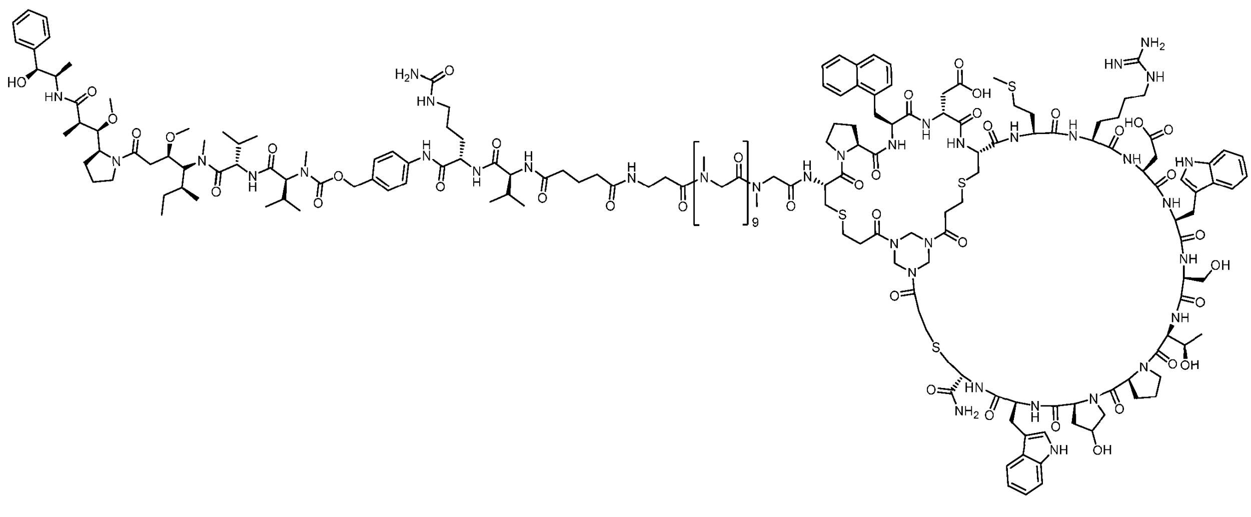

**BT8009**

As used herein, the term "Bicycle tumor targeted immune agonists (TICAs) specific for Nectin-4" refers to Bicycle tumor targeted immune agonists (TICAs) that bind specifically to Nectin-4. Various Bicycle tumor targeted immune agonists (TICAs) specific for Nectin-4 have been described previously, for example, in US 2019/0307836, WO 2019/193328, US 2021/0040154, WO 2021/019244, and WO 2021/019246. BT7480 is referred to as BCY11863 in US 2021/0040154, WO 2021/019244, and WO 2021/019246. The term "BT7480" is a Bicycle tumor targeted immune agonist (TICA), which is a heterotandem bicyclic peptide complex consisting of a Nectin-4 specific peptide linked to two CD137 specific peptides via a N-(acid-$PEG_3$)-N-bis($PEG_3$-azide) linker, having a structure as shown below.

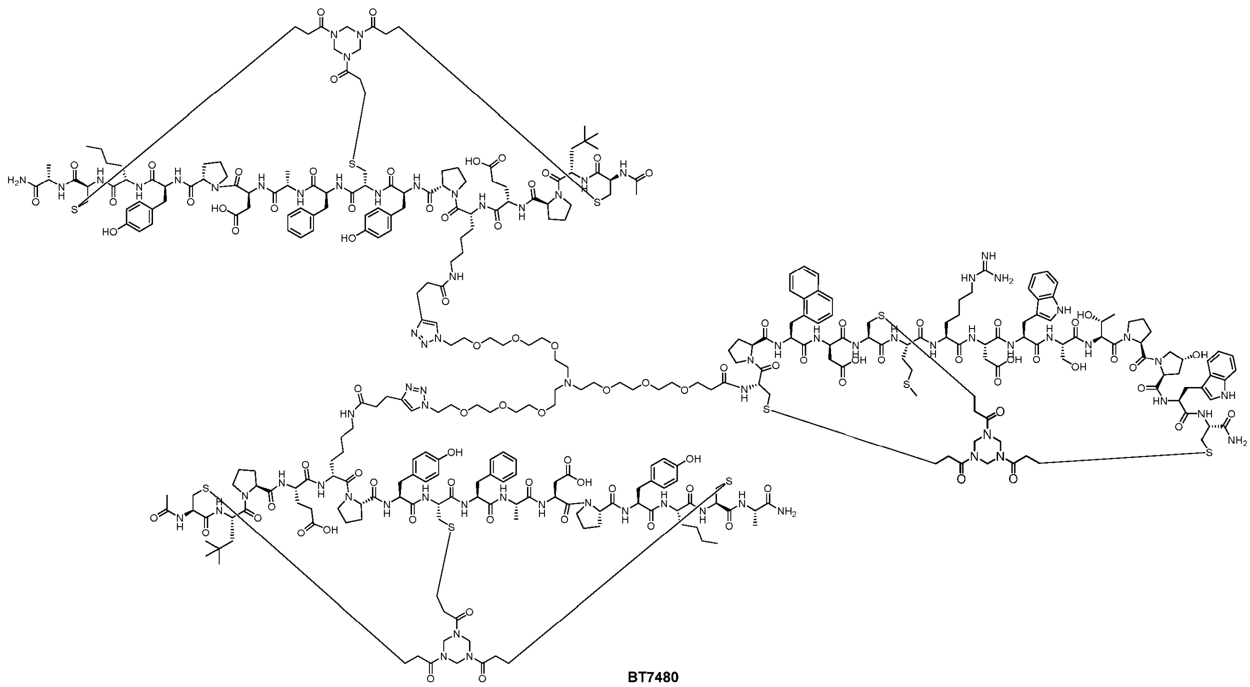

BT7480

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19. Pharmaceutically acceptable salts include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

Salts derived from appropriate bases include alkali metal, alkaline earth metal, ammonium and $N^+(C_{1-4}alkyl)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate. It will be appreciated that references to peptide ligands include the salt forms of said ligands.

The salts can be synthesized from the parent compound that contains a basic or acidic moiety by conventional chemical methods such as methods described in Pharmaceutical Salts: Properties, Selection, and Use, P. Heinrich Stahl (Editor), Camille G. Wermuth (Editor), ISBN: 3-90639-026-8, Hardcover, 388 pages, August 2002. Generally, such salts can be prepared by reacting the free acid or base forms of these compounds with the appropriate base or acid in water or in an organic solvent, or in a mixture of the two.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are disclosed. Unless otherwise stated, all tautomeric forms are disclosed. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a $^{13}C$- or $^{14}C$-enriched carbon are disclosed. Such compounds are

useful, for example, as analytical tools, as probes in biological assays, or as therapeutic agents.

As used herein, the terms "about" or "approximately" have the meaning of within 20% of a given value or range. In some embodiments, the term "about" refers to within 20%, 19%, 18%, 17%, 16%, 15%, 14%, 13%, 12%, 11%, 10%, 9%, 8%, 7%, 6%, 5%, 4%, 3%, 2%, or 1% of a given value.

### 3. Description of Exemplary Embodiments of the Invention

As used herein, the term "a patient who is staining positive" refers to a patient having certain percentage of cells in a tumor tissue section which are staining positive in a Nectin-4 IHC staining assay. In some embodiments, a patient who is staining positive has about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of cells in a tumor tissue section which are staining positive in an Nectin-4 IHC staining assay.

There are a variety of methods to measure staining intensity in an IHC staining assay. Staining intensity may be measured by visual scoring, for example, by manual scoring using conventional light microscopy. Staining intensity may be measured by computational tissue analysis (CTA) scoring. The staining intensity levels can be no staining (0), weak staining (1+), median staining (2+), or strong staining (3+). Staining intensity may be measured on tumor cell membrane of a tumor tissue section. Staining intensity may be measured in tumor cell cytoplasm of a tumor tissue section. Staining intensity may be measured both on tumor cell membrane and in tumor cell cytoplasm of a tumor tissue section.

Staining positive may refer to an H-score of about 15 or more in a tumor tissue section in an IHC staining assay. Staining positive may refer to an H-score of about 20 or more in a tumor tissue section in an IHC staining assay. Staining positive may refer to an H-score of about 30 or more in a tumor tissue section in an IHC staining assay. Staining positive may refer to an H-score of about 40 or more in a tumor tissue section in an IHC staining assay. Staining positive may refer to an H-score of about 50 or more in a tumor tissue section in an IHC staining assay. Staining positive may refer to an H-score of about 75 or more in a tumor tissue section in an IHC staining assay. Staining positive may refer to an H-score of about 100 or more in a tumor tissue section in an IHC staining assay. Staining positive may refer to an H-score of about 125 or more in a tumor tissue section in an IHC staining assay. Staining positive may refer to an H-score of about 150 or more in a tumor tissue section in an IHC staining assay. Staining positive may refer to an H-score of about 200 or more in a tumor tissue section in an IHC staining assay. Staining positive may refer to an H-score of about 300 or more in a tumor tissue section in an IHC staining assay. Staining positive may refer to an H-score of about 400 or more in a tumor tissue section in an IHC staining assay. Staining positive may refer to an H-score of about 500 or more in a tumor tissue section in an IHC staining assay.

An H-score is the sum of the products of the percent of cells x their staining intensity on a scale of 0-3 as described above (no staining (0), weak staining (1+), median staining (2+), or strong staining (3+)):

$$[((0 \text{ x } (\% \text{ cells at } 0)) + ((1 \text{ x } (\% \text{ cells at } 1+)) + ((2 \text{ x } (\% \text{ cells at } 2+)) + ((3 \text{ x } (\% \text{ cells at } 3))]$$

An H-score can be generated for different compartment in a tumor tissue section, including, for example, the tumor cell membrane and cytoplasm. An H-score may refer to an H-score for tumor cell membrane, which is the sum of the products of the percent of cells x their cell membrane staining intensity on a scale of 0-3 as described above. An H-score may refer to an H-score for tumor cell cytoplasm, which is the sum of the products of the percent of cells x their cytoplasm staining intensity on a scale of 0-3 as described above.

Staining positive may refer to an H-score for tumor cell membrane of about 15 or more in a tumor tissue section in an IHC staining assay. Staining positive may refer to an H-score for tumor cell membrane of about 20 or more in a tumor tissue section in an IHC staining assay. Staining positive may refer to an H-score for tumor cell membrane of about 30 or more in a tumor tissue section in an IHC staining assay. Staining positive may refer to an H-score for tumor cell membrane of about 40 or more in a tumor tissue section in an IHC staining assay. Staining positive may refer to an H-score for tumor cell membrane of about 50 or more in a tumor tissue section in an IHC staining assay. Staining positive may refer to an H-score for tumor cell membrane of about 75 or more in a tumor tissue section in an IHC staining assay. Staining positive may refer to an H-score for tumor cell membrane of about 100 or more in a tumor tissue section in an IHC staining assay. Staining positive may refer to an H-score for tumor cell membrane of about 125 or more in a tumor tissue section in an IHC staining assay. Staining positive may refer to an H-score for tumor cell membrane of about 150 or more in a tumor tissue section in an IHC staining assay. Staining positive may refer to an H-score for tumor cell membrane of about 200 or more in a tumor tissue section in an IHC staining assay. Staining positive may refer to an H-score for tumor cell membrane of about 300 or more in a tumor tissue section in an IHC staining assay. Staining positive may refer to an H-score for tumor cell membrane of about 400 or more in a tumor tissue section in an IHC staining assay. Staining positive may refer to an H-score for tumor cell membrane of about 500 or more in a tumor tissue section in an IHC staining assay.

In some embodiments, staining positive refers to an H-score for tumor cell cytoplasm of about 15 or more in a

tumor tissue section in an IHC staining assay. In some embodiments, staining positive refers to an H-score for tumor cell cytoplasm of about 20 or more in a tumor tissue section in an IHC staining assay. In some embodiments, staining positive refers to an H-score for tumor cell cytoplasm of about 30 or more in a tumor tissue section in an IHC staining assay. In some embodiments, staining positive refers to an H-score for tumor cell cytoplasm of about 40 or more in a tumor tissue section in an IHC staining assay. In some embodiments, staining positive refers to an H-score for tumor cell cytoplasm of about 50 or more in a tumor tissue section in an IHC staining assay. In some embodiments, staining positive refers to an H-score for tumor cell cytoplasm of about 75 or more in a tumor tissue section in an IHC staining assay. In some embodiments, staining positive refers to an H-score for tumor cell cytoplasm of about 100 or more in a tumor tissue section in an IHC staining assay. In some embodiments, staining positive refers to an H-score for tumor cell cytoplasm of about 125 or more in a tumor tissue section in an IHC staining assay. In some embodiments, staining positive refers to an H-score for tumor cell cytoplasm of about 150 or more in a tumor tissue section in an IHC staining assay. In some embodiments, staining positive refers to an H-score for tumor cell cytoplasm of about 200 or more in a tumor tissue section in an IHC staining assay. In some embodiments, staining positive refers to an H-score for tumor cell cytoplasm of about 300 or more in a tumor tissue section in an IHC staining assay. In some embodiments, staining positive refers to an H-score for tumor cell cytoplasm of about 400 or more in a tumor tissue section in an IHC staining assay. In some embodiments, staining positive refers to an H-score for tumor cell cytoplasm of about 500 or more in a tumor tissue section in an IHC staining assay.

In some embodiments, staining positive refers to a combined H-score for tumor cell membrane and tumor cell cytoplasm of about 15 or more in a tumor tissue section in an IHC staining assay. In some embodiments, staining positive refers to a combined H-score for tumor cell cytoplasm of about 20 or more in a tumor tissue section in an IHC staining assay. In some embodiments, staining positive refers to a combined H-score for tumor cell cytoplasm of about 30 or more in a tumor tissue section in an IHC staining assay. In some embodiments, staining positive refers to a combined H-score for tumor cell cytoplasm of about 40 or more in a tumor tissue section in an IHC staining assay. In some embodiments, staining positive refers to a combined H-score for tumor cell cytoplasm of about 50 or more in a tumor tissue section in an IHC staining assay. In some embodiments, staining positive refers to a combined H-score for tumor cell cytoplasm of about 75 or more in a tumor tissue section in an IHC staining assay. In some embodiments, staining positive refers to a combined H-score for tumor cell cytoplasm of about 100 or more in a tumor tissue section in an IHC staining assay. In some embodiments, staining positive refers to a combined H-score for tumor cell cytoplasm of about 125 or more in a tumor tissue section in an IHC staining assay. In some embodiments, staining positive refers to a combined H-score for tumor cell cytoplasm of about 150 or more in a tumor tissue section in an IHC staining assay. In some embodiments, staining positive refers to a combined H-score for tumor cell cytoplasm of about 200 or more in a tumor tissue section in an IHC staining assay. In some embodiments, staining positive refers to a combined H-score for tumor cell cytoplasm of about 300 or more in a tumor tissue section in an IHC staining assay. In some embodiments, staining positive refers to a combined H-score for tumor cell cytoplasm of about 400 or more in a tumor tissue section in an IHC staining assay. In some embodiments, staining positive refers to a combined H-score for tumor cell cytoplasm of about 500 or more in a tumor tissue section in an IHC staining assay.

In some embodiments, a patient is a patient having pancreatic cancer. In some embodiments, a patient is a patient having stomach cancer. In some embodiments, a patient is a patient having bladder cancer. In some embodiments, a patient is a patient having head & neck cancer. In some embodiments, a patient is a patient having non-small cell lung cancer (NSCLC). In some embodiments, a patient is a patient having triple negative breast cancer (TNBC). In some embodiments, a patient is a patient having ovarian cancer.

In some embodiments, a tumor tissue is a lung tumor tissue. In some embodiments, a tumor tissue is an ovarian tumor tissue. In some embodiments, a tumor tissue is a breast tumor tissue. In some embodiments, a tumor tissue is a gastric/upper gastrointestinal (GI) tumor tissue. In some embodiments, a tumor tissue is a pancreatic tumor tissue. In some embodiments, a tumor tissue is an urothelial tumor tissue. In some embodiments, a tumor tissue is a non-small-cell lung cancer (NSCLC) tumor tissue. In some embodiments, a tumor tissue is a triple-negative breast cancer (TNBC) tumor tissue.

As used herein, the terms "increases," "elevates," or "enhances," are used interchangeably and encompass any measurable increase in a biological function and/or biological activity and/or a concentration. For example, an increase can be by at least about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, about 100%, about 2-fold, about 3-fold, about 4-fold, about 5-fold, about 6-fold, about 7-fold, about 8-fold, about 9-fold, about 10-fold, about 20-fold, about 25-fold, about 50-fold, about 100-fold, or higher, relative to a control or baseline amount of a function, or activity, or concentration.

As used herein, the terms "elevated level" of a substance (e.g., Nectin-4 protein, Nectin-4 mRNA, Nectin-4 DNA copy number, SDHC DNA copy number, or DDR2 DNA copy number) in a sample refers to an increase in the amount of the substance of about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, about 99%, about 100%, about 2-fold, about 3-fold, about 4-fold, about 5-fold, about 6-fold, about 7-fold, about 8-fold, about 9-fold, about 10-fold, about 20-fold, about 25-fold, about 50-fold, about 100-fold, or higher, relative to the amount of the substance in a control sample or control samples, such as an individual or group of individuals who are not suffering from the disease or disorder (e.g., cancer) or an internal control, as determined by techniques known in the art. A subject can also be determined to have an "elevated level" of a substance if the concentration of the substance is increased by one standard deviation, two standard deviations, three standard deviations, four standard deviations, five standard deviations, or more relative to the mean (average) or median amount of the substance in a control group of samples or a baseline group of samples or a retrospective analysis of patient samples. As practiced in the art, such control or baseline levels can be previously determined, or measured prior to the measurement in the sample, or can be obtained from a database of such control samples. In other words, the control and subject samples do not have to be tested simultaneously. In some embodiments, an elevated Nectin-4 DNA copy number is measured by a Nectin-4 amplification. In some embodiments, an elevated SDHC DNA copy number is measured by an SDHC amplification. In some embodiments, an elevated DDR2 DNA copy number is measured by a DDR2 amplification.

As used herein, the term "an SDHC amplification" refers to an increase in SDHC DNA copy number over normal tissue. In some embodiments, this is expressed as Log2 of the copy number ratio of SDHC Log2(CN ratio). In some embodiments, the SDHC Log2(CN ratio) is > 0.1. In some embodiments, the SDHC Log2(CN ratio) is > 0.2. In some embodiments, the SDHC Log2(CN ratio) is > 0.3. In some embodiments, the SDHC Log2(CN ratio) is > 0.4. In some embodiments, the SDHC Log2(CN ratio) is > 0.5. In some embodiments, the SDHC Log2(CN ratio) is > 0.6. In some embodiments, the SDHC Log2(CN ratio) is > 0.7. In some embodiments, the SDHC Log2(CN ratio) is > 0.8. In some embodiments, the SDHC Log2(CN ratio) is > 0.9. In some embodiments, the SDHC Log2(CN ratio) is > 1.0.

As used herein, the term "a Nectin-4 amplification" refers to an increase in Nectin-4 DNA copy number over normal tissue. In some embodiments, this is expressed as Log2 of the copy number ratio or Nectin-4 Log2(CN ratio). In some embodiments, the Nectin-4 Log2(CN ratio) is > 0.1. In some embodiments, the Nectin-4 Log2(CN ratio) is > 0.2. In some embodiments, the Nectin-4 Log2(CN ratio) is > 0.3. In some embodiments, the Nectin-4 Log2(CN ratio) is > 0.4. In some embodiments, the Nectin-4 Log2(CN ratio) is > 0.5. In some embodiments, the Nectin-4 Log2(CN ratio) is > 0.6. In some embodiments, the Nectin-4 Log2(CN ratio) is > 0.7. In some embodiments, the Nectin-4 Log2(CN ratio) is > 0.8. In some embodiments, the Nectin-4 Log2(CN ratio) is > 0.9. In some embodiments, the Nectin-4 Log2(CN ratio) is > 1.0.

As used herein, the term "a DDR2 amplification" refers to an increase in DDR2 DNA copy number over normal tissue. In some embodiments, this is expressed as Log2 of the copy number ratio or DDR2 Log2(CN ratio). In some embodiments, the DDR2 Log2(CN ratio) is > 0.1. In some embodiments, the DDR2 Log2(CN ratio) is > 0.2. In some embodiments, the DDR2 Log2(CN ratio) is > 0.3. In some embodiments, the DDR2 Log2(CN ratio) is > 0.4. In some embodiments, the DDR2 Log2(CN ratio) is > 0.5. In some embodiments, the DDR2 Log2(CN ratio) is > 0.6. In some embodiments, the DDR2 Log2(CN ratio) is > 0.7. In some embodiments, the DDR2 Log2(CN ratio) is > 0.8. In some embodiments, the DDR2 Log2(CN ratio) is > 0.9. In some embodiments, the DDR2 Log2(CN ratio) is > 1.0.

There are a variety of methods to measure the DNA copy number of Nectin-4, SDHC, and DDR2 in a tissue. In some embodiments, a method of measuring the Nectin-4, SDHC, or DDR2 DNA copy number in tumor tissue or in circulating tumor DNA (ctDNA) of a patient comprises using whole exome sequencing.

The invention provides methods of identifying or selecting a patient having an elevated Nectin-4 protein level in tumor tissue, comprising measuring the SDHC DNA copy number in tumor tissue or in ctDNA, and selecting a patient who has an elevated SDHC DNA copy number.

In some embodiments, the methods of the invention may further comprise measuring the Nectin-4 DNA copy number, and selecting a patient who has an elevated SDHC DNA copy number and an elevated Nectin-4 DNA copy number.

In some embodiments, the methods of the invention may further comprise measuring the DDR2 DNA copy number, and selecting a patient who has an elevated SDHC DNA copy number and an elevated DDR2 DNA copy number.

In some embodiments, the invention provides a method of identifying or selecting a patient having an elevated Nectin-4 protein level in a tumor tissue, comprising measuring the SDHC DNA copy number in tumor tissue or in circulating tumor DNA (ctDNA) section of a patient using whole exome sequencing, and selecting a patient having an SDHC Log2(CN ratio) of about 0.1 or more, about 0.2 or more, about 0.3 or more, about 0.4 or more, about 0.5 or more, about 0.6 or more, about 0.7 or more, about 0.8 or more, about 0.9 or more, or about 1.0 or more.

In some embodiments, the methods of the invention may further comprise measuring the Nectin-4 DNA copy number in tumor tissue or in circulating tumor DNA (ctDNA) section of a patient using whole exome sequencing, and selecting a patient having an elevated SDHC DNA copy number and a Nectin-4 Log2(CN ratio) of about 0.1 or more, about 0.2 or more, about 0.3 or more, about 0.4 or more, about 0.5 or more, about 0.6 or more, about 0.7 or more, about 0.8 or more, about 0.9 or more, or about 1.0 or more.

In some embodiments, the methods of the invention may further comprise measuring the DDR2 DNA copy number in tumor tissue or in circulating tumor DNA (ctDNA) section of a patient using whole exome sequencing, and selecting a patient having an elevated SDHC DNA copy number and a DDR2 Log2(CN ratio) of about 0.1 or more, about 0.2 or more, about 0.3 or more, about 0.4 or more, about 0.5 or more, about 0.6 or more, about 0.7 or more, about 0.8 or

more, about 0.9 or more, or about 1.0 or more.

In some embodiments, a Bicycle toxin conjugate specific for Nectin-4 is selected from the compounds as described in US 2019/03889906, WO 2019/243832, and WO 2019/243833.

In some embodiments, a Bicycle toxin conjugate specific for Nectin-4 is BT8009 as described herein, or a pharmaceutically acceptable salt thereof.

A Bicycle toxin conjugate specific for Nectin-4, or a pharmaceutically acceptable salt thereof, or a pharmaceutical composition thereof, can be administered to a patient at various dose ranges.

In some embodiments, a Bicycle toxin conjugate specific for Nectin-4, or a pharmaceutically acceptable salt thereof, or a pharmaceutical composition thereof may be administered to a patient at a dose of about 1 mg/kg or less. In some embodiments, a Bicycle toxin conjugate specific for Nectin-4, or a pharmaceutically acceptable salt thereof, or a pharmaceutical composition thereof, may be administered to a patient at a dose of about 0.9 mg/kg, about 0.8 mg/kg, about 0.7 mg/kg, about 0.6 mg/kg, about 0.5 mg/kg, about 0.4 mg/kg, about 0.3 mg/kg, about 0.2 mg/kg, or about 0.1 mg/kg.

In some embodiments, a pharmaceutical composition is administered at a dose of about 1-27 mg/m$^2$. In some embodiments, a pharmaceutical composition may be administered at a dose of about 2-20 mg/m$^2$. In some embodiments, a pharmaceutical composition is administered at a dose of about 2-20 mg/m$^2$. In some embodiments, a pharmaceutical composition is administered at a dose of about 2.2, 4.4, 7.3, 11, 14.6, or 19.4 mg/m$^2$. In some embodiments, a pharmaceutical composition is administered at a dose of about 2.5, 5.0, 7.5, 10.0, 13.0, or 17.0 mg/m$^2$. In some embodiments, a pharmaceutical composition is administered at a dose of about 1.5-3.5, 3.5-5.5, 6.5-8.5, 10-12, 13.5-15.5, or 18.5-20.5 mg/m$^2$. In some embodiments, a pharmaceutical composition is administered at a dose of about 1-10 or 10-20 mg/m$^2$. In some embodiments, a pharmaceutical composition is administered at a dose of about 21, 22, 23, 24, 25, 26, or 27 mg/m$^2$.

A Bicycle toxin conjugate specific for Nectin-4, or a pharmaceutically acceptable salt thereof, or a pharmaceutical composition thereof, can be administered to a patient at various dose frequencies. In some embodiments, a Bicycle toxin conjugate specific for Nectin-4, or a pharmaceutically acceptable salt thereof, or a pharmaceutical composition thereof, may be administered to a patient at a dose frequency of one dose every 2 days, one dose every 3 days, one dose every 4 days, one dose every 5 days, one dose every 6 days, or one dose every 7 days. In some embodiments, a Bicycle toxin conjugate specific for Nectin-4, or a pharmaceutically acceptable salt thereof, or a pharmaceutical composition thereof may be administered to a patient at a dose frequency of two doses every week, one dose every week, one dose every two weeks, one dose every three weeks, or one dose every 4 weeks.

In some embodiments, a patient is a patient having pancreatic cancer. In some embodiments, a patient is a patient having stomach cancer. In some embodiments, a patient is a patient having bladder cancer. In some embodiments, a patient is a patient having head & neck cancer. In some embodiments, a patient is a patient having non-small cell lung cancer (NSCLC). In some embodiments, a patient is a patient having triple negative breast cancer (TNBC). In some embodiments, a patient is a patient having ovarian cancer.

In some embodiments, a tumor tissue is a pancreatic tumor tissue. In some embodiments, a tumor tissue is a stomach tumor tissue. In some embodiments, a tumor tissue is a bladder tumor tissue. In some embodiments, a tumor tissue is a head & neck tumor tissue. In some embodiments, a tumor tissue is a non-small cell lung cancer (NSCLC) tumor tissue. In some embodiments, a tumor tissue is a triple negative breast cancer (TNBC) tumor tissue. In some embodiments, a tumor tissue is an ovarian tumor tissue.

As used herein, the term "an elevated Nectin-4 protein level" refers to that certain percentage of cells in a tumor tissue have a detectable amount of Nectin-4 protein, for example, on tumor cell membrane, or in tumor cell cytoplasm, or both. Nectin-4 positive may refer to about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of cells in a tumor tissue that have a detectable amount of Nectin-4 protein, for example, on tumor cell membrane, or in tumor cell cytoplasm, or both.

There are a variety of methods to measure the amount of Nectin-4 protein in a tissue. One such method of measuring Nectin-4 protein level in a tumor tissue of a patient comprises using a Nectin-4 multiplexed immunofluorescence (mIF) assay. A Nectin-4 mIF assay may comprise staining a tumor tissue section using a rabbit monoclonal α-Nectin-4 primary antibody, for example a rabbit monoclonal α-Nectin-4 primary antibody that selectively binds to the extracellular domain (ECD) of Nectin-4. One such rabbit monoclonal α-Nectin-4 primary antibody that selectively binds to the ECD of Nectin-4 is the rabbit monoclonal α-Nectin-4 primary antibody YMW-1-58.

A rabbit monoclonal α-Nectin-4 primary antibody may be used at a concentration of up to about 50 μg/mL, up to about 40 μg/mL, up to about 30 μg/mL, up to about 20 μg/mL, or up to about 10 μg/mL. A rabbit monoclonal α-Nectin-4 primary antibody may be used at a concentration of about 5 μg/mL, about 6 μg/mL, about 7 μg/mL, about 8 μg/mL, about 9 μg/mL, about 10 μg/mL, about 11 μg/mL, about 12 μg/mL, about 13 μg/mL, about 14 μg/mL, or about 15 μg/mL. A rabbit monoclonal α-Nectin-4 primary antibody selectively binding to the ECD of Nectin-4, such as YMW-1-58, may be used at a concentration of about 5 μg/mL, about 6 μg/mL, about 7 μg/mL, about 8 μg/mL, about 9 μg/mL, about 10 μg/mL, about 11

μg/mL, about 12 μg/mL, about 13 μg/mL, about 14 μg/mL, or about 15 μg/mL. A rabbit monoclonal α-Nectin-4 primary antibody selectively binding to the ECD of Nectin-4, such as YMW-1-58, may be used at a concentration of about 10 μg/mL.

Disclosed herein are methods of identifying or selecting a patient having an elevated Nectin-4 protein level in a tumor tissue, comprising measuring staining intensity in a tumor tissue section of a patient using a Nectin-4 mIF assay, and selecting a patient who is staining positive in the Nectin-4 mIF assay. An exemplary Nectin-4 mIF assay is as described in Example 3 herein.

As used herein, the term "a patient who is staining positive" refers to a patient having certain percentage of cells in a tumor tissue section which are staining positive in a Nectin-4 mIF assay. A patient who is staining positive may have about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, or about 95% of cells in a tumor tissue section which are staining positive in a Nectin-4 mIF assay.

There are a variety of methods to measure staining intensity in a mIF assay. Staining intensity may be measured by visual scoring, for example, by manual scoring using conventional light microscopy. Staining intensity may be measured by computational tissue analysis (CTA) scoring. The staining intensity levels can be no staining (0), weak staining (1+), median staining (2+), or strong staining (3+). Staining intensity may be measured on tumor cell membrane of a tumor tissue section, in tumor cell cytoplasm of a tumor tissue section, or both on tumor cell membrane and in tumor cell cytoplasm of a tumor tissue section.

Staining positive may refer to an H-score of about 15 or more , about 20 or more, about 30 or more, about 40 or more, about 50 or more, about 75 or more, about 100 or more, about 125 or more or about 150 or more in a tumor tissue section in a mIF assay.

An H-score is the sum of the products of the percent of cells x their staining intensity on a scale of 0-3 as described above (no staining (0), weak staining (1+), median staining (2+), or strong staining (3+)):

$$[((0 \text{ x } (\% \text{ cells at } 0)) + ((1 \text{ x } (\% \text{ cells at } 1+)) + ((2 \text{ x } (\% \text{ cells at } 2+)) + ((3 \text{ x } (\% \text{ cells at } 3))]$$

An H-score can be generated for different compartment in a tumor tissue section, including, for example, the tumor cell membrane and cytoplasm. An H-score may refer to an H-score for tumor cell membrane, which is the sum of the products of the percent of cells x their cell membrane staining intensity on a scale of 0-3 as described above. An H-score may refer to an H-score for tumor cell cytoplasm, which is the sum of the products of the percent of cells x their cytoplasm staining intensity on a scale of 0-3 as described above.

Staining positive may refer to an H-score for tumor cell membrane of about 15 or more, about 20 or more, about 30 or more, 40 or more, about 50 or more, about 75 or more, about 100, about 125 or more or about 150 or more in a tumor tissue section in a mIF assay.

In some embodiments, staining positive refers to an H-score for tumor cell cytoplasm of about 15 or more, about 20 or more, about 30 or more, about 40 or more, about 50 or more, about 75 or more, about 100 or more, about 125 or more or about 150 or more in a tumor tissue section in a mIF assay.

In some embodiments, a cancer is pancreatic cancer. In some embodiments, a cancer is stomach cancer. In some embodiments, a cancer is bladder cancer. In some embodiments, a cancer is head & neck cancer. In some embodiments, a cancer is non-small cell lung cancer (NSCLC). In some embodiments, a cancer is a triple negative breast cancer (TNBC). In some embodiments, a cancer is ovarian cancer.

In some embodiments, a Bicycle TICA specific for Nectin-4 is BT7480 as described herein, or a pharmaceutically acceptable salt thereof.

A Bicycle TICA specific for Nectin-4, or a pharmaceutically acceptable salt thereof, or a pharmaceutical composition thereof, can be administered to a patient at various dose ranges.

In some embodiments, a Bicycle TICA specific for Nectin-4, or a pharmaceutically acceptable salt thereof, or a pharmaceutical composition thereof, may be administered to a patient at a dose of about 1 mg/kg or less. In some embodiments, a Bicycle TICA specific for Nectin-4, or a pharmaceutically acceptable salt thereof, or a pharmaceutical composition thereof, may be administered to a patient at a dose of about 0.9 mg/kg, about 0.8 mg/kg, about 0.7 mg/kg, about 0.6 mg/kg, about 0.5 mg/kg, about 0.4 mg/kg, about 0.3 mg/kg, about 0.2 mg/kg, or about 0.1 mg/kg.

In some embodiments, a Bicycle TICA specific for Nectin-4, or a pharmaceutically acceptable salt thereof, or a pharmaceutical composition thereof, may be administered to a patient at a dose of about 100 mg/$m^2$ or less. In some embodiments, a Bicycle TICA specific for Nectin-4, or a pharmaceutically acceptable salt thereof, or a pharmaceutical composition thereof, may be administered to a patient at a dose of about 90 mg/$m^2$, about 80 mg/$m^2$, about 70 mg/$m^2$, about 60 mg/$m^2$, about 50 mg/$m^2$, about 40 mg/$m^2$, about 30 mg/$m^2$, about 25 mg/$m^2$, about 22.5 mg/$m^2$, about 20 mg/$m^2$, about 17.5 mg/$m^2$, about 15 mg/$m^2$, about 12.5 mg/$m^2$, about 10 mg/$m^2$, about 7.5 mg/$m^2$, about 5 mg/$m^2$, about 2.5 mg/$m^2$, or about 1 mg/$m^2$. In some embodiments, a Bicycle TICA specific for Nectin-4, or a pharmaceutically acceptable

salt thereof, or a pharmaceutical composition thereof, may be administered to a patient at a dose of about 2 $mg/m^2$ to about 25 $mg/m^2$.

A Bicycle TICA specific for Nectin-4, or a pharmaceutically acceptable salt thereof, or a pharmaceutical composition thereof, can be administered to a patient at various dose frequencies. In some embodiments, a Bicycle TICA specific for Nectin-4, or a pharmaceutically acceptable salt thereof, or a pharmaceutical composition thereof, may be administered to a patient at a dose frequency of one dose every 2 days, one dose every 3 days, one dose every 4 days, one dose every 5 days, one dose every 6 days, or one dose every 7 days. In some embodiments, a Bicycle TICA specific for Nectin-4, or a pharmaceutically acceptable salt thereof, or a pharmaceutical composition thereof, may be administered to a patient at a dose frequency of two doses every week, one dose every week, one dose every two weeks, one dose every three weeks, or one dose every 4 weeks.

### 4. Formulation and Administration

In some embodiments, a pharmaceutical composition comprising a Bicycle toxin conjugate specific for Nectin-4, or a pharmaceutically acceptable salt thereof, as described herein, and a pharmaceutically acceptable carrier, adjuvant, or vehicle. In some embodiments, a Bicycle toxin conjugate specific for Nectin-4, or a pharmaceutically acceptable salt thereof, is formulated for IV administration to a patient.

In some embodiments, a pharmaceutical composition comprising a Bicycle TICA specific for Nectin-4, or a pharmaceutically acceptable salt thereof, as described herein, and a pharmaceutically acceptable carrier, adjuvant, or vehicle. In some embodiments, a Bicycle TICA specific for Nectin-4, or a pharmaceutically acceptable salt thereof, is formulated for IV administration to a patient.

The term "pharmaceutically acceptable carrier, adjuvant, or vehicle" refers to a non-toxic carrier, adjuvant, or vehicle that does not destroy the pharmacological activity of the compound with which it is formulated. Pharmaceutically acceptable carriers, adjuvants or vehicles that may be used pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

Pharmaceutical compositions may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intraperitoneally or intravenously. Sterile injectable forms of the compositions may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For this purpose, any bland fixed oil may be employed including synthetic mono- or di-glycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

Pharmaceutically acceptable compositions may be orally administered in any orally acceptable dosage form including, but not limited to, capsules, tablets, aqueous suspensions or solutions. In the case of tablets for oral use, carriers commonly used include lactose and corn starch. Lubricating agents, such as magnesium stearate, are also typically added. For oral administration in a capsule form, useful diluents include lactose and dried cornstarch. When aqueous suspensions are required for oral use, the active ingredient is combined with emulsifying and suspending agents. If desired, certain sweetening, flavoring or coloring agents may also be added.

Alternatively, pharmaceutically acceptable compositions may be administered in the form of suppositories for rectal administration. These can be prepared by mixing the agent with a suitable non-irritating excipient that is solid at room temperature but liquid at rectal temperature and therefore will melt in the rectum to release the drug. Such materials include cocoa butter, beeswax and polyethylene glycols.

Pharmaceutically acceptable compositions may also be administered topically, especially when the target of treatment includes areas or organs readily accessible by topical application, including diseases of the eye, the skin, or the lower intestinal tract. Suitable topical formulations are readily prepared for each of these areas or organs.

Topical application for the lower intestinal tract can be effected in a rectal suppository formulation (see above) or in a suitable enema formulation. Topically-transdermal patches may also be used.

For topical applications, provided pharmaceutically acceptable compositions may be formulated in a suitable ointment containing the active component suspended or dissolved in one or more carriers. Carriers for topical administration of compounds include, but are not limited to, mineral oil, liquid petrolatum, white petrolatum, propylene glycol, polyoxyethylene, polyoxypropylene compound, emulsifying wax and water. Alternatively, provided pharmaceutically acceptable compositions can be formulated in a suitable lotion or cream containing the active components suspended or dissolved in one or more pharmaceutically acceptable carriers. Suitable carriers include, but are not limited to, mineral oil, sorbitan monostearate, polysorbate 60, cetyl esters wax, cetearyl alcohol, 2-octyldodecanol, benzyl alcohol and water.

For ophthalmic use, provided pharmaceutically acceptable compositions may be formulated as micronized suspensions in isotonic, pH adjusted sterile saline, or, preferably, as solutions in isotonic, pH adjusted sterile saline, either with or without a preservative such as benzylalkonium chloride. Alternatively, for ophthalmic uses, the pharmaceutically acceptable compositions may be formulated in an ointment such as petrolatum.

Pharmaceutically acceptable compositions may also be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other conventional solubilizing or dispersing agents.

Most preferably, pharmaceutically acceptable compositions are formulated for oral administration. Such formulations may be administered with or without food. In some embodiments, pharmaceutically acceptable compositions are administered without food. In other embodiments, pharmaceutically acceptable compositions are administered with food.

The amount of the bicyclic peptide complex toxin conjugate or heterotandem bicyclic peptide complex specific for Nectin-4 that may be combined with the carrier materials to produce a composition in a single dosage form will vary depending upon the host treated, the particular mode of administration. Preferably, provided compositions should be formulated so that a dosage of between 0.01 - 1 mg/kg body weight/day can be administered to a patient receiving these compositions.

It should also be understood that a specific dosage and treatment regimen for any particular patient will depend upon a variety of factors, including the activity of the specific compound employed, the age, body weight, general health, sex, diet, time of administration, rate of excretion, drug combination, and the judgment of the treating physician and the severity of the particular disease being treated. The amount of a compound of the present invention in the composition will also depend upon the particular compound in the composition.

## 5. Uses

**In** some embodiments, the present invention provides a bicyclic peptide complex toxin conjugate or heterotandem bicyclic peptide complex specific for Nectin-4, or a pharmaceutically acceptable salt thereof, or a pharmaceutical composition thereof for use in a method of treating a cancer in a patient having an elevated Nectin-4 protein and/or RNA expression level in tumor tissue, said method comprising selecting a patient having a Log2 of the SDHC copy number ratio of 0.1 or more, 0.2 or more, 0.3 or more, 0.4 or more, 0.5 or more, 0.6 or more, 0.7 or more, 0.8 or more, 0.9 or more, or 1.0 or more in tumor tissue or in circulating tumor DNA (ctDNA) as determined by Next-generation sequencing (NGS) technologies or an array-based sequence capture, and administering to the patient the bicyclic peptide complex toxin conjugate or heterotandem bicyclic peptide complex specific for Nectin-4, the a pharmaceutically acceptable salt thereof, or the pharmaceutical composition thereof.

*Cancer*

The cancer to be treated using the bicyclic peptide complex toxin conjugate or heterotandem bicyclic peptide complex specific for Nectin-4, or a pharmaceutically acceptable salt thereof, or a pharmaceutical composition thereof includes, but is not limited to, a hematological cancer, a lymphoma, a myeloma, a leukemia, a neurological cancer, skin cancer, breast cancer, a prostate cancer, a colorectal cancer, lung cancer, head and neck cancer, a gastrointestinal cancer, a liver cancer, a pancreatic cancer, a genitourinary cancer, a bone cancer, renal cancer, and a vascular cancer.

A cancer to be treated using the bicyclic peptide complex toxin conjugate or heterotandem bicyclic peptide complex specific for Nectin-4, or a pharmaceutically acceptable salt thereof, or a pharmaceutical composition thereof can be selected from colorectal cancer, such as microsatellite-stable (MSS) metastatic colorectal cancer, including advanced or progressive microsatellite-stable (MSS) CRC; non-small cell lung cancer (NSCLC), such as advanced and/or

metastatic NSCLC; ovarian cancer; breast cancer, such as inflammatory breast cancer; endometrial cancer; cervical cancer; head and neck cancer; gastric cancer; gastroesophageal junction cancer; and bladder cancer. In some embodiments, a cancer is colorectal cancer. In some embodiments, the colorectal cancer is metastatic colorectal cancer. In some embodiments, the colorectal cancer is microsatellite-stable (MSS) metastatic colorectal cancer. In some embodiments, a cancer is advanced or progressive microsatellite-stable (MSS) CRC. In some embodiments, a cancer is non-small cell lung cancer (NSCLC). In some embodiments, a cancer is advanced and/or metastatic NSCLC. In some embodiments, a cancer is ovarian cancer. In some embodiments, a cancer is breast cancer. In some embodiments, a cancer is inflammatory breast cancer. In some embodiments, a cancer is endometrial cancer. In some embodiments, a cancer is cervical cancer. In some embodiments, a cancer is head and neck cancer. In some embodiments, a cancer is gastric cancer. In some embodiments, a cancer is gastroesophageal junction cancer. In some embodiments, a cancer is bladder cancer.

Cancer includes, in some embodiments, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (e.g., Hodgkin's disease or non-Hodgkin's disease), Waldenstrom's macroglobulinemia, multiple myeloma, heavy chain disease, and solid tumors such as sarcomas and carcinomas (*e.g.*, fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, and retinoblastoma).

In some embodiments, the cancer is glioma, astrocytoma, glioblastoma multiforme (GBM, also known as glioblastoma), medulloblastoma, craniopharyngioma, ependymoma, pinealoma, hemangioblastoma, acoustic neuroma, oligodendroglioma, schwannoma, neurofibrosarcoma, meningioma, melanoma, neuroblastoma, or retinoblastoma.

In some embodiments, the cancer is acoustic neuroma, astrocytoma (e.g. Grade I - Pilocytic Astrocytoma, Grade II - Low-grade Astrocytoma, Grade III - Anaplastic Astrocytoma, or Grade IV - Glioblastoma (GBM)), chordoma, CNS lymphoma, craniopharyngioma, brain stem glioma, ependymoma, mixed glioma, optic nerve glioma, subependymoma, medulloblastoma, meningioma, metastatic brain tumor, oligodendroglioma, pituitary tumors, primitive neuroectodermal (PNET) tumor, or schwannoma. In some embodiments, the cancer is a type found more commonly in children than adults, such as brain stem glioma, craniopharyngioma, ependymoma, juvenile pilocytic astrocytoma (JPA), medulloblastoma, optic nerve glioma, pineal tumor, primitive neuroectodermal tumors (PNET), or rhabdoid tumor. In some embodiments, the patient is an adult human. In some embodiments, the patient is a child or pediatric patient.

Cancer includes, in another embodiment, without limitation, mesothelioma, hepatobilliary (hepatic and billiary duct), bone cancer, pancreatic cancer, skin cancer, cancer of the head or neck, cutaneous or intraocular melanoma, ovarian cancer, colon cancer, rectal cancer, cancer of the anal region, stomach cancer, gastrointestinal (gastric, colorectal, and duodenal), uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, testicular cancer, chronic or acute leukemia, chronic myeloid leukemia, lymphocytic lymphomas, cancer of the bladder, cancer of the kidney or ureter, renal cell carcinoma, carcinoma of the renal pelvis, non-Hodgkins's lymphoma, spinal axis tumors, brain stem glioma, pituitary adenoma, adrenocortical cancer, gall bladder cancer, multiple myeloma, cholangiocarcinoma, fibrosarcoma, neuroblastoma, retinoblastoma, or a combination of one or more of the foregoing cancers.

In some embodiments, the cancer is selected from hepatocellular carcinoma, ovarian cancer, ovarian epithelial cancer, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical adenoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from hepatocellular carcinoma (HCC), hepatoblastoma, colon cancer, rectal cancer, ovarian cancer, ovarian epithelial cancer, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical adenoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, the cancer is a solid tumor, such as a sarcoma, carcinoma, or lymphoma. Solid tumors generally comprise an abnormal mass of tissue that typically does not include cysts or liquid areas. In some embodiments, the cancer is selected from renal cell carcinoma, or kidney cancer; hepatocellular carcinoma (HCC) or hepatoblastoma, or liver cancer; melanoma; breast cancer; colorectal carcinoma, or colorectal cancer; colon cancer; rectal cancer; anal cancer; lung cancer, such as non-small cell lung cancer (NSCLC) or small cell lung cancer (SCLC); ovarian cancer, ovarian epithelial cancer, ovarian carcinoma, or fallopian tube cancer; papillary serous cystadenocarcinoma or uterine papillary serous carcinoma (UPSC); prostate cancer; testicular cancer; gallbladder cancer; hepatocholangiocarcinoma; soft tissue and bone synovial sarcoma; rhabdomyosarcoma; osteosarcoma; chondrosarcoma; Ewing sarcoma; anaplastic thyroid cancer; adrenocortical carcinoma; pancreatic cancer; pancreatic ductal carcinoma or pancreatic adenocarcinoma; gastrointestinal/stomach (GIST) cancer; lymphoma; squamous cell carcinoma of the head and neck (SCCHN); salivary gland cancer; glioma, or brain cancer; neurofibromatosis-1 associated malignant peripheral nerve sheath tumors (MPNST); Waldenstrom's macroglobulinemia; or medulloblastoma.

In some embodiments, the cancer is selected from renal cell carcinoma, hepatocellular carcinoma (HCC), hepatoblastoma, colorectal carcinoma, colorectal cancer, colon cancer, rectal cancer, anal cancer, ovarian cancer, ovarian epithelial cancer, ovarian carcinoma, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, chondrosarcoma, anaplastic thyroid cancer, adrenocortical carcinoma, pancreatic cancer, pancreatic ductal carcinoma, pancreatic adenocarcinoma, glioma, brain cancer, neurofibromatosis-1 associated malignant peripheral nerve sheath tumors (MPNST), Waldenstrom's macroglobulinemia, or medulloblastoma.

In some embodiments, the cancer is selected from hepatocellular carcinoma (HCC), hepatoblastoma, colon cancer, rectal cancer, ovarian cancer, ovarian epithelial cancer, ovarian carcinoma, fallopian tube cancer, papillary serous cystadenocarcinoma, uterine papillary serous carcinoma (UPSC), hepatocholangiocarcinoma, soft tissue and bone synovial sarcoma, rhabdomyosarcoma, osteosarcoma, anaplastic thyroid cancer, adrenocortical carcinoma, pancreatic cancer, pancreatic ductal carcinoma, pancreatic adenocarcinoma, glioma, neurofibromatosis-1 associated malignant peripheral nerve sheath tumors (MPNST), Waldenstrom's macroglobulinemia, or medulloblastoma.

In some embodiments, the cancer is hepatocellular carcinoma (HCC). In some embodiments, the cancer is hepatoblastoma. In some embodiments, the cancer is colon cancer. In some embodiments, the cancer is rectal cancer. In some embodiments, the cancer is ovarian cancer, or ovarian carcinoma. In some embodiments, the cancer is ovarian epithelial cancer. In some embodiments, the cancer is fallopian tube cancer. In some embodiments, the cancer is papillary serous cystadenocarcinoma. In some embodiments, the cancer is uterine papillary serous carcinoma (UPSC). In some embodiments, the cancer is hepatocholangiocarcinoma. In some embodiments, the cancer is soft tissue and bone synovial sarcoma. In some embodiments, the cancer is rhabdomyosarcoma. In some embodiments, the cancer is osteosarcoma. In some embodiments, the cancer is anaplastic thyroid cancer. In some embodiments, the cancer is adrenocortical carcinoma. In some embodiments, the cancer is pancreatic cancer, or pancreatic ductal carcinoma. In some embodiments, the cancer is pancreatic adenocarcinoma. In some embodiments, the cancer is glioma. In some embodiments, the cancer is malignant peripheral nerve sheath tumors (MPNST). In some embodiments, the cancer is neurofibromatosis-1 associated MPNST. In some embodiments, the cancer is Waldenstrom's macroglobulinemia. In some embodiments, the cancer is medulloblastoma.

In some embodiments, the cancer is Acute Lymphoblastic Leukemia (ALL), Acute Myeloid Leukemia (AML), Adrenocortical Carcinoma, Anal Cancer, Appendix Cancer, Atypical Teratoid/Rhabdoid Tumor, Basal Cell Carcinoma, Bile Duct Cancer, Bladder Cancer, Bone Cancer, Brain Tumor, Astrocytoma, Brain and Spinal Cord Tumor, Brain Stem Glioma, Central Nervous System Atypical Teratoid/Rhabdoid Tumor, Central Nervous System Embryonal Tumors, Breast Cancer, Bronchial Tumors, Burkitt Lymphoma, Carcinoid Tumor, Carcinoma of Unknown Primary, Central Nervous System Cancer, Cervical Cancer, Childhood Cancers, Chordoma, Chronic Lymphocytic Leukemia (CLL), Chronic Myelogenous Leukemia (CML), Chronic Myeloproliferative Disorders, Colon Cancer, Colorectal Cancer, Craniopharyngioma, Cutaneous T-Cell Lymphoma, Ductal Carcinoma In Situ (DCIS), Embryonal Tumors, Endometrial Cancer, Ependymoblastoma, Ependymoma, Esophageal Cancer, Esthesioneuroblastoma, Ewing Sarcoma, Extracranial Germ Cell Tumor, Extragonadal Germ Cell Tumor, Extrahepatic Bile Duct Cancer, Eye Cancer, Fibrous Histiocytoma of Bone, Gallbladder Cancer, Gastric Cancer, Gastrointestinal Carcinoid Tumor, Gastrointestinal Stromal Tumors (GIST), Germ Cell Tumor, Ovarian Germ Cell Tumor, Gestational Trophoblastic Tumor, Glioma, Hairy Cell Leukemia, Head and Neck Cancer, Heart Cancer, Hepatocellular Cancer, Histiocytosis, Langerhans Cell Cancer, Hodgkin Lymphoma, Hypopharyngeal Cancer, Intraocular Melanoma, Islet Cell Tumors, Kaposi Sarcoma, Kidney Cancer, Langerhans Cell Histiocytosis, Laryngeal Cancer, Leukemia, Lip and Oral Cavity Cancer, Liver Cancer, Lobular Carcinoma In Situ (LCIS), Lung Cancer, Lymphoma, AIDS-Related Lymphoma, Macroglobulinemia, Male Breast Cancer, Medulloblastoma, Medulloepithelioma, Melanoma,

Merkel Cell Carcinoma, Malignant Mesothelioma, Metastatic Squamous Neck Cancer with Occult Primary, Midline Tract Carcinoma Involving NUT Gene, Mouth Cancer, Multiple Endocrine Neoplasia Syndrome, Multiple Myeloma/Plasma Cell Neoplasm, Mycosis Fungoides, Myelodysplastic Syndrome, Myelodysplastic/Myeloproliferative Neoplasm, Chronic Myelogenous Leukemia (CML), Acute Myeloid Leukemia (AML), Myeloma, Multiple Myeloma, Chronic Myeloproliferative Disorder, Nasal Cavity Cancer, Paranasal Sinus Cancer, Nasopharyngeal Cancer, Neuroblastoma, Non-Hodgkin Lymphoma, Non-Small Cell Lung Cancer, Oral Cancer, Oral Cavity Cancer, Lip Cancer, Oropharyngeal Cancer, Osteosarcoma, Ovarian Cancer, Pancreatic Cancer, Papillomatosis, Paraganglioma, Paranasal Sinus Cancer, Nasal Cavity Cancer, Parathyroid Cancer, Penile Cancer, Pharyngeal Cancer, Pheochromocytoma, Pineal Parenchymal Tumors of Intermediate Differentiation, Pineoblastoma, Pituitary Tumor, Plasma Cell Neoplasm, Pleuropulmonary Blastoma, Breast Cancer, Primary Central Nervous System (CNS) Lymphoma, Prostate Cancer, Rectal Cancer, Renal Cell Cancer, Clear cell renal cell carcinoma, Renal Pelvis Cancer, Ureter Cancer, Transitional Cell Cancer, Retinoblastoma, Rhabdomyosarcoma, Salivary Gland Cancer, Sarcoma, Sezary Syndrome, Skin Cancer, Small Cell Lung Cancer, Small Intestine Cancer, Soft Tissue Sarcoma, Squamous Cell Carcinoma, Squamous Neck Cancer with Occult Primary, Squamous Cell Carcinoma of the Head and Neck (HNSCC), Stomach Cancer, Supratentorial Primitive Neuroectodermal Tumors, T-Cell Lymphoma, Testicular Cancer, Throat Cancer, Thymoma, Thymic Carcinoma, Thyroid Cancer, Transitional Cell Cancer of the Renal Pelvis and Ureter, Triple Negative Breast Cancer (TNBC), Gestational Trophoblastic Tumor, Unknown Primary, Unusual Cancer of Childhood, Urethral Cancer, Uterine Cancer, Uterine Sarcoma, Waldenstrom Macroglobulinemia, or Wilms Tumor.

In certain embodiments, the cancer is selected from bladder cancer, breast cancer (including TNBC), cervical cancer, colorectal cancer, chronic lymphocytic leukemia (CLL), diffuse large B-cell lymphoma (DLBCL), esophageal adenocarcinoma, glioblastoma, head and neck cancer, leukemia (acute and chronic), low-grade glioma, lung cancer (including adenocarcinoma, non-small cell lung cancer, and squamous cell carcinoma), Hodgkin's lymphoma, non-Hodgkin lymphoma (NHL), melanoma, multiple myeloma (MM), ovarian cancer, pancreatic cancer, prostate cancer, renal cancer (including renal clear cell carcinoma and kidney papillary cell carcinoma), and stomach cancer.

In some embodiments, the cancer is small cell lung cancer, non-small cell lung cancer, colorectal cancer, multiple myeloma, acute myeloid leukemia (AML), acute lymphoblastic leukemia (ALL), pancreatic cancer, liver cancer, hepatocellular cancer, neuroblastoma, other solid tumors or other hematological cancers.

In some embodiments, the cancer is small cell lung cancer, non-small cell lung cancer, colorectal cancer, multiple myeloma, or AML.

In some embodiments, the cancer is a viral-associated cancer, including human immunodeficiency virus (HIV) associated solid tumors, human papilloma virus (HPV)-16 positive incurable solid tumors, and adult T-cell leukemia, which is caused by human T-cell leukemia virus type I (HTLV-I) and is a highly aggressive form of CD4+ T-cell leukemia characterized by clonal integration of HTLV-I in leukemic cells (See https://clinicaltrials.gov/ct2/show/study/NCT02631746); as well as virus-associated tumors in gastric cancer, nasopharyngeal carcinoma, cervical cancer, vaginal cancer, vulvar cancer, squamous cell carcinoma of the head and neck, and Merkel cell carcinoma. (See https://clinicaltrials.gov/ct2/show/study/NCT02488759; see also https://clinicaltrials.gov/ct2/show/study/NCT0240886; https://clinicaltrials.gov/ct2/show/ NCT02426892)

In some embodiments, the cancer or tumor comprises any of the cancers described herein. In some embodiments, the cancer comprises melanoma cancer. In some embodiments, the cancer comprises breast cancer. In some embodiments, the cancer comprises lung cancer. In some embodiments the cancer comprises small cell lung cancer (SCLC). In some embodiments, the cancer comprises non-small cell lung cancer (NSCLC).

In some embodiments, the growth or spread of a cancer or tumor is inhibited or reduced or arrested. In some embodiments, the size (e.g., volume or mass) of the cancer or tumor is reduced by at least 5%, at least 10%, at least 25%, at least 50%, at least 75%, at least 90% or at least 99% relative to the size of the cancer or tumor prior to administration of the bicyclic peptide complex toxin conjugate or heterotandem bicyclic peptide complex specific for Nectin-4, or a pharmaceutically acceptable salt thereof, or a pharmaceutical composition thereof. In some embodiments, the quantity of the cancers or tumors in the patient is reduced by at least 5%, at least 10%, at least 25%, at least 50%, at least 75%, at least 90% or at least 99% relative to the quantity of cancers or tumors prior to administration of the bicyclic peptide complex toxin conjugate or heterotandem bicyclic peptide complex specific for Nectin-4, or a pharmaceutically acceptable salt thereof, or a pharmaceutical composition thereof.

The compounds and compositions for use of the invention can be administered using any amount and any route of administration effective for treating or lessening the severity of a cancer or tumor. The exact amount required varies from subject to subject, depending on the species, age, and general condition of the subject, the severity of the disease or condition, the particular agent, its mode of administration, and the like. The compounds and compositions are preferably formulated in dosage unit form for ease of administration and uniformity of dosage. The expression "dosage unit form" as used herein refers to a physically discrete unit of agent appropriate for the patient to be treated. It will be understood, however, that the total daily usage of the compounds and compositions is decided by the attending physician within the scope of sound medical judgment. The specific effective dose level for any particular patient or organism depends upon a

variety of factors, including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The terms "patient" or "subject," as used herein, means an animal, preferably a mammal, and most preferably a human.

Pharmaceutically acceptable compositions can be administered to humans and other animals orally, rectally, parenterally, intracisternally, intravaginally, intraperitoneally, topically (as by powders, ointments, or drops), bucally, as an oral or nasal spray, or the like, depending on the severity of the disease or disorder being treated. In certain embodiments, the bicyclic peptide complex toxin conjugate or heterotandem bicyclic peptide complex specific for Nectin-4 can be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound as described herein, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the bicyclic peptide complex toxin conjugate or heterotandem bicyclic peptide complex specific for Nectin-4 with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type can also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric

coatings and other coatings well known in the pharmaceutical formulating art. They can optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a bicyclic peptide complex toxin conjugate or heterotandem bicyclic peptide complex specific for Nectin-4 include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated. Additionally, transdermal patches may be used, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

***Co-Administration with One or More Other Therapeutic Agent(s)***

Depending upon the particular condition, or disease, to be treated, additional therapeutic agents that are normally administered to treat that condition, can also be present in the compositions administered to a patient. As used herein, additional therapeutic agents that are normally administered to treat a particular disease, or condition, are known as "appropriate for the disease, or condition, being treated."

In some embodiments, an effective amount of one or more additional therapeutic agents, such as those described herein may be co-administered simultaneously or sequentially to the patient. In some embodiments, one additional therapeutic agent is co-administered. In some embodiments, two additional therapeutic agents are co-administered. In some embodiments, the combination of the disclosed compound and the additional therapeutic agent or agents acts synergistically.

A bicyclic peptide complex toxin conjugate or heterotandem bicyclic peptide complex specific for Nectin-4, or a pharmaceutically acceptable salt thereof, or a pharmaceutical composition thereof can also be used in combination with known therapeutic processes, for example, the administration of hormones or radiation. In certain embodiments, a bicyclic peptide complex toxin conjugate or heterotandem bicyclic peptide complex specific for Nectin-4, or a pharmaceutically acceptable salt thereof, or a pharmaceutical composition thereof is used as a radiosensitizer, especially for the treatment of tumors which exhibit poor sensitivity to radiotherapy.

A bicyclic peptide complex toxin conjugate or heterotandem bicyclic peptide complex specific for Nectin-4, or a pharmaceutically acceptable salt thereof, or a pharmaceutical composition thereof can be administered alone or in combination with one or more other therapeutic compounds, possible combination therapy taking the form of fixed combinations or the administration of a bicyclic peptide complex toxin conjugate or heterotandem bicyclic peptide complex specific for Nectin-4, or a pharmaceutically acceptable salt thereof, or a pharmaceutical composition thereof and one or more other therapeutic compounds being staggered or given independently of one another, or the combined administration of fixed combinations and one or more other therapeutic compounds. A bicyclic peptide complex toxin conjugate or heterotandem bicyclic peptide complex specific for Nectin-4, or a pharmaceutically acceptable salt thereof, or a pharmaceutical composition thereof can besides, or in addition, be administered especially for tumor therapy in combination with chemotherapy, radiotherapy, immunotherapy, phototherapy, surgical intervention, or a combination of these. Long-term therapy is equally possible, as is adjuvant therapy in the context of other treatment strategies, as described above. Other possible treatments are therapy to maintain the patient's status after tumor regression, or even chemopreventive therapy, for example in patients at risk.

One or more other therapeutic agent(s) can be administered separately from a bicyclic peptide complex toxin conjugate or heterotandem bicyclic peptide complex specific for Nectin-4, or a pharmaceutically acceptable salt thereof, or

a pharmaceutical composition thereof, as part of a multiple dosage regimen. Alternatively, one or more other therapeutic agents can be part of a single dosage form, mixed together with a bicyclic peptide complex toxin conjugate or heterotandem bicyclic peptide complex specific for Nectin-4, or a pharmaceutically acceptable salt thereof, or a pharmaceutical composition thereof in a single composition. If administered as a multiple dosage regime, one or more other therapeutic agent(s) and a bicyclic peptide complex toxin conjugate or heterotandem bicyclic peptide complex specific for Nectin-4, or a pharmaceutically acceptable salt thereof, or a pharmaceutical composition thereof can be administered simultaneously, sequentially or within a period of time from one another, for example within 1, 2, 3, 4, 5, 6, 7, 8, 9, 10, 11, 12, 13, 14, 15, 16, 17, 18, 18, 20, 21, 22, 23, or 24 hours from one another. In some embodiments, one or more other therapeutic agent(s) and a bicyclic peptide complex toxin conjugate or heterotandem bicyclic peptide complex specific for Nectin-4, or a pharmaceutically acceptable salt thereof, or a pharmaceutical composition thereof are administered as a multiple dosage regimen within greater than 24 hours apart.

As used herein, the term "combination," "combined," and related terms refers to the simultaneous or sequential administration of therapeutic agents. For example, a bicyclic peptide complex toxin conjugate or heterotandem bicyclic peptide complex specific for Nectin-4, or a pharmaceutically acceptable salt thereof, or a pharmaceutical composition thereof can be administered with one or more other therapeutic agent(s) simultaneously or sequentially in separate unit dosage forms or together in a single unit dosage form. In one embodiments a single unit dosage form may comprise a bicyclic peptide complex toxin conjugate or heterotandem bicyclic peptide complex specific for Nectin-4, or a pharmaceutically acceptable salt thereof, or a pharmaceutical composition thereof, one or more other therapeutic agent(s), and a pharmaceutically acceptable carrier, adjuvant, or vehicle.

The amount of a bicyclic peptide complex toxin conjugate or heterotandem bicyclic peptide complex specific for Nectin-4, or a pharmaceutically acceptable salt thereof, or a pharmaceutical composition thereof and one or more other therapeutic agent(s) (in those compositions which comprise an additional therapeutic agent as described above) that can be combined with the carrier materials to produce a single dosage form varies depending upon the host treated and the particular mode of administration. Preferably, a composition should be formulated so that a dosage of between 0.01 - 100 mg/kg body weight/day of a bicyclic peptide complex toxin conjugate or heterotandem bicyclic peptide complex specific for Nectin-4, or a pharmaceutically acceptable salt thereof, or a pharmaceutical composition thereof can be administered.

In those compositions which comprise one or more other therapeutic agent(s), the one or more other therapeutic agent(s) and a bicyclic peptide complex toxin conjugate or heterotandem bicyclic peptide complex specific for Nectin-4, or a pharmaceutically acceptable salt thereof, or a pharmaceutical composition thereof can act synergistically. Therefore, the amount of the one or more other therapeutic agent(s) in such compositions may be less than that required in a monotherapy utilizing only that therapeutic agent. In such compositions a dosage of between 0.01 - 1,000 μg/kg body weight/day of the one or more other therapeutic agent(s) can be administered.

The amount of one or more other therapeutic agent(s) present in a composition may be no more than the amount that would normally be administered in a composition comprising that therapeutic agent as the only active agent. Preferably the amount of one or more other therapeutic agent(s) in the presently disclosed compositions ranges from about 50% to 100% of the amount normally present in a composition comprising that agent as the only therapeutically active agent. In some embodiments, one or more other therapeutic agent(s) is administered at a dosage of about 50%, about 55%, about 60%, about 65%, about 70%, about 75%, about 80%, about 85%, about 90%, or about 95% of the amount normally administered for that agent. As used herein, the phrase "normally administered" means the amount an FDA approved therapeutic agent is approved for dosing per the FDA label insert.

The bicyclic peptide complex toxin conjugate or heterotandem bicyclic peptide complex specific for Nectin-4, or a pharmaceutically acceptable salt thereof, or a pharmaceutical composition thereof, can also be incorporated into compositions for coating an implantable medical device, such as prostheses, artificial valves, vascular grafts, stents and catheters. Vascular stents, for example, have been used to overcome restenosis (re-narrowing of the vessel wall after injury). However, patients using stents or other implantable devices risk clot formation or platelet activation. These unwanted effects may be prevented or mitigated by pre-coating the device with a pharmaceutically acceptable composition comprising a kinase inhibitor. Implantable devices coated with a bicyclic peptide complex toxin conjugate or heterotandem bicyclic peptide complex specific for Nectin-4, or a pharmaceutically acceptable salt thereof, or a pharmaceutical composition thereof are disclosed.

### *Exemplary Other Therapeutic Agents*

Embodiments disclosed in this section are not claimed as such, but may be useful in combination with the claimed medical uses, which always involve the bicyclic peptide substances or compositions defined in independent claim 7.

In some embodiments, one or more other therapeutic agent is a Poly ADP ribose polymerase (PARP) inhibitor. In some embodiments, a PARP inhibitor is selected from olaparib (LYNPARZA®, AstraZeneca); rucaparib (RUBRACA®, Clovis Oncology); niraparib (ZEJULA®, Tesaro); talazoparib (MDV3800/BMN 673/LT00673, Medivation/Pfizer/Biomarin); veliparib (ABT-888, AbbVie); and BGB-290 (BeiGene, Inc.).

In some embodiments, one or more other therapeutic agent is a histone deacetylase (HDAC) inhibitor. In some embodiments, an HDAC inhibitor is selected from vorinostat (ZOLINZA®, Merck); romidepsin (ISTODAX®, Celgene); panobinostat (FARYDAK®, Novartis); belinostat (BELEODAQ®, Spectrum Pharmaceuticals); entinostat (SNDX-275, Syndax Pharmaceuticals) (NCT00866333); and chidamide (EPIDAZA®, HBI-8000, Chipscreen Biosciences, China).

In some embodiments, one or more other therapeutic agent is a CDK inhibitor, such as a CDK4/CDK6 inhibitor. In some embodiments, a CDK 4/6 inhibitor is selected from palbociclib (IBRANCE®, Pfizer); ribociclib (KISQALI®, Novartis); abemaciclib (Ly2835219, Eli Lilly); and trilaciclib (G1T28, G1 Therapeutics).

In some embodiments, one or more other therapeutic agent is a phosphatidylinositol 3 kinase (PI3K) inhibitor. In some embodiments, a PI3K inhibitor is selected from idelalisib (ZYDELIG®, Gilead), alpelisib (BYL719, Novartis), taselisib (GDC-0032, Genentech/Roche); pictilisib (GDC-0941, Genentech/Roche); copanlisib (BAY806946, Bayer); duvelisib (formerly IPI-145, Infinity Pharmaceuticals); PQR309 (Piqur Therapeutics, Switzerland); and TGR1202 (formerly RP5230, TG Therapeutics).

In some embodiments, one or more other therapeutic agent is a platinum-based therapeutic, also referred to as platins. Platins cause cross-linking of DNA, such that they inhibit DNA repair and/or DNA synthesis, mostly in rapidly reproducing cells, such as cancer cells. In some embodiments, a platinum-based therapeutic is selected from cisplatin (PLATINOL®, Bristol-Myers Squibb); carboplatin (PARAPLATIN®, Bristol-Myers Squibb; also, Teva; Pfizer); oxaliplatin (ELOXITIN® Sanofi-Aventis); nedaplatin (AQUPLA®, Shionogi), picoplatin (Poniard Pharmaceuticals); and satraplatin (JM-216, Agennix).

In some embodiments, one or more other therapeutic agent is a taxane compound, which causes disruption of microtubules, which are essential for cell division. In some embodiments, a taxane compound is selected from paclitaxel (TAXOL®, Bristol-Myers Squibb), docetaxel (TAXOTERE®, Sanofi-Aventis; DOCEFREZ®, Sun Pharmaceutical), albumin-bound paclitaxel (ABRAXANE®; Abraxis/Celgene), cabazitaxel (JEVTANA®, Sanofi-Aventis), and SID530 (SK Chemicals, Co.) (NCT00931008).

In some embodiments, one or more other therapeutic agent is a nucleoside inhibitor, or a therapeutic agent that interferes with normal DNA synthesis, protein synthesis, cell replication, or will otherwise inhibit rapidly proliferating cells.

In some embodiments, a nucleoside inhibitor is selected from trabectedin (guanidine alkylating agent, YONDELIS®, Janssen Oncology), mechlorethamine (alkylating agent, VALCHLOR®, Aktelion Pharmaceuticals); vincristine (ONCOVIN®, Eli Lilly; VINCASAR®, Teva Pharmaceuticals; MARQIBO®, Talon Therapeutics); temozolomide (prodrug to alkylating agent 5-(3-methyltriazen-1-yl)-imidazole-4-carboxamide (MTIC) TEMODAR®, Merck); cytarabine injection (ara-C, antimetabolic cytidine analog, Pfizer); lomustine (alkylating agent, CEENU®, Bristol-Myers Squibb; GLEOSTINE®, NextSource Biotechnology); azacitidine (pyrimidine nucleoside analog of cytidine, VIDAZA®, Celgene); omacetaxine mepesuccinate (cephalotaxine ester) (protein synthesis inhibitor, SYNRIBO®; Teva Pharmaceuticals); asparaginase *Erwinia chrysanthemi* (enzyme for depletion of asparagine, ELSPAR®, Lundbeck; ERWINAZE®, EUSA Pharma); eribulin mesylate (microtubule inhibitor, tubulin-based antimitotic, HALAVEN®, Eisai); cabazitaxel (microtubule inhibitor, tubulin-based antimitotic, JEVTANA®, Sanofi-Aventis); capacetrine (thymidylate synthase inhibitor, XELODA®, Genentech); bendamustine (bifunctional mechlorethamine derivative, believed to form interstrand DNA cross-links, TREANDA®, Cephalon/Teva); ixabepilone (semi-synthetic analog of epothilone B, microtubule inhibitor, tubulin-based antimitotic, IXEMPRA®, Bristol-Myers Squibb); nelarabine (prodrug of deoxyguanosine analog, nucleoside metabolic inhibitor, ARRANON®, Novartis); clorafabine (prodrug of ribonucleotide reductase inhibitor, competitive inhibitor of deoxycytidine, CLOLAR®, Sanofi-Aventis); and trifluridine and tipiracil (thymidine-based nucleoside analog and thymidine phosphorylase inhibitor, LONSURF®, Taiho Oncology).

In some embodiments, one or more other therapeutic agent is a kinase inhibitor or VEGF-R antagonist. Approved VEGF inhibitors and kinase inhibitors include: bevacizumab (AVASTIN®, Genentech/Roche) an anti-VEGF monoclonal antibody; ramucirumab (CYRAMZA®, Eli Lilly), an anti-VEGFR-2 antibody and ziv-aflibercept, also known as VEGF Trap (ZALTRAP®; Regeneron/Sanofi). VEGFR inhibitors, such as regorafenib (STIVARGA®, Bayer); vandetanib (CAPRELSA®, AstraZeneca); axitinib (INLYTA®, Pfizer); and lenvatinib (LENVIMA®, Eisai); Raf inhibitors, such as sorafenib (NEXAVAR®, Bayer AG and Onyx); dabrafenib (TAFINLAR®, Novartis); and vemurafenib (ZELBORAF®, Genentech/Roche); MEK inhibitors, such as cobimetanib (COTELLIC®, Exelexis/Genentech/Roche); trametinib (MEKINIST®, Novartis); Bcr-Abl tyrosine kinase inhibitors, such as imatinib (GLEEVEC®, Novartis); nilotinib (TASIGNA®, Novartis); dasatinib (SPRYCEL®, BristolMyersSquibb); bosutinib (BOSULIF®, Pfizer); and ponatinib (INCLUSIG®, Ariad Pharmaceuticals); Her2 and EGFR inhibitors, such as gefitinib (IRESSA®, AstraZeneca); erlotinib (TARCEEVA®, Genentech/Roche/Astellas); lapatinib (TYKERB®, Novartis); afatinib (GILOTRIF®, Boehringer Ingelheim); osimertinib (targeting activated EGFR, TAGRISSO®, AstraZeneca); and brigatinib (ALUNBRIG®, Ariad Pharmaceuticals); c-Met and VEGFR2 inhibitors, such as cabozanitib (COMETRIQ®, Exelexis); and multikinase inhibitors, such as sunitinib (SUTENT®, Pfizer); pazopanib (VOTRIENT®, Novartis); ALK inhibitors, such as crizotinib (XALKORI®, Pfizer); ceritinib (ZYKADIA®, Novartis); and alectinib (ALECENZa®, Genentech/Roche); Bruton's tyrosine kinase inhibitors, such as ibrutinib (IMBRUVICA®, Pharmacyclics/Janssen); and Flt3 receptor inhibitors, such as midostaurin (RYDAPT®, Novartis).

Other kinase inhibitors and VEGF-R antagonists that are in development include tivozanib (Aveo Pharmaecuticals); vatalanib (Bayer/Novartis); lucitanib (Clovis Oncology); dovitinib (TKI258, Novartis); Chiauanib (Chipscreen Biosciences); CEP-11981 (Cephalon); linifanib (Abbott Laboratories); neratinib (HKI-272, Puma Biotechnology); radotinib (SUPECT®, IY5511, Il-Yang Pharmaceuticals, S. Korea); ruxolitinib (JAKAFI®, Incyte Corporation); PTC299 (PTC Therapeutics); CP-547,632 (Pfizer); foretinib (Exelexis, GlaxoSmithKline); quizartinib (Daiichi Sankyo) and motesanib (Amgen/Takeda).

In some embodiments, one or more other therapeutic agent is an mTOR inhibitor, which inhibits cell proliferation, angiogenesis and glucose uptake. In some embodiments, an mTOR inhibitor is everolimus (AFINITOR®, Novartis); temsirolimus (TORISEL®, Pfizer); and sirolimus (RAPAMUNE®, Pfizer).

In some embodiments, one or more other therapeutic agent is a proteasome inhibitor. Approved proteasome inhibitors include bortezomib (VELCADE®, Takeda); carfilzomib (KYPROLIS®, Amgen); and ixazomib (NINLARO®, Takeda).

In some embodiments, one or more other therapeutic agent is a growth factor antagonist, such as an antagonist of platelet-derived growth factor (PDGF), or epidermal growth factor (EGF) or its receptor (EGFR). Approved PDGF antagonists include olaratumab (LARTRUVO®; Eli Lilly). Approved EGFR antagonists include cetuximab (ERBITUX®, Eli Lilly); necitumumab (PORTRAZZA®, Eli Lilly), panitumumab (VECTIBIX®, Amgen); and osimertinib (targeting activated EGFR, TAGRISSO®, AstraZeneca).

In some embodiments, one or more other therapeutic agent is an aromatase inhibitor. In some embodiments, an aromatase inhibitor is selected from exemestane (AROMASIN®, Pfizer); anastazole (ARIMIDEX®, AstraZeneca) and letrozole (FEMARA®, Novartis).

In some embodiments, one or more other therapeutic agent is an antagonist of the hedgehog pathway. Approved hedgehog pathway inhibitors include sonidegib (ODOMZO®, Sun Pharmaceuticals); and vismodegib (ERIVEDGE®, Genentech), both for treatment of basal cell carcinoma.

In some embodiments, one or more other therapeutic agent is a folic acid inhibitor. Approved folic acid inhibitors include pemetrexed (ALIMTA®, Eli Lilly).

In some embodiments, one or more other therapeutic agent is a CC chemokine receptor 4 (CCR4) inhibitor. CCR4 inhibitors being studied include mogamulizumab (POTELIGEO®, Kyowa Hakko Kirin, Japan).

In some embodiments, one or more other therapeutic agent is an isocitrate dehydrogenase (IDH) inhibitor. IDH inhibitors being studied include AG120 (Celgene; NCT02677922); AG221 (Celgene, NCT02677922; NCT02577406); BAY1436032 (Bayer, NCT02746081); IDH305 (Novartis, NCT02987010).

In some embodiments, one or more other therapeutic agent is an arginase inhibitor. Arginase inhibitors being studied include AEB1102 (pegylated recombinant arginase, Aeglea Biotherapeutics), which is being studied in Phase 1 clinical trials for acute myeloid leukemia and myelodysplastic syndrome (NCT02732184) and solid tumors (NCT02561234); and CB-1158 (Calithera Biosciences).

In some embodiments, one or more other therapeutic agent is a glutaminase inhibitor. Glutaminase inhibitors being studied include CB-839 (Calithera Biosciences).

In some embodiments, one or more other therapeutic agent is an antibody that binds to tumor antigens, that is, proteins expressed on the cell surface of tumor cells. Approved antibodies that bind to tumor antigens include rituximab (RITUXAN®, Genentech/BiogenIdec); ofatumumab (anti-CD20, ARZERRA®, GlaxoSmithKline); obinutuzumab (anti-CD20, GAZYVA®, Genentech), ibritumomab (anti-CD20 and Yttrium-90, ZEVALIN®, Spectrum Pharmaceuticals); daratumumab (anti-CD38, DARZALEX®, Janssen Biotech), dinutuximab (anti-glycolipid GD2, UNITUXIN®, United Therapeutics); trastuzumab (anti-HER2, HERCEPTIN®, Genentech); ado-trastuzumab emtansine (anti-HER2, fused to emtansine, KADCYLA®, Genentech); and pertuzumab (anti-HER2, PERJETA®, Genentech); and brentuximab vedotin (anti-CD30-drug conjugate, ADCETRIS®, Seattle Genetics).

In some embodiments, one or more other therapeutic agent is a topoisomerase inhibitor. Approved topoisomerase inhibitors include irinotecan (ONIVYDE®, Merrimack Pharmaceuticals); topotecan (HYCAMTIN®, GlaxoSmithKline). Topoisomerase inhibitors being studied include pixantrone (PIXUVRI®, CTI Biopharma).

In some embodiments, one or more other therapeutic agent is an inhibitor of anti-apoptotic proteins, such as BCL-2. Approved anti-apoptotics include venetoclax (VENCLEXTA®, AbbVie/Genentech); and blinatumomab (BLINCYTO®, Amgen). Other therapeutic agents targeting apoptotic proteins which have undergone clinical testing include navitoclax (ABT-263, Abbott), a BCL-2 inhibitor (NCT02079740).

In some embodiments, one or more other therapeutic agent is an androgen receptor inhibitor. Approved androgen receptor inhibitors include enzalutamide (XTANDI®, Astellas/Medivation); approved inhibitors of androgen synthesis include abiraterone (ZYTIGA®, Centocor/Ortho); approved antagonist of gonadotropin-releasing hormone (GnRH) receptor (degaralix, FIRMAGON®, Ferring Pharmaceuticals).

In some embodiments, one or more other therapeutic agent is a selective estrogen receptor modulator (SERM), which interferes with the synthesis or activity of estrogens. Approved SERMs include raloxifene (EVISTA®, Eli Lilly).

In some embodiments, one or more other therapeutic agent is an inhibitor of bone resorption. An approved therapeutic which inhibits bone resorption is Denosumab (XGEVA®, Amgen), an antibody that binds to RANKL, prevents

binding to its receptor RANK, found on the surface of osteoclasts, their precursors, and osteoclast-like giant cells, which mediates bone pathology in solid tumors with osseous metastases. Other approved therapeutics that inhibit bone resorption include bisphosphonates, such as zoledronic acid (ZOMETA®, Novartis).

In some embodiments, one or more other therapeutic agent is an inhibitor of interaction between the two primary p53 suppressor proteins, MDMX and MDM2. Inhibitors of p53 suppression proteins being studied include ALRN-6924 (Aileron), a stapled peptide that equipotently binds to and disrupts the interaction of MDMX and MDM2 with p53. ALRN-6924 is currently being evaluated in clinical trials for the treatment of AML, advanced myelodysplastic syndrome (MDS) and peripheral T-cell lymphoma (PTCL) (NCT02909972; NCT02264613).

In some embodiments, one or more other therapeutic agent is an inhibitor of transforming growth factor-beta (TGF-beta or TGFβ). Inhibitors of TGF-beta proteins being studied include NIS793 (Novartis), an anti-TGF-beta antibody being tested in the clinic for treatment of various cancers, including breast, lung, hepatocellular, colorectal, pancreatic, prostate and renal cancer (NCT 02947165). In some embodiments, the inhibitor of TGF-beta proteins is fresolimumab (GC1008; Sanofi-Genzyme), which is being studied for melanoma (NCT00923169); renal cell carcinoma (NCT00356460); and non-small cell lung cancer (NCT02581787). Additionally, in some embodiments, the additional therapeutic agent is a TGF-beta trap, such as described in Connolly et al. (2012) Int'l J. Biological Sciences 8:964-978. One therapeutic compound currently in clinical trials for treatment of solid tumors is M7824 (Merck KgaA - formerly MSB0011459X), which is a bispecific, anti-PD-L1/TGFI3 trap compound (NCT02699515); and (NCT02517398). M7824 is comprised of a fully human IgG1 antibody against PD-L1 fused to the extracellular domain of human TGF-beta receptor II, which functions as a TGFβ"trap."

In some embodiments, one or more other therapeutic agent is selected from glembatumumab vedotin-monomethyl auristatin E (MMAE) (Celldex), an anti-glycoprotein NMB (gpNMB) antibody (CR011) linked to the cytotoxic MMAE. gpNMB is a protein overexpressed by multiple tumor types associated with cancer cells' ability to metastasize.

In some embodiments, one or more other therapeutic agents is an antiproliferative compound. Such antiproliferative compounds include, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, TEMODAL CNF1010, CNF2024, CNF1010 from Conforma Therapeutics; temozolomide (TEMODAL®); kinesin spindle protein inhibitors, such as SB715992 or SB743921 from GlaxoSmithKline, or pentamidine/-chlorpromazine from CombinatoRx; MEK inhibitors such as ARRY142886 from Array BioPharma, $AZd_6244$ from AstraZeneca, PD181461 from Pfizer and leucovorin.

The term "aromatase inhibitor" as used herein relates to a compound which inhibits estrogen production, for instance, the conversion of the substrates androstenedione and testosterone to estrone and estradiol, respectively. The term includes, but is not limited to steroids, especially atamestane, exemestane and formestane and, in particular, non-steroids, especially aminoglutethimide, roglethimide, pyridoglutethimide, trilostane, testolactone, ketokonazole, vorozole, fadrozole, anastrozole and letrozole. Exemestane is marketed under the trade name AROMASIN™. Formestane is marketed under the trade name LENTARON™. Fadrozole is marketed under the trade name AFEMA™. Anastrozole is marketed under the trade name ARIMIDEX™. Letrozole is marketed under the trade names FEMARA™ or FEMAr™. Aminoglutethimide is marketed under the trade name ORIMETEN™. A combination comprising a chemotherapeutic agent which is an aromatase inhibitor is particularly useful for the treatment of hormone receptor positive tumors, such as breast tumors.

The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen is marketed under the trade name NOLVADEX™. Raloxifene hydrochloride is marketed under the trade name EVISTA™. Fulvestrant can be administered under the trade name FASLODEX™. A combination comprising a chemotherapeutic agent which is an antiestrogen is particularly useful for the treatment of estrogen receptor positive tumors, such as breast tumors.

The term "anti-androgen" as used herein relates to any substance which is capable of inhibiting the biological effects of androgenic hormones and includes, but is not limited to, bicalutamide (CASODEX™). The term "gonadorelin agonist" as used herein includes, but is not limited to abarelix, goserelin, and goserelin acetate. Goserelin can be administered under the trade name ZOLADEX™.

The term "topoisomerase I inhibitor" as used herein includes, but is not limited to topotecan, gimatecan,

irinotecan, camptothecian and its analogues, 9-nitrocamptothecin and the macromolecular camptothecin conjugate PNU-166148. Irinotecan can be administered, *e.g.*, in the form as it is marketed, *e.g.*, under the trademark CAMPTOSAR™, Topotecan is marketed under the trade name HYCAMPTIN™.

The term "topoisomerase II inhibitor" as used herein includes, but is not limited to the anthracyclines such as doxorubicin (including liposomal formulation, such as CAELYX™), daunorubicin, epirubicin, idarubicin and nemorubicin, the anthraquinones mitoxantrone and losoxantrone, and the podophillotoxines etoposide and teniposide. Etoposide is marketed under the trade name ETOPOPHOS™. Teniposide is marketed under the trade name VM 26-Bristol Doxorubicin is marketed under the trade name ACRIBLASTIN™ or ADRIAMYCIN™, Epirubicin is marketed under the trade name FARMORUBICIN™. Idarubicin is marketed. under the trade name ZAVEDOS™. Mitoxantrone is marketed under the trade name NOVANTRON™.

The term "microtubule active agent" relates to microtubule stabilizing, microtubule destabilizing compounds and microtublin polymerization inhibitors including, but not limited to taxanes, such as paclitaxel and docetaxel; vinca alkaloids, such as vinblastine or vinblastine sulfate, vincristine or vincristine sulfate, and vinorelbine; discodermolides; cochicine and epothilones and derivatives thereof. Paclitaxel is marketed under the trade name TAXOL™. Docetaxel is marketed under the trade name TAXOTERE™. Vinblastine sulfate is marketed under the trade name VINBLASTIN R.P™. Vincristine sulfate is marketed under the trade name FARMISTIN™.

The term "alkylating agent" as used herein includes, but is not limited to, cyclophosphamide, ifosfamide, melphalan or nitrosourea (BCNU or Gliadel). Cyclophosphamide is marketed under the trade name CYCLOSTIN™. Ifosfamide is marketed under the trade name HOLOXAN™.

The term "histone deacetylase inhibitors" or "HDAC inhibitors" relates to compounds which inhibit the histone deacetylase and which possess antiproliferative activity. This includes, but is not limited to, suberoylanilide hydroxamic acid (SAHA).

The term "antineoplastic antimetabolite" includes, but is not limited to, 5-fluorouracil or 5-FU, capecitabine, gemcitabine, DNA demethylating compounds, such as 5-azacytidine and decitabine, methotrexate and edatrexate, and folic acid antagonists such as pemetrexed. Capecitabine is marketed under the trade name XELODA™. Gemcitabine is marketed under the trade name GEMZAR™.

The term "platin compound" as used herein includes, but is not limited to, carboplatin, cis-platin, cisplatinum and oxaliplatin. Carboplatin can be administered, *e.g.*, in the form as it is marketed, *e.g.,* under the trademark CARBOPLAT™. Oxaliplatin can be administered, *e.g.,* in the form as it is marketed, *e.g.* under the trademark ELOXATIN™.

The term "compounds targeting/decreasing a protein or lipid kinase activity; or a protein or lipid phosphatase activity; or further anti-angiogenic compounds" as used herein includes, but is not limited to, protein tyrosine kinase and/or serine and/or threonine kinase inhibitors or lipid kinase inhibitors, such as a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, such as an N-phenyl-2-pyrimidine-amine derivative, such as imatinib, SU101, SU6668 and GFB-111; b) compounds targeting, decreasing or inhibiting the activity of the fibroblast growth factor-receptors (FGFR); c) compounds targeting, decreasing or inhibiting the activity of the insulin-like growth factor receptor I (IGF-IR), such as compounds which target, decrease or inhibit the activity of IGF-IR, especially compounds which inhibit the kinase activity of IGF-I receptor, or antibodies that target the extracellular domain of IGF-I receptor or its growth factors; d) compounds targeting, decreasing or inhibiting the activity of the Trk receptor tyrosine kinase family, or ephrin B4 inhibitors; e) compounds targeting, decreasing or inhibiting the activity of the Axl receptor tyrosine kinase family; f) compounds targeting, decreasing or inhibiting the activity of the Ret receptor tyrosine kinase; g) compounds targeting, decreasing or inhibiting the activity of the Kit/SCFR receptor tyrosine kinase, such as imatinib; h) compounds targeting, decreasing or inhibiting the activity of the C-kit receptor tyrosine kinases, which are part of the PDGFR family, such as compounds which target, decrease or inhibit the activity of the c-Kit receptor tyrosine kinase family, especially compounds which inhibit the c-Kit receptor, such as imatinib; i) compounds targeting, decreasing or inhibiting the activity of members of the c-Abl family, their gene-fusion products (*e.g.*, BCR-Abl kinase) and mutants, such as compounds which target decrease or inhibit the activity of c-Abl family members and their gene fusion products, such as an N-phenyl-2-pyrimidine-amine derivative, such as imatinib or nilotinib (AMN107); PD180970; AG957; NSC 680410; PD173955 from ParkeDavis; or dasatinib (BMS-354825); j) compounds targeting, decreasing or inhibiting the activity of members of the protein kinase C (PKC) and Raf family of serine/threonine kinases, members of the MEK, SRC, JAK/pan-JAK, FAK, PDK1, PKB/Akt, Ras/MAPK, PI3K, SYK, TYK2, BTK and TEC family, and/or members of the cyclin-dependent kinase family (CDK) including staurosporine derivatives, such as midostaurin; examples of further compounds include UCN-01, safingol, BAY 43-9006, Bryostatin 1, Perifosine; Ilmofosine; RO 318220 and RO 320432; GO 6976; Isis 3521; LY333531/LY379196; isochinoline compounds; FTIs; PD184352 or QAN697 (a P13K inhibitor) or AT7519 (CDK inhibitor); k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC™) or tyrphostin such as Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556,

AG957 and adaphostin (4-{[(2,5- dihydroxyphenyl)methyl]amino}-benzoic acid adamantyl ester; NSC 680410, adaphostin); 1) compounds targeting, decreasing or inhibiting the activity of the epidermal growth factor family of receptor tyrosine kinases ($EGFR_1$ ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, such as EGF receptor, ErbB2, ErbB3 and ErbB4 or bind to EGF or EGF related ligands, CP 358774, ZD 1839, ZM 105180; trastuzumab (HERCEPTIN™), cetuximab (ERBITUX™), Iressa, Tarceva, OSI-774, Cl-1033, EKB-569, GW-2016, E1.1, E2.4, E2.5, E6.2, E6.4, E2.11, E6.3 or E7.6.3, and 7H-pyrrolo-[2,3-d]pyrimidine derivatives; m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF, n) compounds targeting, decreasing or inhibiting the kinase activity of one or more JAK family members (JAK1/JAK2/-JAK3/TYK2 and/or pan-JAK), including but not limited to PRT-062070, SB-1578, baricitinib, pacritinib, momelotinib, VX-509, AZD-1480, TG-101348, tofacitinib, and ruxolitinib; o) compounds targeting, decreasing or inhibiting the kinase activity of PI3 kinase (PI3K) including but not limited to ATU-027, SF-1126, DS-7423, PBI-05204, GSK-2126458, ZSTK-474, buparlisib, pictrelisib, PF-4691502, BYL-719, dactolisib, XL-147, XL-765, and idelalisib; and; and q) compounds targeting, decreasing or inhibiting the signaling effects of hedgehog protein (Hh) or smoothened receptor (SMO) pathways, including but not limited to cyclopamine, vismodegib, itraconazole, erismodegib, and IPI-926 (saridegib).

The term "PI3K inhibitor" as used herein includes, but is not limited to compounds having inhibitory activity against one or more enzymes in the phosphatidylinositol-3-kinase family, including, but not limited to PI3Kα, PI3Kγ, PI3Kδ, PI3Kβ, PI3K-C2α, PI3K-C2β, PI3K-C2y, Vps34, p110-α, p110-β, p110-γ, p110-δ, p85-α, p85-β, p55-y, p150, p101, and p87. Examples of PI3K inhibitors include but are not limited to ATU-027, SF-1126, DS-7423, PBI-05204, GSK-2126458, ZSTK-474, buparlisib, pictrelisib, PF-4691502, BYL-719, dactolisib, XL-147, XL-765, and idelalisib.

The term "Bcl-2 inhibitor" as used herein includes, but is not limited to compounds having inhibitory activity against B-cell lymphoma 2 protein (Bcl-2), including but not limited to ABT-199, ABT-731, ABT-737, apogossypol, Ascenta's pan-Bcl-2 inhibitors, curcumin (and analogs thereof), dual Bcl-2/Bcl-xL inhibitors (Infinity Pharmaceuticals/-Novartis Pharmaceuticals), Genasense (G3139), HA14-1 (and analogs thereof; see WO2008118802), navitoclax (and analogs thereof, see US7390799), NH-1 (Shenayng Pharmaceutical University), obatoclax (and analogs thereof, see WO2004106328), S-001 (Gloria Pharmaceuticals), TW series compounds (Univ. of Michigan), and venetoclax. In some embodiments the Bcl-2 inhibitor is a small molecule therapeutic. In some embodiments the Bcl-2 inhibitor is a peptidomimetic.

The term "BTK inhibitor" as used herein includes, but is not limited to compounds having inhibitory activity against Bruton's Tyrosine Kinase (BTK), including, but not limited to AVL-292 and ibrutinib.

The term "SYK inhibitor" as used herein includes, but is not limited to compounds having inhibitory activity against spleen tyrosine kinase (SYK), including but not limited to PRT-062070, R-343, R-333, Excellair, PRT-062607, and fostamatinib.

Further examples of BTK inhibitory compounds, and conditions treatable by such compounds in combination with bicyclic peptide complex toxin conjugate or heterotandem bicyclic peptide complex specific for Nectin-4, or a pharmaceutically acceptable salt thereof, or a pharmaceutical composition thereof can be found in WO2008039218 and WO2011090760.

Further examples of SYK inhibitory compounds, and conditions treatable by such compounds in combination with bicyclic peptide complex toxin conjugate or heterotandem bicyclic peptide complex specific for Nectin-4, or a pharmaceutically acceptable salt thereof, or a pharmaceutical composition thereof can be found in WO2003063794, WO2005007623, and WO2006078846.

Further examples of PI3K inhibitory compounds, and conditions treatable by such compounds in combination with bicyclic peptide complex toxin conjugate or heterotandem bicyclic peptide complex specific for Nectin-4, or a pharmaceutically acceptable salt thereof, or a pharmaceutical composition thereof can be found in WO2004019973, WO2004089925, WO2007016176, US8138347, WO2002088112, WO2007084786, WO2007129161, WO2006122806, WO2005113554, and WO2007044729.

Further examples of JAK inhibitory compounds, and conditions treatable by such compounds in combination with bicyclic peptide complex toxin conjugate or heterotandem bicyclic peptide complex specific for Nectin-4, or a pharmaceutically acceptable salt thereof, or a pharmaceutical composition thereof can be found in WO2009114512, WO2008109943, WO2007053452, WO2000142246, and WO2007070514.

Further anti-angiogenic compounds include compounds having another mechanism for their activity, *e.g.,* unrelated to protein or lipid kinase inhibition *e.g.,* thalidomide (THALOMID™) and TNP-470.

Examples of proteasome inhibitors useful for use in combination with bicyclic peptide complex toxin conjugate or heterotandem bicyclic peptide complex specific for Nectin-4, or a pharmaceutically acceptable salt thereof, or a pharmaceutical composition thereof include, but are not limited to bortezomib, disulfiram, epigallocatechin-3-gallate (EGCG), salinosporamide A, carfilzomib, ONX-0912, CEP-18770, and MLN9708.

Compounds which target, decrease or inhibit the activity of a protein or lipid phosphatase are *e.g*. inhibitors of phosphatase 1, phosphatase 2A, or CDC25, such as okadaic acid or a derivative thereof.

Compounds which induce cell differentiation processes include, but are not limited to, retinoic acid, α- γ- or δ-tocopherol or α- γ- or δ-tocotrienol.

The term cyclooxygenase inhibitor as used herein includes, but is not limited to, Cox-2 inhibitors, 5-alkyl substituted 2-arylaminophenylacetic acid and derivatives, such as celecoxib (CELEBREX™), rofecoxib (VIOXX™), etoricoxib, valdecoxib or a 5-alkyl-2-arylaminophenylacetic acid, such as 5-methyl-2-(2'-chloro-6'-fluoroanilino)phenyl acetic acid, lumiracoxib.

The term "bisphosphonates" as used herein includes, but is not limited to, etridonic, clodronic, tiludronic, pamidronic, alendronic, ibandronic, risedronic and zoledronic acid. Etridonic acid is marketed under the trade name DIDRONEL™. Clodronic acid is marketed under the trade name BONEFOS™. Tiludronic acid is marketed under the trade name Skelid™. Pamidronic acid is marketed under the trade name AREDIA™. Alendronic acid is marketed under the trade name FOSAMAX™. Ibandronic acid is marketed under the trade name BONDRANAT™. Risedronic acid is marketed under the trade name ACTONEL™. Zoledronic acid is marketed under the trade name ZOMETA™. The term "mTOR inhibitors" relates to compounds which inhibit the mammalian target of rapamycin (mTOR) and which possess antiproliferative activity such as sirolimus (RAPAMUNE®), everolimus (CERTICAN™), CCI-779 and ABT578.

The term "heparanase inhibitor" as used herein refers to compounds which target, decrease or inhibit heparin sulfate degradation. The term includes, but is not limited to, PI-88. The term "biological response modifier" as used herein refers to a lymphokine or interferons.

The term "inhibitor of Ras oncogenic isoforms", such as H-Ras, K-Ras, or N-Ras, as used herein refers to compounds which target, decrease or inhibit the oncogenic activity of Ras; for example, a "farnesyl transferase inhibitor" such as L-744832, DK8G557 or R115777 (ZARNESTRA™). The term "telomerase inhibitor" as used herein refers to compounds which target, decrease or inhibit the activity of telomerase. Compounds which target, decrease or inhibit the activity of telomerase are especially compounds which inhibit the telomerase receptor, such as telomestatin.

The term "methionine aminopeptidase inhibitor" as used herein refers to compounds which target, decrease or inhibit the activity of methionine aminopeptidase. Compounds which target, decrease or inhibit the activity of methionine aminopeptidase include, but are not limited to, bengamide or a derivative thereof.

The term "proteasome inhibitor" as used herein refers to compounds which target, decrease or inhibit the activity of the proteasome. Compounds which target, decrease or inhibit the activity of the proteasome include, but are not limited to, Bortezomib (VELCADE™) and MLN 341.

The term "matrix metalloproteinase inhibitor" or ("MMP" inhibitor) as used herein includes, but is not limited to, collagen peptidomimetic and nonpeptidomimetic inhibitors, tetracycline derivatives, *e.g.*, hydroxamate peptidomimetic inhibitor batimastat and its orally bioavailable analogue marimastat (BB-2516), prinomastat (AG3340), metastat (NSC 683551) BMS-279251, BAY 12-9566, TAA211, MMI270B or AAJ996.

The term "compounds used in the treatment of hematologic malignancies" as used herein includes, but is not limited to, FMS-like tyrosine kinase inhibitors, which are compounds targeting, decreasing or inhibiting the activity of FMS-like tyrosine kinase receptors (Flt-3R); interferon, 1-β-D-arabinofuransylcytosine (ara-c) and bisulfan; and ALK inhibitors, which are compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, such as PKC412, midostaurin, a staurosporine derivative, SU11248 and MLN518.

The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90, such as 17-allylamino,17-demethoxygeldanamycin (17AAG), a geldanamycin derivative; other geldanamycin related compounds; radicicol and HDAC inhibitors.

The term "antiproliferative antibodies" as used herein includes, but is not limited to, trastuzumab (HERCEPTIN™), Trastuzumab-DM1, erbitux, bevacizumab (AVASTIN™), rituximab (RITUXAN®), PRO64553 (anti-CD40) and 2C4 Antibody. By antibodies is meant intact monoclonal antibodies, polyclonal antibodies, multispecific antibodies formed from at least 2 intact antibodies, and antibodies fragments so long as they exhibit the desired biological activity.

For the treatment of acute myeloid leukemia (AML), bicyclic peptide complex toxin conjugate or heterotandem bicyclic peptide complex specific for Nectin-4, or a pharmaceutically acceptable salt thereof, or a pharmaceutical composition thereof can be used in combination with standard leukemia therapies, especially in combination with therapies used for the treatment of AML. In particular, bicyclic peptide complex toxin conjugate or heterotandem bicyclic peptide complex specific for Nectin-4, or a pharmaceutically acceptable salt thereof, or a pharmaceutical composition thereof can be administered in combination with, for example, farnesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

Other anti-leukemic compounds include, for example, Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate. Compounds which target, decrease or inhibit activity of histone deacetylase (HDAC) inhibitors such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in US 6,552,065 including, but not limited to, N-hydroxy-3-[4-[[[2-(2-methyl-1H-indol-3-yl)-ethyl]-amino]methyl]phenyl]-2E-2-propenamide, or a pharmaceutically acceptable salt thereof and N-hydroxy-3-[4-[(2-hydroxyethyl){2-(1H-indol-3-yl)ethyl]-amino]methyl]phenyl]-2E-2- propenamide, or a pharmaceutically acceptable salt thereof, especially the lactate salt. Somatostatin receptor antagonists as used herein refer to compounds which target, treat or inhibit the somatostatin receptor such as octreotide, and SOM230. Tumor cell damaging approaches refer to approaches such as ionizing radiation. The term "ionizing radiation" referred to above and hereinafter means ionizing radiation that occurs as either electromagnetic rays (such as X-rays and gamma rays) or particles (such as alpha and beta particles). Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol. 1, pp. 248-275 (1993).

Also included are EDG binders and ribonucleotide reductase inhibitors. The term "EDG binders" as used herein refers to a class of immunosuppressants that modulates lymphocyte recirculation, such as FTY720. The term "ribonucleotide reductase inhibitors" refers to pyrimidine or purine nucleoside analogs including, but not limited to, fludarabine and/or cytosine arabinoside (ara-C), 6-thioguanine, 5-fluorouracil, cladribine, 6-mercaptopurine (especially in combination with ara-C against ALL) and/or pentostatin. Ribonucleotide reductase inhibitors are especially hydroxyurea or 2-hydroxy-1H-isoindole-1,3-dione derivatives.

Also included are in particular those compounds, proteins or monoclonal antibodies of VEGF such as 1-(4-chloroanilino)-4-(4-pyridylmethyl)phthalazine or a pharmaceutically acceptable salt thereof, 1-(4-chloroanilino)-4-(4-pyridylmethyl)phthalazine succinate; ANGIOSTATIN™; ENDOSTATIN™; anthranilic acid amides; ZD4190; $Zd_6474$; SU5416; SU6668; bevacizumab; or anti-VEGF antibodies or anti-VEGF receptor antibodies, such as rhuMAb and RHUFab, VEGF aptamer such as Macugon; FLT-4 inhibitors, FLT-3 inhibitors, VEGFR-2 IgGI antibody, Angiozyme (RPI 4610) and Bevacizumab (AVASTIN™).

Photodynamic therapy as used herein refers to therapy which uses certain chemicals known as photosensitizing compounds to treat or prevent cancers. Examples of photodynamic therapy include treatment with compounds, such as VISUDYNE™ and porfimer sodium.

Angiostatic steroids as used herein refers to compounds which block or inhibit angiogenesis, such as, *e.g.*, anecortave, triamcinolone, hydrocortisone, 11-$\alpha$-epihydrocotisol, cortexolone, 17$\alpha$-hydroxyprogesterone, corticosterone, desoxycorticosterone, testosterone, estrone and dexamethasone.

Implants containing corticosteroids refers to compounds, such as fluocinolone and dexamethasone.

Other chemotherapeutic compounds include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The structure of the active compounds identified by code numbers, generic or trade names may be taken from the actual edition of the standard compendium "The Merck Index" or from databases, *e.g.,* Patents International (*e.g.,* IMS World Publications).

*Exemplary Immuno-Oncology agents*

In some embodiments, one or more other therapeutic agent is an immuno-oncology agent. As used herein, the term "an immuno-oncology agent" refers to an agent which is effective to enhance, stimulate, and/or up-regulate immune responses in a subject. In some embodiments, the administration of an immuno-oncology agent with a bicyclic peptide complex toxin conjugate or heterotandem bicyclic peptide complex specific for Nectin-4, or a pharmaceutically acceptable salt thereof, or a pharmaceutical composition thereof has a synergic effect in treating a cancer.

An immuno-oncology agent can be, for example, a small molecule drug, an antibody, or a biologic or small molecule. Examples of biologic immuno-oncology agents include, but are not limited to, cancer vaccines, antibodies, and cytokines. In some embodiments, an antibody is a monoclonal antibody. In some embodiments, a monoclonal antibody is humanized or human.

In some embodiments, an immuno-oncology agent is (i) an agonist of a stimulatory (including a co-stimulatory) receptor or (ii) an antagonist of an inhibitory (including a co-inhibitory) signal on T cells, both of which result in amplifying antigen-specific T cell responses.

Certain of the stimulatory and inhibitory molecules are members of the immunoglobulin super family (IgSF). One

important family of membrane-bound ligands that bind to co-stimulatory or co-inhibitory receptors is the B7 family, which includes B7-1, B7-2, B7-H1 (PD-L1), B7-DC (PD-L2), B7-H2 (ICOS-L), B7-H3, B7-H4, B7-H5 (VISTA), and B7-H6. Another family of membrane bound ligands that bind to co-stimulatory or co-inhibitory receptors is the TNF family of molecules that bind to cognate TNF receptor family members, which includes CD40 and CD40L, OX-40, OX-40L, CD70, CD27L, CD30, CD30L, 4-1BBL, CD137 (4-1BB), TRAIL/Apo2-L, TRAILR1/DR4, TRAILR2/DR5, TRAILR3, TRAILR4, OPG, RANK, RANKL, TWEAKR/Fn14, TWEAK, BAFFR, EDAR, XEDAR, TACI, APRIL, BCMA, LTβR, LIGHT, DcR3, HVEM, VEGI/TL1A, TRAMP/DR3, EDAR, EDA1, XEDAR, EDA2, TNFR1, Lymphotoxin α/TNFβ, TNFR2, TNFα, LTβR, Lymphotoxin α1β2, FAS, FASL, RELT, DR6, TROY, NGFR.

In some embodiments, an immuno-oncology agent is a cytokine that inhibits T cell activation (*e.g.*, IL-6, IL-10, TGF-β, VEGF, and other immunosuppressive cytokines) or a cytokine that stimulates T cell activation, for stimulating an immune response.

In some embodiments, a combination of a bicyclic peptide complex toxin conjugate or heterotandem bicyclic peptide complex specific for Nectin-4, or a pharmaceutically acceptable salt thereof, or a pharmaceutical composition thereof and an immuno-oncology agent can stimulate T cell responses. In some embodiments, an immuno-oncology agent is: (i) an antagonist of a protein that inhibits T cell activation (*e.g.*, immune checkpoint inhibitors) such as CTLA-4, PD-1, PD-L1, PD-L2, LAG-3, TIM-3, Galectin 9, CEACAM-1, BTLA, CD69, Galectin-1, TIGIT, CD113, GPR56, VISTA, 2B4, CD48, GARP, PD1H, LAIR1, TIM-1, and TIM-4; or (ii) an agonist of a protein that stimulates T cell activation such as B7-1, B7-2, CD28, 4-1BB (CD137), 4-1BBL, ICOS, ICOS-L, OX40, OX40L, GITR, GITRL, CD70, CD27, CD40, DR3 and CD28H.

In some embodiments, an immuno-oncology agent is an antagonist of inhibitory receptors on NK cells or an agonists of activating receptors on NK cells. In some embodiments, an immuno-oncology agent is an antagonist of KIR, such as lirilumab.

In some embodiments, an immuno-oncology agent is an agent that inhibits or depletes macrophages or monocytes, including but not limited to CSF-1R antagonists such as CSF-1R antagonist antibodies including RG7155 (WO11/70024, WO11/107553, WO11/131407, WO13/87699, WO13/119716, WO13/132044) or FPA-008 (WO11/140249; WO13169264; WO14/036357).

In some embodiments, an immuno-oncology agent is selected from agonistic agents that ligate positive costimulatory receptors, blocking agents that attenuate signaling through inhibitory receptors, antagonists, and one or more agents that increase systemically the frequency of anti-tumor T cells, agents that overcome distinct immune suppressive pathways within the tumor microenvironment (*e.g.*, block inhibitory receptor engagement (*e.g.*, PD-L1/PD-1 interactions), deplete or inhibit Tregs (*e.g.*, using an anti-CD25 monoclonal antibody (*e.g.*, daclizumab) or by *ex vivo* anti-CD25 bead depletion), inhibit metabolic enzymes such as IDO, or reverse/prevent T cell energy or exhaustion) and agents that trigger innate immune activation and/or inflammation at tumor sites.

In some embodiments, an immuno-oncology agent is a CTLA-4 antagonist. In some embodiments, a CTLA-4 antagonist is an antagonistic CTLA-4 antibody. In some embodiments, an antagonistic CTLA-4 antibody is YERVOY (ipilimumab) or tremelimumab.

In some embodiments, an immuno-oncology agent is a PD-1 antagonist. In some embodiments, a PD-1 antagonist is administered by infusion. In some embodiments, an immuno-oncology agent is an antibody or an antigen-binding portion thereof that binds specifically to a Programmed Death-1 (PD-1) receptor and inhibits PD-1 activity. In some embodiments, a PD-1 antagonist is an antagonistic PD-1 antibody. In some embodiments, an antagonistic PD-1 antibody is OPDIVO (nivolumab), KEYTRUDA (pembrolizumab), or MEDI-0680 (AMP-514; WO2012/145493). In some embodiments, an immuno-oncology agent may be pidilizumab (CT-011). In some embodiments, an immuno-oncology agent is a recombinant protein composed of the extracellular domain of PD-L2 (B7-DC) fused to the Fc portion of IgG1, called AMP-224.

In some embodiments, an immuno-oncology agent is a PD-L1 antagonist. In some embodiments, a PD-L1 antagonist is an antagonistic PD-L1 antibody. In some embodiments, a PD-L1 antibody is atezolizumab (MPDL3280A, RG7446; WO2010/077634), durvalumab (MEDI4736), BMS-936559 (WO2007/005874), avelumab (MSB0010718C, WO2013/79174), or cemiplimab (REGN2810).

In some embodiments, an immuno-oncology agent is a LAG-3 antagonist. In some embodiments, a LAG-3 antagonist is an antagonistic LAG-3 antibody. In some embodiments, a LAG3 antibody is BMS-986016 (WO10/19570, WO14/08218), or IMP-731 or IMP-321 (WO08/132601, WO009/44273).

In some embodiments, an immuno-oncology agent is a CD137 (4-1BB) agonist. In some embodiments, a CD137 (4-1BB) agonist is an agonistic CD137 antibody. In some embodiments, a CD137 antibody is urelumab or PF-05082566 (WO12/32433).

In some embodiments, an immuno-oncology agent is a GITR agonist. In some embodiments, a GITR agonist is an agonistic GITR antibody. In some embodiments, a GITR antibody is BMS-986153, BMS-986156, TRX-518 (WO006/105021, WO009/009116), or MK-4166 (WO11/028683).

In some embodiments, an immuno-oncology agent is an indoleamine (2,3)-dioxygenase (IDO) antagonist. In

some embodiments, an IDO antagonist is selected from epacadostat (INCB024360, Incyte); indoximod (NLG-8189, NewLink Genetics Corporation); capmanitib (INC280, Novartis); GDC-0919 (Genentech/Roche); PF-06840003 (Pfizer); BMS:F001287 (Bristol-Myers Squibb); Phy906/KD108 (Phytoceutica); an enzyme that breaks down kynurenine (Kynase, Ikena Oncology, formerly known as Kyn Therapeutics); and NLG-919 (WO09/73620, WO009/1156652, WO11/56652, WO12/142237).

In some embodiments, an immuno-oncology agent is an OX40 agonist. In some embodiments, an OX40 agonist is an agonistic OX40 antibody. In some embodiments, an OX40 antibody is MEDI-6383 or MEDI-6469.

In some embodiments, an immuno-oncology agent is an OX40L antagonist. In some embodiments, an OX40L antagonist is an antagonistic OX40 antibody. In some embodiments, an OX40L antagonist is RG-7888 (WO06/029879).

In some embodiments, an immuno-oncology agent is a CD40 agonist. In some embodiments, a CD40 agonist is an agonistic CD40 antibody. In some embodiments, an immuno-oncology agent is a CD40 antagonist. In some embodiments, a CD40 antagonist is an antagonistic CD40 antibody. In some embodiments, a CD40 antibody is lucatumumab or dacetuzumab.

In some embodiments, an immuno-oncology agent is a CD27 agonist. In some embodiments, a CD27 agonist is an agonistic CD27 antibody. In some embodiments, a CD27 antibody is varlilumab.

In some embodiments, an immuno-oncology agent is MGA271 (to B7H3) (WO11/109400).

In some embodiments, an immuno-oncology agent is abagovomab, adecatumumab, afutuzumab, alemtuzumab, anatumomab mafenatox, apolizumab, atezolimab, avelumab, blinatumomab, BMS-936559, catumaxomab, durvalumab, epacadostat, epratuzumab, indoximod, inotuzumab ozogamicin, intelumumab, ipilimumab, isatuximab, lambrolizumab, MED14736, MPDL3280A, nivolumab, obinutuzumab, ocaratuzumab, ofatumumab, olatatumab, pembrolizumab, pidilizumab, rituximab, ticilimumab, samalizumab, or tremelimumab.

In some embodiments, an immuno-oncology agent is an immunostimulatory agent. For example, antibodies blocking the PD-1 and PD-L1 inhibitory axis can unleash activated tumor-reactive T cells and have been shown in clinical trials to induce durable anti-tumor responses in increasing numbers of tumor histologies, including some tumor types that conventionally have not been considered immunotherapy sensitive. See, *e.g.,* Okazaki, T. et al. (2013) Nat. Immunol. 14, 1212-1218; Zou et al. (2016) Sci. Transl. Med. 8. The anti-PD-1 antibody nivolumab (OPDIVO®, Bristol-Myers Squibb, also known as ONO-4538, MDX1106 and BMS-936558), has shown potential to improve the overall survival in patients with RCC who had experienced disease progression during or after prior anti-angiogenic therapy.

In some embodiments, the immunomodulatory therapeutic specifically induces apoptosis of tumor cells. Approved immunomodulatory therapeutics include pomalidomide (POMALYST®, Celgene); lenalidomide (REVLIMID®, Celgene); ingenol mebutate (PICATO®, LEO Pharma).

In some embodiments, an immuno-oncology agent is a cancer vaccine. In some embodiments, the cancer vaccine is selected from sipuleucel-T (PROVENGE®, Dendreon/Valeant Pharmaceuticals), which has been approved for treatment of asymptomatic, or minimally symptomatic metastatic castrate-resistant (hormone-refractory) prostate cancer; and talimogene laherparepvec (IMLYGIC®, BioVex/Amgen, previously known as T-VEC), a genetically modified oncolytic viral therapy approved for treatment of unresectable cutaneous, subcutaneous and nodal lesions in melanoma. In some embodiments, an immuno-oncology agent is selected from an oncolytic viral therapy such as pexastimogene devacirepvec (PexaVec/JX-594, SillaJen/formerly Jennerex Biotherapeutics), a thymidine kinase- (TK-) deficient vaccinia virus engineered to express GM-CSF, for hepatocellular carcinoma (NCT02562755) and melanoma (NCT00429312); pelareorep (REOLYSIN®, Oncolytics Biotech), a variant of respiratory enteric orphan virus (reovirus) which does not replicate in cells that are not RAS-activated, in numerous cancers, including colorectal cancer (NCT01622543); prostate cancer (NCT01619813); head and neck squamous cell cancer (NCT01166542); pancreatic adenocarcinoma (NCT00998322); and non-small cell lung cancer (NSCLC) (NCT 00861627); enadenotucirev (NG-348, PsiOxus, formerly known as ColoAd1), an adenovirus engineered to express a full length CD80 and an antibody fragment specific for the T-cell receptor CD3 protein, in ovarian cancer (NCT02028117); metastatic or advanced epithelial tumors such as in colorectal cancer, bladder cancer, head and neck squamous cell carcinoma and salivary gland cancer (NCT02636036); ONCOS-102 (Targovax/formerly Oncos), an adenovirus engineered to express GM-CSF, in melanoma (NCT03003676); and peritoneal disease, colorectal cancer or ovarian cancer (NCT02963831); GL-ONC1 (GLV-1h68/GLV-1h153, Genelux GmbH), vaccinia viruses engineered to express beta-galactosidase (beta-gal)/beta-glucoronidase or beta-gal/human sodium iodide symporter (hNIS), respectively, were studied in peritoneal carcinomatosis (NCT01443260); fallopian tube cancer, ovarian cancer (NCT 02759588); or CG0070 (Cold Genesys), an adenovirus engineered to express GM-CSF, in bladder cancer (NCT02365818).

In some embodiments, an immuno-oncology agent is selected from JX-929 (SillaJen/formerly Jennerex Biotherapeutics), a TK- and vaccinia growth factor-deficient vaccinia virus engineered to express cytosine deaminase, which is able to convert the prodrug 5-fluorocytosine to the cytotoxic drug 5-fluorouracil; TG01 and TG02 (Targovax/-formerly Oncos), peptide-based immunotherapy agents targeted for difficult-to-treat RAS mutations; and TILT-123 (TILT Biotherapeutics), an engineered adenovirus designated: Ad5/3-E2F-delta24-hTNF$\alpha$-IRES-hIL20; and VSV-GP (ViraTherapeutics) a vesicular stomatitis virus (VSV) engineered to express the glycoprotein (GP) of lymphocytic choriomeningitis virus (LCMV), which can be further engineered to express antigens designed to raise an antigen-specific $CD8^+$ T cell response.

In some embodiments, an immuno-oncology agent is a T-cell engineered to express a chimeric antigen receptor, or CAR. The T-cells engineered to express such chimeric antigen receptor are referred to as a CAR-T cells.

CARs have been constructed that consist of binding domains, which may be derived from natural ligands, single chain variable fragments (scFv) derived from monoclonal antibodies specific for cell-surface antigens, fused to endodomains that are the functional end of the T-cell receptor (TCR), such as the CD3-zeta signaling domain from TCRs, which is capable of generating an activation signal in T lymphocytes. Upon antigen binding, such CARs link to endogenous signaling pathways in the effector cell and generate activating signals similar to those initiated by the TCR complex.

For example, in some embodiments the CAR-T cell is one of those described in U.S. Patent 8,906,682 (June et al.), which discloses CAR-T cells engineered to comprise an extracellular domain having an antigen binding domain (such as a domain that binds to CD19), fused to an intracellular signaling domain of the T cell antigen receptor complex zeta chain (such as CD3 zeta). When expressed in the T cell, the CAR is able to redirect antigen recognition based on the antigen binding specificity. In the case of CD19, the antigen is expressed on malignant B cells. Over 200 clinical trials are currently in progress employing CAR-T in a wide range of indications. [https://clinicaltrials.gov/ct2/results?term=chimeric+antigen+receptors&pg=1].

In some embodiments, an immunostimulatory agent is an activator of retinoic acid receptor-related orphan receptor y (RORyt). RORyt is a transcription factor with key roles in the differentiation and maintenance of Type 17 effector subsets of CD4+ (Th17) and CD8+ (Tc17) T cells, as well as the differentiation of IL-17 expressing innate immune cell subpopulations such as NK cells. In some embodiments, an activator of RORyt is LYC-55716 (Lycera), which is currently being evaluated in clinical trials for the treatment of solid tumors (NCT02929862).

In some embodiments, an immunostimulatory agent is an agonist or activator of a toll-like receptor (TLR). Suitable activators of TLRs include an agonist or activator of TLR9 such as SD-101 (Dynavax). SD-101 is an immunostimulatory CpG which is being studied for B-cell, follicular and other lymphomas (NCT02254772). Agonists or activators of TLR8 which may be used include motolimod (VTX-2337, VentiRx Pharmaceuticals) which is being studied for squamous cell cancer of the head and neck (NCT02124850) and ovarian cancer (NCT02431559).

Other immuno-oncology agents that can be used include urelumab (BMS-663513, Bristol-Myers Squibb), an anti-CD137 monoclonal antibody; varlilumab (CDX-1127, Celldex Therapeutics), an anti-CD27 monoclonal antibody; BMS-986178 (Bristol-Myers Squibb), an anti-OX40 monoclonal antibody; lirilumab (IPH2102/BMS-986015, Innate Pharma, Bristol-Myers Squibb), an anti-KIR monoclonal antibody; monalizumab (IPH2201, Innate Pharma, AstraZeneca) an anti-NKG2A monoclonal antibody; andecaliximab (GS-5745, Gilead Sciences), an anti-MMP9 antibody; MK-4166 (Merck & Co.), an anti-GITR monoclonal antibody.

In some embodiments, an immunostimulatory agent is selected from elotuzumab, mifamurtide, an agonist or activator of a toll-like receptor, and an activator of RORyt.

In some embodiments, an immunostimulatory therapeutic is recombinant human interleukin 15 (rhIL-15). rhIL-15 has been tested in the clinic as a therapy for melanoma and renal cell carcinoma (NCT01021059 and NCT01369888) and leukemias (NCT02689453). In some embodiments, an immunostimulatory agent is recombinant human interleukin 12 (rhIL-12). In some embodiments, an IL-15 based immunotherapeutic is heterodimeric IL-15 (hetIL-15, Novartis/Admune), a fusion complex composed of a synthetic form of endogenous IL-15 complexed to the soluble IL-15 binding protein IL-15 receptor alpha chain (IL15:sIL-15RA), which has been tested in Phase 1 clinical trials for melanoma, renal cell carcinoma, non-small cell lung cancer and head and neck squamous cell carcinoma (NCT02452268). In some embodiments, a recombinant human interleukin 12 (rhIL-12) is NM-IL-12 (Neumedicines, Inc.), NCT02544724, or NCT02542124.

In some embodiments, an immuno-oncology agent is selected from those descripted in Jerry L. Adams et al., "Big opportunities for small molecules in immuno-oncology," Cancer Therapy 2015, Vol. 14, pages 603-622. In some embodiments, an immuno-oncology agent is selected from the examples described in Table 1 of Jerry L. Adams *et al.* In some embodiments, an immuno-oncology agent is a small molecule targeting an immuno-oncology target selected from those listed in Table 2 of Jerry L. Adams *et al.* In some embodiments, an immuno-oncology agent is a small molecule agent selected from those listed in Table 2 of Jerry L. Adams *et al.*

In some embodiments, an immuno-oncology agent is selected from the small molecule immuno-oncology agents described in Peter L. Toogood, "Small molecule immuno-oncology therapeutic agents," Bioorganic & Medicinal Chemistry Letters 2018, Vol. 28, pages 319-329. In some embodiments, an immuno-oncology agent is an agent targeting the pathways as described in Peter L. Toogood.

In some embodiments, an immuno-oncology agent is selected from those described in Sandra L. Ross et al., "Bispecific T cell engager (BITE® ) antibody constructs can mediate bystander tumor cell killing", PLoS ONE 12(8): e0183390. In some embodiments, an immuno-oncology agent is a bispecific T cell engager (BITE®) antibody construct. In some embodimens, a bispecific T cell engager (BITE®) antibody construct is a CD19/CD3 bispecific antibody construct. In some embodimens, a bispecific T cell engager (BITE®) antibody construct is an EGFR/CD3 bispecific antibody construct. In some embodimens, a bispecific T cell engager (BITE®) antibody construct activates T cells. In some embodimens, a

bispecific T cell engager (BITE®) antibody construct activates T cells, which release cytokines inducing upregulation of intercellular adhesion molecule 1 (ICAM-1) and FAS on bystander cells. In some embodimens, a bispecific T cell engager (BITE®) antibody construct activates T cells which result in induced bystander cell lysis. In some embodiments, the bystander cells are in solid tumors. In some embodiments, the bystander cells being lysed are in proximity to the BITE®-acticvated T cells. In some embodiment, the bystander cells comprises tumor-associated antigen (TAA) negatgive cancer cells. In some embodiment, the bystander cells comprise EGFR-negative cancer cells. In some embodiments, an immuno-oncology agent is an antibody which blocks the PD-L1/PD1 axis and/or CTLA4. In some embodiments, an immuno-oncology agent is an *ex vivo* expanded tumor-infiltrating T cell. In some embodiments, an immuno-oncology agent is a bispecific antibody construct or chimeric antigen receptors (CARs) that directly connect T cells with tumor-associated surface antigens (TAAs).

Exemplary Immune Checkpoint Inhibitors

In some embodiments, an immuno-oncology agent is an immune checkpoint inhibitor as described herein.

The term "checkpoint inhibitor" as used herein relates to agents useful in preventing cancer cells from avoiding the immune system of the patient. One of the major mechanisms of anti-tumor immunity subversion is known as "T-cell exhaustion," which results from chronic exposure to antigens that has led to up-regulation of inhibitory receptors. These inhibitory receptors serve as immune checkpoints in order to prevent uncontrolled immune reactions.

PD-1 and co-inhibitory receptors such as cytotoxic T-lymphocyte antigen 4 (CTLA-4, B and T Lymphocyte Attenuator (BTLA; CD272), T cell Immunoglobulin and Mucin domain-3 (Tim-3), Lymphocyte Activation Gene-3 (Lag-3; CD223), and others are often referred to as a checkpoint regulators. They act as molecular "gatekeepers" that allow extracellular information to dictate whether cell cycle progression and other intracellular signaling processes should proceed.

In some embodiments, an immune checkpoint inhibitor is an antibody to PD-1. PD-1 binds to the programmed cell death 1 receptor (PD-1) to prevent the receptor from binding to the inhibitory ligand PDL-1, thus overriding the ability of tumors to suppress the host anti-tumor immune response.

In some embodiments, the checkpoint inhibitor is a biologic therapeutic or a small molecule. In some embodiments, the checkpoint inhibitor is a monoclonal antibody, a humanized antibody, a fully human antibody, a fusion protein or a combination thereof. In some embodiments, the checkpoint inhibitor inhibits a checkpoint protein selected from CTLA-4, PDLl, PDL2, PDl, B7-H3, B7-H4, BTLA, HVEM, TIM3, GAL9, LAG3, VISTA, KIR, 2B4, CD160, CGEN-15049, CHK 1, CHK2, A2aR, B-7 family ligands or a combination thereof. In some embodiments, the checkpoint inhibitor interacts with a ligand of a checkpoint protein selected from CTLA-4, PDLl, PDL2, PDl, B7-H3, B7-H4, BTLA, HVEM, TIM3, GAL9, LAG3, VISTA, KIR, 2B4, CD160, CGEN-15049, CHK 1, CHK2, A2aR, B-7 family ligands or a combination thereof. In some embodiments, the checkpoint inhibitor is an immunostimulatory agent, a T cell growth factor, an interleukin, an antibody, a vaccine or a combination thereof. In some embodiments, the interleukin is IL-7 or IL-15. In some embodiments, the interleukin is glycosylated IL-7. In an additional aspect, the vaccine is a dendritic cell (DC) vaccine.

Checkpoint inhibitors include any agent that blocks or inhibits in a statistically significant manner, the inhibitory pathways of the immune system. Such inhibitors can include small molecule inhibitors or can include antibodies, or antigen binding fragments thereof, that bind to and block or inhibit immune checkpoint receptors or antibodies that bind to and block or inhibit immune checkpoint receptor ligands. Illustrative checkpoint molecules that can be targeted for blocking or inhibition include, but are not limited to, CTLA-4, PDL1, PDL2, PD1, B7-H3, B7-H4, BTLA, HVEM, GAL9, LAG3, TIM3, VISTA, KIR, 2B4 (belongs to the CD2 family of molecules and is expressed on all NK, $\gamma\delta$, and memory $CD8^+$ ($\alpha\beta$) T cells), CD160 (also referred to as BY55), CGEN-15049, CHK 1 and CHK2 kinases, A2aR, and various B-7 family ligands. B7 family ligands include, but are not limited to, B7- 1, B7-2, B7-DC, B7-H1, B7-H2, B7-H3, B7-H4, B7-H5, B7-H6 and B7-H7. Checkpoint inhibitors include antibodies, or antigen binding fragments thereof, other binding proteins, biologic therapeutics, or small molecules, that bind to and block or inhibit the activity of one or more of CTLA-4, PDL1, PDL2, PD1, BTLA, HVEM, TIM3, GAL9, LAG3, VISTA, KIR, 2B4, CD 160 and CGEN-15049. Illustrative immune checkpoint inhibitors include, but are not limited to, Tremelimumab (CTLA-4 blocking antibody), anti-OX40, PD-Ll monoclonal Antibody (Anti-B7-Hl; MEDI4736), MK-3475 (PD-1 blocker), Nivolumab (anti-PDl antibody), CT-011 (anti-PDl antibody), BY55 monoclonal antibody, AMP224 (anti-PDLl antibody), BMS- 936559 (anti-PDLl antibody), MPLDL3280A (anti-PDLl antibody), MSB0010718C (anti-PDLl antibody), and ipilimumab (anti-CTLA-4 checkpoint inhibitor). Checkpoint protein ligands include, but are not limited to PD-Ll, PD-L2, B7-H3, B7-H4, CD28, CD86 and TIM-3.

In certain embodiments, the immune checkpoint inhibitor is selected from a PD-1 antagonist, a PD-L1 antagonist, and a CTLA-4 antagonist. In some embodiments, the checkpoint inhibitor is selected from the group consisting of nivolumab (OPDIVO®), ipilimumab (YERVOY®), and pembrolizumab (KEYTRUDA®). In some embodiments, the checkpoint inhibitor is selected from nivolumab (anti-PD-1 antibody, OPDIVO®, Bristol-Myers Squibb); pembrolizumab (anti-PD-1 antibody, KEYTRUDA®, Merck); ipilimumab (anti-CTLA-4 antibody, YERVOY®, Bristol-Myers Squibb); durvalumab (anti-PD-L1 antibody, IMFINZI®, AstraZeneca); and atezolizumab (anti-PD-L1 antibody, TECENTRIQ®, Genentech).

In some embodiments, the checkpoint inhibitor is selected from the group consisting of lambrolizumab (MK-3475), nivolumab (BMS-936558), pidilizumab (CT-011), AMP-224, MDX-1105, MEDI4736, MPDL3280A, BMS-936559, ipilimumab, lirlumab, IPH2101, pembrolizumab (KEYTRUDA®), and tremelimumab.

In some embodiments, an immune checkpoint inhibitor is REGN2810 (Regeneron), an anti-PD-1 antibody tested in patients with basal cell carcinoma (NCT03132636); NSCLC (NCT03088540); cutaneous squamous cell carcinoma (NCT02760498); lymphoma (NCT02651662); and melanoma (NCT03002376); pidilizumab (CureTech), also known as CT-011, an antibody that binds to PD-1, in clinical trials for diffuse large B-cell lymphoma and multiple myeloma; avelumab (BAVENCIO®, Pfizer/Merck KGaA), also known as MSB0010718C), a fully human IgG1 anti-PD-L1 antibody, in clinical trials for non-small cell lung cancer, Merkel cell carcinoma, mesothelioma, solid tumors, renal cancer, ovarian cancer, bladder cancer, head and neck cancer, and gastric cancer; or PDR001 (Novartis), an inhibitory antibody that binds to PD-1, in clinical trials for non-small cell lung cancer, melanoma, triple negative breast cancer and advanced or metastatic solid tumors. Tremelimumab (CP-675,206; Astrazeneca) is a fully human monoclonal antibody against CTLA-4 that has been in studied in clinical trials for a number of indications, including: mesothelioma, colorectal cancer, kidney cancer, breast cancer, lung cancer and non-small cell lung cancer, pancreatic ductal adenocarcinoma, pancreatic cancer, germ cell cancer, squamous cell cancer of the head and neck, hepatocellular carcinoma, prostate cancer, endometrial cancer, metastatic cancer in the liver, liver cancer, large B-cell lymphoma, ovarian cancer, cervical cancer, metastatic anaplastic thyroid cancer, urothelial cancer, fallopian tube cancer, multiple myeloma, bladder cancer, soft tissue sarcoma, and melanoma. AGEN-1884 (Agenus) is an anti-CTLA4 antibody that is being studied in Phase 1 clinical trials for advanced solid tumors (NCT02694822).

In some embodiments, a checkpoint inhibitor is an inhibitor of T-cell immunoglobulin mucin containing protein-3 (TIM-3). TIM-3 inhibitors that may be used include TSR-022, LY3321367 and MBG453. TSR-022 (Tesaro) is an anti-TIM-3 antibody which is being studied in solid tumors (NCT02817633). LY3321367 (Eli Lilly) is an anti-TIM-3 antibody which is being studied in solid tumors (NCT03099109). MBG453 (Novartis) is an anti-TIM-3 antibody which is being studied in advanced malignancies (NCT02608268).

In some embodiments, a checkpoint inhibitor is an inhibitor of T cell immunoreceptor with Ig and ITIM domains, or TIGIT, an immune receptor on certain T cells and NK cells. TIGIT inhibitors that may be used include BMS-986207 (Bristol-Myers Squibb), an anti-TIGIT monoclonal antibody (NCT02913313); OMP-313M32 (Oncomed); and anti-TIGIT monoclonal antibody (NCT03119428).

In some embodiments, a checkpoint inhibitor is an inhibitor of Lymphocyte Activation Gene-3 (LAG-3). LAG-3 inhibitors that may be used include BMS-986016 and REGN3767 and IMP321. BMS-986016 (Bristol-Myers Squibb), an anti-LAG-3 antibody, is being studied in glioblastoma and gliosarcoma (NCT02658981). REGN3767 (Regeneron), is also an anti-LAG-3 antibody, and is being studied in malignancies (NCT03005782). IMP321 (Immutep S.A.) is an LAG-3-Ig fusion protein, being studied in melanoma (NCT02676869); adenocarcinoma (NCT02614833); and metastatic breast cancer (NCT00349934).

Checkpoint inhibitors that can be used include OX40 agonists. OX40 agonists that are being studied in clinical trials include PF-04518600/PF-8600 (Pfizer), an agonistic anti-OX40 antibody, in metastatic kidney cancer (NCT03092856) and advanced cancers and neoplasms (NCT02554812; NCT05082566); GSK3174998 (Merck), an agonistic anti-OX40 antibody, in Phase 1 cancer trials (NCT02528357); MEDI0562 (Medimmune/AstraZeneca), an agonistic anti-OX40 antibody, in advanced solid tumors (NCT02318394 and NCT02705482); MEDI6469, an agonistic anti-OX40 antibody (Medimmune/AstraZeneca), in patients with colorectal cancer (NCT02559024), breast cancer (NCT01862900), head and neck cancer (NCT02274155) and metastatic prostate cancer (NCT01303705); and BMS-986178 (Bristol-Myers Squibb) an agonistic anti-OX40 antibody, in advanced cancers (NCT02737475).

Checkpoint inhibitors that can be used include CD137 (also called 4-1BB) agonists. CD137 agonists that are being studied in clinical trials include utomilumab (PF-05082566, Pfizer) an agonistic anti-CD137 antibody, in diffuse large B-cell lymphoma (NCT02951156) and in advanced cancers and neoplasms (NCT02554812 and NCT05082566); urelumab (BMS-663513, Bristol-Myers Squibb), an agonistic anti-CD137 antibody, in melanoma and skin cancer (NCT02652455) and glioblastoma and gliosarcoma (NCT02658981); and CTX-471 (Compass Therapeutics), an agonistic anti-CD137 antibody in metastatic or locally advanced malignancies (NCT03881488).

Checkpoint inhibitors that can be used include CD27 agonists. CD27 agonists that are being studied in clinical trials include varlilumab (CDX-1127, Celldex Therapeutics) an agonistic anti-CD27 antibody, in squamous cell head and neck cancer, ovarian carcinoma, colorectal cancer, renal cell cancer, and glioblastoma (NCT02335918); lymphomas (NCT01460134); and glioma and astrocytoma (NCT02924038).

Checkpoint inhibitors that can be used include glucocorticoid-induced tumor necrosis factor receptor (GITR) agonists. GITR agonists that are being studied in clinical trials include TRX518 (Leap Therapeutics), an agonistic anti-GITR antibody, in malignant melanoma and other malignant solid tumors (NCT01239134 and NCT02628574); GWN323 (Novartis), an agonistic anti-GITR antibody, in solid tumors and lymphoma (NCT 02740270); INCAGN01876 (Incyte/Agenus), an agonistic anti-GITR antibody, in advanced cancers (NCT02697591 and NCT03126110); MK-4166 (Merck), an

agonistic anti-GITR antibody, in solid tumors (NCT02132754) and MEDI1873 (Medimmune/AstraZeneca), an agonistic hexameric GITR-ligand molecule with a human IgG1 Fc domain, in advanced solid tumors (NCT02583165).

Checkpoint inhibitors that can be used include inducible T-cell co-stimulator (ICOS, also known as CD278) agonists. ICOS agonists that are being studied in clinical trials include MEDI-570 (Medimmune), an agonistic anti-ICOS antibody, in lymphomas (NCT02520791); GSK3359609 (Merck), an agonistic anti-ICOS antibody, in Phase 1 (NCT02723955); JTX-2011 (Jounce Therapeutics), an agonistic anti-ICOS antibody, in Phase 1 (NCT02904226).

Checkpoint inhibitors that can be used include killer IgG-like receptor (KIR) inhibitors. KIR inhibitors that are being studied in clinical trials include lirilumab (IPH2102/BMS-986015, Innate Pharma/Bristol-Myers Squibb), an anti-KIR antibody, in leukemias (NCT01687387, NCT02399917, NCT02481297, NCT02599649), multiple myeloma (NCT02252263), and lymphoma (NCT01592370); IPH2101 (1-7F9, Innate Pharma) in myeloma (NCT01222286 and NCT01217203); and IPH4102 (Innate Pharma), an anti-KIR antibody that binds to three domains of the long cytoplasmic tail (KIR3DL2), in lymphoma (NCT02593045).

Checkpoint inhibitors that can be used include CD47 inhibitors of interaction between CD47 and signal regulatory protein alpha (SIRPa). CD47/SIRPa inhibitors that are being studied in clinical trials include ALX-148 (Alexo Therapeutics), an antagonistic variant of (SIRPa) that binds to CD47 and prevents CD47/SIRPa-mediated signaling, in phase 1 (NCT03013218); TTI-621 (SIRPa-Fc, Trillium Therapeutics), a soluble recombinant fusion protein created by linking the N-terminal CD47-binding domain of SIRPa with the Fc domain of human IgG1, acts by binding human CD47, and preventing it from delivering its "do not eat" signal to macrophages, is in clinical trials in Phase 1 (NCT02890368 and NCT02663518); CC-90002 (Celgene), an anti-CD47 antibody, in leukemias (NCT02641002); and Hu5F9-G4 (Forty Seven, Inc.), in colorectal neoplasms and solid tumors (NCT02953782), acute myeloid leukemia (NCT02678338) and lymphoma (NCT02953509).

Checkpoint inhibitors that can be used include CD73 inhibitors. CD73 inhibitors that are being studied in clinical trials include MEDI9447 (Medimmune), an anti-CD73 antibody, in solid tumors (NCT02503774); and BMS-986179 (Bristol-Myers Squibb), an anti-CD73 antibody, in solid tumors (NCT02754141).

Checkpoint inhibitors that can be used include agonists of stimulator of interferon genes protein (STING, also known as transmembrane protein 173, or TMEM173). Agonists of STING that are being studied in clinical trials include MK-1454 (Merck), an agonistic synthetic cyclic dinucleotide, in lymphoma (NCT03010176); and ADU-S100 (MIW815, Aduro Biotech/Novartis), an agonistic synthetic cyclic dinucleotide, in Phase 1 (NCT02675439 and NCT03172936).

Checkpoint inhibitors that can be used include CSF1R inhibitors. CSF1R inhibitors that are being studied in clinical trials include pexidartinib (PLX3397, Plexxikon), a CSF1R small molecule inhibitor, in colorectal cancer, pancreatic cancer, metastatic and advanced cancers (NCT02777710) and melanoma, non-small cell lung cancer, squamous cell head and neck cancer, gastrointestinal stromal tumor (GIST) and ovarian cancer (NCT02452424); and IMC-CS4 (LY3022855, Lilly), an anti-CSF-1R antibody, in pancreatic cancer (NCT03153410), melanoma (NCT03101254), and solid tumors (NCT02718911); and BLZ945 (4-[2((1R,2R)-2-hydroxycyclohexylamino)-benzothiazol-6-yloxyl]-pyridine-2-carboxylic acid methylamide, Novartis), an orally available inhibitor of CSF1R, in advanced solid tumors (NCT02829723).

Checkpoint inhibitors that can be used include NKG2A receptor inhibitors. NKG2A receptor inhibitors that are being studied in clinical trials include monalizumab (IPH2201, Innate Pharma), an anti-NKG2A antibody, in head and neck neoplasms (NCT02643550) and chronic lymphocytic leukemia (NCT02557516).

In some embodiments, the immune checkpoint inhibitor is selected from nivolumab, pembrolizumab, ipilimumab, avelumab, durvalumab, atezolizumab, or pidilizumab.

## EXEMPLIFICATION

The following examples are intended to illustrate the invention and are not to be construed as being limitations thereon. All amino acids, unless noted otherwise, were used in the L- configurations.

| Abbreviations | Name |
|---|---|
| Ac | Acetyl |
| β-Ala | β-Alanine |
| D-Asp | D-Aspartic acid |
| HArg | HomoArginine |
| HyP | Hydroxyproline |
| 1Nal | 1-naphthylalanine |
| Sar | Sarcosine, such that $Sar_x$ represents x Sar residues |

**Example 1. A multi tumor survey of Nectin-4 expression to guide BT8009 indication selection 1.1 Nectin-4 IHC Protocol**

***Reagents/Probes/Antibodies***

| Name | Storage Temperature |
|---|---|
| Nectin-4 Rabbit Monoclonal Antibody (Abcam, Project #YMW-1-58) | -20°C |
| BOND Primary Antibody Diluent (Leica, cat# AR9352) | 2-8°C |
| BOND Dewax solution (Leica, cat# AR9222) | 2-8°C |
| BOND Wash Solution 10x (Leica, cat# AR9590) | 2-8°C |
| BOND Epitope Retrieval Solution 1 (Leica, cat# AR9961) | 2-8°C |
| Protein Block (Dako, cat# X090930-2) | 2-8°C |
| BOND Polymer Refine Detection Kit: Peroxide block, post primary reagent, polymer HRP reagent, DAB chromogen, and hematoxylin counterstain (Leica, cat# DS9800) | 2-8°C |
| BOND RTU Neg (Rabbit) Isotype Control (Leica, cat# PA0777) | 2-8°C |
| Deionized Water | Ambient |
| 100% Alcohol | Ambient |
| 95% Alcohol | Ambient |
| Xylene | Ambient |

***Equipment***

| Name | Model |
|---|---|
| Refrigerator | Thermo Scientific REL3004A22 or equivalent |
| Microtome | Leica RM2235 or equivalent |
| Drying Oven | Biocare 10-180 Aer Desert Chamber or equivalent |
| Automated IHC Stainer | Leica BOND III |
| Linear Stainer | Leica Autostainer XL |
| Coverslipper | Sakura Tissue-Tek Film 4740 or equivalent |
| Flotation Bath | Boekel or equivalent |

***Procedures***

1. Fix and embed the tissue, cut and mount the sections to positively charged slides, deparaffinize and rehydrate the section per standard practice
2. Load the samples into the Leica Bond III
3. Incubate with Bond Dewax solution for 2 minutes at room temperature
4. Incubate with alcohol for 2 minutes at room temperature
5. Wash with Bond Wash Solution
6. Incubate with Bond Epitope Retrieval Solution 1 for 20 minutes at 100°C
7. Rinse with Bond wash solution
8. Block with Bond Peroxide Block for 5 minutes at ambient

9. Rinse with Bond Wash Solution
10. Block with Dako Protein Block for 10 minutes at ambient
11. Rinse with Bond Wash Solution
12. Incubate with anti-Nectin-4 antibody (10 ug/ml) diluted in Dako Background Reducing Diluent for 30 min at ambient
13. Rinse with Bond wash solution
14. Incubate with Post Primary for 15 minutes at ambient
15. Rinse with Bond wash solution
16. Incubate with Bond Polymer for 15 minutes at ambient
17. Rinse with Bond wash solution
18. Incubate with Bond Mixed DAB Refine Reagent (Part 1) followed by Bond Mixed DAB Refine Reagent (Part 2) for 10 minutes at ambient
19. Rinse with DI water
20. Incubate with Bond Hematoxylin for 5 minutes at ambient
21. Rinse with DI water
22. Rinse with Bond wash solution
23. Rinse with DI water
24. Unload, dehydrate in graded alcohols and clear in xylene for 7 min at ambient
25. Coverslip

***Nectin-4 IHC Scoring***

Score the Nectin-4 staining results using the H-score method (defined as the sum of the products of the percent of cells x their staining intensity, on a scale of 0-3 where 0 is negative and 3 is strongly stained). Independent H-scores for the cell membrane and cytoplasm can be generated to differentiate between the two compartments.

**1.2 Results**

A clinical grade Nectin-4 IHC assay was developed on the Leica platform using a rabbit monoclonal α-Nectin-4 primary antibody (Abcam, Burlingame, CA) and the BOND Polymer Refine detection kit. TMAs of cancer types reported to have high Nectin-4 expression, including esophageal, pancreatic, bladder, head & neck, stomach, non-small cell lung, breast, and ovarian cancer, were stained and manually scored for Nectin-4 levels, with the tumor microenvironment evaluated in a subset of samples. Nectin-4 H-scores (stain intensity on a scale of 0-3 x percent positive tumor cells) were generated by a pathologist independently for tumor cell membrane and tumor cytoplasm. H-scores ≥ 100 in the tumor membrane or cytoplasm were considered positive.

| Indication | Total cores (N) | Percent Tumor Membrane Positive | Percent Tumor Cytoplasm Positive | Percent Tumor Membrane or Cytoplasm Positive |
|---|---|---|---|---|
| Breast | 225 | 16 | 49 | 57 |
| Bladder | 142 | 27 | 42 | 56 |
| Esophagus | 140 | 7 | 30 | 32 |
| Head & Neck | 69 | 10 | 25 | 32 |
| Lung | 157 | 3 | 17 | 17 |
| Ovarian | 89 | 2 | 13 | 15 |
| Pancreas | 96 | 1 | 2 | 3 |
| Stomach | 131 | 0 | 2 | 2 |

In all indications tested, a greater degree of Nectin-4 positivity was observed in the tumor cytoplasm as compared to tumor membrane. Breast and bladder cancer had the greatest frequency of Nectin-4 positivity. Subtype analysis in breast cancer identified enrichment of Nectin-4 expression in hormone receptor negative and human epidermal growth factor receptor 2 positive tumors.

Conclusion: The frequency of Nectin-4 expression as measured by IHC across multiple tumor types may guide clinical strategy for the BT8009 program.

**Example 2. Molecular-based enrichment strategy for Nectin-4 targeted Bicycle toxin conjugate BT8009**

**Material:** Tumor cores from US Biomax (Rockville, MD) were acquired and used both for TMA construction followed by IHC staining as well as DNA extraction and subsequent whole exome sequencing. The TMA (BR1301: https://www.biomax.us/tissue-arrays/Breast/BR1301) consists of 120 TNBC cases as well as controls (TNBC controls: 2 each of ER+, PR+, HER2+ & Nectin-4 protein expression controls: 2 spleen tissue and 2 skin tissue).

**Nectin-4 protein expression:** IHC was run using the Nectin-4 IHC assay. Tumor membrane and cytoplasmic H-scores were determined by a pathologist.

**Whole exome sequencing:** Whole exome sequencing was run on ~110 TNBC samples. DNA and RNA were co-extracted from FFPE tissue using Qiagen's AllPrep DNA/RNA FFPE kit. Next samples were run through DNA QC, whole exome library construction (including library preparation and hybrid capture), and exome sequence generation. All samples that passed DNA QC and sequence metric cutoffs were moved forward to DNA variant analysis.

**DNA variant analysis:** Tumor-only somatic analysis was run against a panel of normals using the GATK4 MuTect2 (SNV/Indels) and GATK4 CNV pipeline.

**Whole exome sequencing data preprocessing:** A total of 100 copy number segmentation files were in a pre-annotated format using Oncotator. Annotated copy number segmentation files specified genomic location, copy number calls (diploid, amplification or deletion), mean log2-transformed copy ratios, and genes located within the region of each continuous chromosome segment with consistent ploidy. These segment data were then expanded to the gene-level data in which genes were assigned the allelic frequencies of their parent chromosome segments. Genes with inconsistent ploidy, as indicated by their presence within multiple chromosome segments, were excluded from the analysis.

A goal of the experiment was to identify a routinely measured molecular surrogate of tumor Nectin-4 protein expression (i.e. somatic mutation or gene amplification) that could potentially increase patient screening frequency, yield, and likelihood of response to BT8009. Here it is demonstrated that Nectin-4 copy number is associated with Nectin-4 mRNA expression in 9 cancer indications in TCGA including breast, bladder, and lung cancer. In addition, across >30 cancer indications in TCGA, copy number of Nectin-4 is highly correlated with SDHC copy number (these two genes are ~225 kb apart on 1q23), which is included on multiple commercially available NGS panels. Taken together, these results suggest that the existence of an SDHC amplification could be used as an enrichment tool to identify patients with Nectin-4 positive tumours. 100 TNBC human tumor samples have been tested to determine Nectin-4 and SDHC copy number as well as Nectin-4 protein expression status by IHC.

Results: SDHC and Nectin-4 copy number were observed to be highly positively correlated ($R^2$=0.93). Furthermore, all (N = 22) TNBC tumors with a Nectin-4 copy number $\geq$ 3 were identified as positive for Nectin-4 protein expression and were at or above the H-score threshold required for enrolment to BT8009-100. In contrast, within the subset of tumors that were diploid for Nectin-4/SDHC (n=30), there were both Nectin-4 positive and negative tumors. This suggests that the presence of SDHC/Nectin-4 amplification can be used to increase the probability of identifying Nectin-4 expressing tumors.

Conclusions: it has been demonstrated that pre-existing patient NGS data with SDHC amplifications is useful for identifying patients with high Nectin-4 expressing tumors via IHC.

**Example 3. MultiOmyx™ hyperplexed immunofluorescence assay.**

MultiOmyx™ technology was utilized to evaluate the expression of a panel of 19-biomarkers including EphA2, Nectin-4, CD137, CD19, CD3, CD4, CD8, FOXP3, CD69, CD45RO, CD56, CD68, CD11b, GranzymeB, PD-1, PD-L1, HLA-ABC, Ki67 and tumor segmentation marker PanCK on head and neck squamous carcinoma (HNSCC), non-small cell lung cancer (NSCLC), breast and bladder cancer FFPE samples. Each FFPE slide was presented to NeoGenomics pathologist for tissue annotation and selection. The selected tumor relevant areas by the pathologist were used for staining and analysis. The staining was performed using a single 4uM FFPE slide. Within each staining round, two cyanine dye-labeled (Cy3, Cy5) antibodies were paired together and recognized up to two markers. The staining signal was then imaged and followed by novel dye inactivation, enabling repeated rounds of staining. Proprietary deep learning-based workflows were applied to identify individual cells and perform cell classification for all individual markers, as well as to identify tissue and tumor regions for analysis. Individual cell and region classification results were combined to generate co-expression summaries and compute spatial distribution statistics for phenotypes of interest.

A MultiOmyxTM hyperplexed immunofluorescence assay was developed to simultaneously quantify the presence of Nectin-4 and CD137-positive cells and the topography of these cell types in situ from 15 primary human tumor samples. (illustrated in **FIG. 10B**). The frequency of Nectin-4 and CD137- positive cells detected in each tumor sample demonstrated that a sizable CD137+ immune cell infiltrate was present within Nectin-4-positive NSCLC, HNSCC, and bladder cancer samples. Spatial profiling analysis further revealed that the CD137+ immune infiltrate was detected within the tumor stroma and a fraction of these were deeply penetrant within the tumor core and are in closest proximity to Nectin-4-positive tumor cells (**FIG. 10C**) Upon deeper profiling, CD137-expressing immune cells detected across all tumor

types analyzed were largely comprised of included CD4+ and CD8+ T cells, and CD68+ macrophages (**FIG. 10D**). While the action of a CD137 TICA might not be limited to T cells, these data indicate that at least T cells bearing CD137 are likely to contact tumor cells bearing Nectin-4. Thus, these observations support the development of a tumor-targeted CD137 agonist for the treatment of Nectin-4-positive human cancers.

The scope of this invention is to be defined by the claims rather than by the specific embodiments that have been represented by way of example.

**Claims**

1. A method of identifying or selecting a patient having an elevated Nectin-4 protein and/or RNA expression level in tumor tissue, comprising measuring the Succinate Dehydrogenase Complex Subunit C (SDHC) DNA copy number in tumor tissue or in circulating tumor DNA (ctDNA) of a patient, and selecting a patient having an elevated SDHC DNA copy number.

2. The method of claim 1, wherein the step of measuring the SDHC DNA copy number in tumor tissue or in circulating tumor DNA (ctDNA) of a patient comprises using Next-generation sequencing (NGS) technologies or an array-based sequence capture.

3. The method of claim 1 or 2, wherein the SDHC copy number is measured by sequencing the whole genome

4. The method of claim 1 or 2, wherein the patient has pancreatic cancer, stomach cancer, bladder cancer, head & neck cancer, non-small cell lung cancer (NSCLC), triple negative breast cancer (TNBC), or ovarian cancer.

5. The method of any one of claims 1-3, wherein the patient selected has a Log2 of the SDHC copy number ratio of 0.1 or more, 0.2 or more, 0.3 or more, 0.4 or more, 0.5 or more, 0.6 or more, 0.7 or more, 0.8 or more, 0.9 or more, or 1.0 or more.

6. The method of any one of claims 1-5, wherein the method further comprises measuring the Nectin-4 DNA copy number in tumor tissue or in ctDNA of the patient and selecting a patient having an elevated SDHC DNA copy number and an elevated Nectin-4 DNA copy number.

7. A bicyclic peptide complex toxin conjugate or heterotandem bicyclic peptide complex specific for Nectin-4, or a pharmaceutically acceptable salt thereof, or a pharmaceutical composition thereof for use in a method of treating a cancer in a patient having an elevated Nectin-4 protein and/or RNA expression level in tumor tissue, said method comprising selecting a patient having a Log2 of the SDHC copy number ratio of 0.1 or more, 0.2 or more, 0.3 or more, 0.4 or more, 0.5 or more, 0.6 or more, 0.7 or more, 0.8 or more, 0.9 or more, or 1.0 or more in tumor tissue or in circulating tumor DNA (ctDNA) as determined by Next-generation sequencing (NGS) technologies or an array-based sequence capture, and administering to the patient the bicyclic peptide complex toxin conjugate or heterotandem bicyclic peptide complex specific for Nectin-4, the pharmaceutically acceptable salt thereof, or the pharmaceutical composition thereof.

8. The bicyclic peptide complex toxin conjugate or heterotandem bicyclic peptide complex specific for Nectin-4, pharmaceutically acceptable salt thereof, or pharmaceutical composition thereof for use of claim 7, wherein the cancer is pancreatic cancer, stomach cancer, bladder cancer, head & neck cancer, non-small cell lung cancer (NSCLC), triple negative breast cancer (TNBC), or ovarian cancer.

9. The bicyclic peptide complex toxin conjugate or heterotandem bicyclic peptide complex specific for Nectin-4, or a pharmaceutically acceptable salt thereof, or a pharmaceutical composition thereof for use of claim 7 or 8, wherein the SDHC DNA copy number is measured by sequencing the whole exome.

10. The bicyclic peptide complex toxin conjugate or heterotandem bicyclic peptide complex specific for Nectin-4, pharmaceutically acceptable salt thereof, or pharmaceutical composition thereof for use of any one of claims 7 to 9, wherein the method further comprises administering an immuno-oncology agent.

11. The bicyclic peptide complex toxin conjugate or heterotandem bicyclic peptide complex specific for Nectin-4, pharmaceutically acceptable salt thereof, or pharmaceutical composition thereof for use of claim 10, wherein the immune-oncology agent is a PD-1 antagonist.

**12.** The bicyclic peptide complex toxin conjugate or heterotandem bicyclic peptide complex specific for Nectin-4, pharmaceutically acceptable salt thereof, or pharmaceutical composition thereof for use of claim 11, wherein the PD-1 antagonist is an antagonistic PD-1 antibody.

**13.** The bicyclic peptide complex toxin conjugate or heterotandem bicyclic peptide complex specific for Nectin-4, pharmaceutically acceptable salt thereof, or pharmaceutical composition thereof for use of claim 12, wherein the antagonistic PD-1 antibody is selected from nivolumab, pembrolizumab, MEDI-0680, pidilizumab, AMP-224, atezolizumab, durvalumab, BMS-936559, avelumab, or cemiplimab.

**14.** The bicyclic peptide complex toxin conjugate or heterotandem bicyclic peptide complex specific for Nectin-4, pharmaceutically acceptable salt thereof, or pharmaceutical composition thereof for use of any one of claims 7-13, wherein the bicyclic peptide complex toxin conjugate specific for Nectin-4 is BT8009 or the heterotandem bicyclic peptide complex specific for Nectin-4 is BT7480.

## Patentansprüche

**1.** Verfahren zur Identifizierung oder Auswahl eines Patienten mit einem erhöhten Nectin-4-Protein- und/oder -RNA-Expressionsniveau im Tumorgewebe, umfassend die Messung der DNA-Kopienzahl der Succinat-Dehydrogenase-Komplex-Untereinheit C (SDHC) im Tumorgewebe oder in der zirkulierenden Tumor-DNA (ctDNA) eines Patienten und die Auswahl eines Patienten mit einer erhöhten SDHC-DNA-Kopienzahl.

**2.** Verfahren nach Anspruch 1, wobei der Schritt des Messens der SDHC-DNA-Kopienzahl im Tumorgewebe oder in der zirkulierenden Tumor-DNA (ctDNA) eines Patienten die Verwendung von Next-Generation-Sequencing-Technologien (NGS-Technologien) oder einer Array-basierten Sequenzerfassung umfasst.

**3.** Verfahren nach Anspruch 1 oder 2, wobei die SDHC-Kopienzahl durch Sequenzierung des gesamten Genoms gemessen wird.

**4.** Verfahren nach Anspruch 1 oder 2, wobei der Patient an Bauchspeicheldrüsenkrebs, Magenkrebs, Blasenkrebs, Kopf-Hals-Krebs, nicht-kleinzelligem Lungenkrebs (NSCLC), dreifach negativem Brustkrebs (TNBC) oder Eierstockkrebs leidet.

**5.** Verfahren nach einem der Ansprüche 1 bis 3, wobei der ausgewählte Patient einen Log2-Wert des SDHC-Kopienzahlverhältnisses von 0,1 oder mehr, 0,2 oder mehr, 0,3 oder mehr, 0,4 oder mehr, 0,5 oder mehr, 0,6 oder mehr, 0,7 oder mehr, 0,8 oder mehr, 0,9 oder mehr oder 1,0 oder mehr aufweist.

**6.** Verfahren nach einem der Ansprüche 1 bis 5, wobei das Verfahren ferner das Messen der Nectin-4-DNA-Kopienzahl in Tumorgewebe oder in ctDNA des Patienten und das Auswählen eines Patienten mit einer erhöhten SDHC-DNA-Kopienzahl und einer erhöhten Nectin-4-DNA-Kopienzahl umfasst.

**7.** Bicyclisches Peptidkomplex-Toxin-Konjugat oder bicyclischer Heterotandem-Peptidkomplex, die für Nectin-4 spezifisch sind, oder pharmazeutisch verträgliches Salz davon, oder pharmazeutische Zusammensetzung davon zur Verwendung in einem Verfahren zur Behandlung einer Krebserkrankung bei einem Patienten mit einem erhöhten Nectin-4-Protein- und/oder -RNA-Expressionsniveau im Tumorgewebe, wobei das Verfahren Folgendes umfasst: die Auswahl eines Patienten mit einem Log2-Wert des SDHC-Kopienzahlverhältnisses von 0,1 oder mehr, 0,2 oder mehr, 0,3 oder mehr, 0,4 oder mehr, 0,5 oder mehr, 0,6 oder mehr, 0,7 oder mehr, 0,8 oder mehr, 0,9 oder mehr oder 1,0 oder mehr im Tumorgewebe oder in zirkulierender Tumor-DNA (ctDNA), bestimmt durch Next-Generation-Sequencing-Technologien (NGS-Technologien) oder eine Array-basierte Sequenzerfassung, und Verabreichung des für Nectin-4 spezifischen bicyclischen Peptidkomplex-Toxin-Konjugats oder bicyclischen Heterotandem-Peptidkomplexes, des pharmazeutisch verträglichen Salzes davon oder einer pharmazeutischen Zusammensetzung davon an den Patienten.

**8.** Bicyclisches Peptidkomplex-Toxin-Konjugat oder bicyclischer Heterotandem-Peptidkomplex, die für Nectin-4 spezifisch sind, pharmazeutisch verträgliches Salz davon oder pharmazeutische Zusammensetzung davon zur Verwendung nach Anspruch 7, wobei es sich bei dem Krebs um Bauchspeicheldrüsenkrebs, Magenkrebs, Blasenkrebs, Kopf-Hals-Krebs, nicht-kleinzelligen Lungenkrebs (NSCLC), dreifach negativen Brustkrebs (TNBC) oder Eierstockkrebs handelt.

9. Bicyclisches Peptidkomplex-Toxin-Konjugat oder bicyclischer Heterotandem-Peptidkomplex, die für Nectin-4 spezifisch sind, oder pharmazeutisch verträgliches Salz davon oder pharmazeutische Zusammensetzung davon zur Verwendung nach Anspruch 7 oder 8, wobei die SDHC-DNA-Kopienzahl durch Sequenzierung des gesamten Exoms gemessen wird.

10. Bicyclisches Peptidkomplex-Toxin-Konjugat oder bicyclischer Heterotandem-Peptidkomplex, die für Nectin-4 spezifisch sind, pharmazeutisch verträgliches Salz davon oder pharmazeutische Zusammensetzung davon zur Verwendung nach einem der Ansprüche 7 bis 9, wobei das Verfahren ferner die Verabreichung eines immunonkologischen Wirkstoffs umfasst.

11. Bicyclisches Peptidkomplex-Toxin-Konjugat oder bicyclischer Heterotandem-Peptidkomplex, die für Nectin-4 spezifisch sind, pharmazeutisch verträgliches Salz davon oder pharmazeutische Zusammensetzung davon zur Verwendung nach Anspruch 10, wobei der immunonkologische Wirkstoff ein PD-1-Antagonist ist.

12. Bicyclisches Peptidkomplex-Toxin-Konjugat oder bicyclischer Heterotandem-Peptidkomplex, die für Nectin-4 spezifisch sind, pharmazeutisch verträgliches Salz davon oder pharmazeutische Zusammensetzung davon zur Verwendung nach Anspruch 11, wobei der PD-1-Antagonist ein antagonistischer PD-1-Antikörper ist.

13. Bicyclisches Peptidkomplex-Toxin-Konjugat oder bicyclischer Heterotandem-Peptidkomplex, die für Nectin-4 spezifisch sind, pharmazeutisch verträgliches Salz davon oder pharmazeutische Zusammensetzung davon zur Verwendung nach Anspruch 12, wobei der antagonistische PD-1-Antikörper ausgewählt ist aus Nivolumab, Pembrolizumab, MEDI-0680, Pidilizumab, AMP-224, Atezolizumab, Durvalumab, BMS-936559, Avelumab oder Cemiplimab.

14. Bicyclisches Peptidkomplex-Toxin-Konjugat oder bicyclischer Heterotandem-Peptidkomplex, die für Nectin-4 spezifisch sind, pharmazeutisch verträgliches Salz davon oder pharmazeutische Zusammensetzung davon zur Verwendung nach einem der Ansprüche 7 bis 13, wobei das für Nectin-4 spezifische bicyclische Peptidkomplex-Toxin-Konjugat BT8009 ist oder der für Nectin-4 spezifische bicyclische Heterotandem-Peptidkomplex BT7480 ist.

## Revendications

1. Procédé d'identification ou de sélection d'un patient présentant un niveau élevé d'expression de protéine de la nectine 4 et/ou d'ARN dans un tissu tumoral, comprenant la mesure du nombre de copies d'ADN de sous-unité C du complexe de succinate déshydrogénase (SDHC) dans le tissu tumoral ou dans l'ADN tumoral circulant (ADNct) d'un patient, et la sélection d'un patient présentant un nombre de copies d'ADN de SDHC élevé.

2. Procédé selon la revendication 1, dans lequel l'étape consistant à mesurer le nombre de copies d'ADN de SDHC dans le tissu tumoral ou dans l'ADN tumoral circulant (ADNct) d'un patient comprend l'utilisation de technologies de séquençage de nouvelle génération (NGS) ou d'une capture de séquence basée sur une matrice.

3. Procédé selon la revendication 1 ou 2, dans lequel le nombre de copies de SDHC est mesuré par séquençage du génome entier.

4. Procédé selon la revendication 1 ou 2, dans lequel le patient est atteint d'un cancer du pancréas, d'un cancer de l'estomac, d'un cancer de la vessie, d'un cancer de la tête et du cou, d'un cancer du poumon non à petites cellules (CPNPC), d'un cancer du sein triple négatif (CSTN), ou d'un cancer de l'ovaire.

5. Procédé selon l'une quelconque des revendications 1 à 3, dans lequel le patient sélectionné présente un Log2 du rapport de nombre de copies de SDHC de 0,1 ou plus, 0,2 ou plus, 0,3 ou plus, 0,4 ou plus, 0,5 ou plus, 0,6 ou plus, 0,7 ou plus, 0,8 ou plus, 0,9 ou plus, ou 1,0 ou plus.

6. Procédé selon l'une quelconque des revendications 1 à 5, dans lequel le procédé comprend en outre la mesure du nombre de copies d'ADN de nectine 4 dans un tissu tumoral ou dans l'ADNct du patient et la sélection d'un patient présentant un nombre de copies d'ADN de SDHC élevé et un nombre de copies d'ADN de lanectine 4 élevé.

7. Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour

une utilisation dans un procédé de traitement d'un cancer chez un patient présentant un niveau élevé d'expression de protéine de la nectine 4 et/ou d'ARN dans un tissu tumoral, ledit procédé comprenant la sélection d'un patient présentant un Log2 du rapport de nombre copies de SDHC de 0,1 ou plus, 0,2 ou plus, 0,3 ou plus, 0,4 ou plus, 0,5 ou plus, 0,6 ou plus, 0,7 ou plus, 0,8 ou plus, 0,9 ou plus, ou 1,0 ou plus dans un tissu tumoral ou dans l'ADN tumoral circulant (ADNct) tel que déterminé par des technologies de séquençage de nouvelle génération (NGS) ou une capture de séquence basée sur une matrice, et l'administration au patient du conjugué toxine-complexe peptidique bicyclique ou du complexe peptidique bicyclique hétéroandémique spécifique pour la nectine 4, du sel pharmaceutiquement acceptable de celui-ci, ou de la composition pharmaceutique de celui-ci.

**8.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour une utilisation selon la revendication 7, dans lequel le cancer est un cancer du pancréas, un cancer de l'estomac, un cancer de la vessie, un cancer de la tête et du cou, un cancer du poumon non à petites cellules (CPNPC), un cancer du sein triple négatif (CSTN), ou un cancer de l'ovaire.

**9.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci destinés pour une utilisation selon la revendication 7 ou 8, dans lequel le nombre de copies d'ADN de SDHC est mesuré par séquençage de l'exome entier.

**10.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour une utilisation selon l'une quelconque des revendications 7 à 9, dans lequel le procédé comprend en outre l'administration d'un agent immuno-oncologique.

**11.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour une utilisation selon la revendication 10, dans lequel l'agent immuno-oncologique est un antagoniste de PD-1.

**12.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour une utilisation selon la revendication 11, dans lequel l'antagoniste de PD-1 est un anticorps antagoniste de PD-1.

**13.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour une utilisation selon la revendication 12, dans lequel l'anticorps antagoniste de PD-1 est sélectionné parmi le nivulomab, le pembrolizumab, MEDI-0680, le pidilizumab, AMP-224, l'atézolizumab, le durvalumab, BMS-936559, l'avélumab, ou le cémiplimab.

**14.** Conjugué toxine-complexe peptidique bicyclique ou complexe peptidique bicyclique hétéroandémique spécifique pour la nectine 4, ou sel pharmaceutiquement acceptable de celui-ci, ou composition pharmaceutique de celui-ci pour une utilisation selon l'une quelconque des revendications 7 à 13, dans lequel le conjugué toxine-complexe peptidique bicyclique spécifique pour la nectine 4 est BT8009 ou le complexe peptidique bicyclique hétéroandémique spécifique pour la nectine 4 est BT7480.

FIG. 1

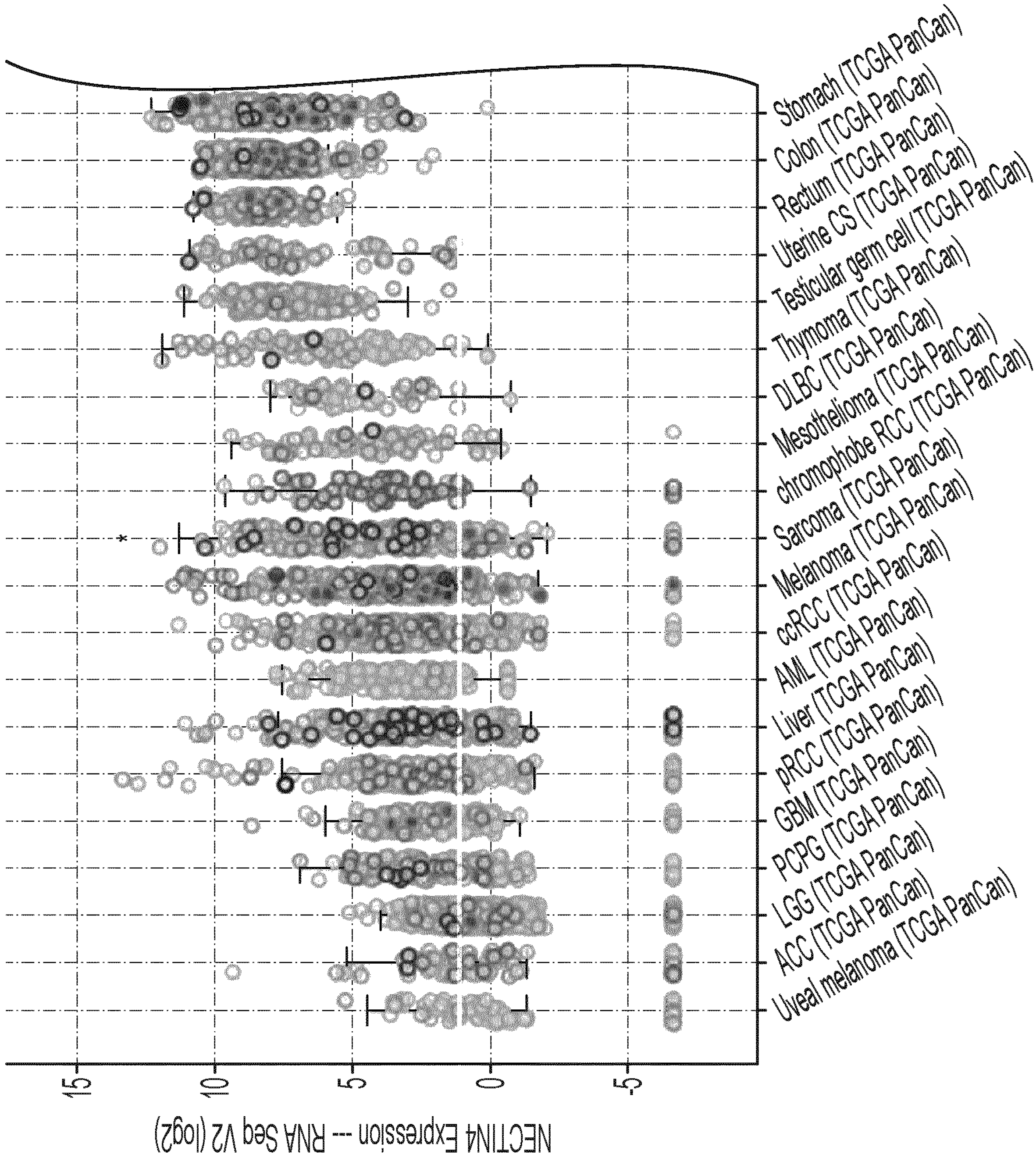
NECTIN4 Expression --- RNA Seq V2 (log2)
15
10
5
0
-5
Stomach (TCGA PanCan)
Colon (TCGA PanCan)
Rectum (TCGA PanCan)
Uterine CS (TCGA PanCan)
Testicular germ cell (TCGA PanCan)
Thymoma (TCGA PanCan)
DLBC (TCGA PanCan)
Mesothelioma (TCGA PanCan)
chromophobe RCC (TCGA PanCan)
Sarcoma (TCGA PanCan)
Melanoma (TCGA PanCan)
ccRCC (TCGA PanCan)
AML (TCGA PanCan)
Liver (TCGA PanCan)
pRCC (TCGA PanCan)
GBM (TCGA PanCan)
PCPG (TCGA PanCan)
LGG (TCGA PanCan)
ACC (TCGA PanCan)
Uveal melanoma (TCGA PanCan)

FIG. 1 CONTINUED

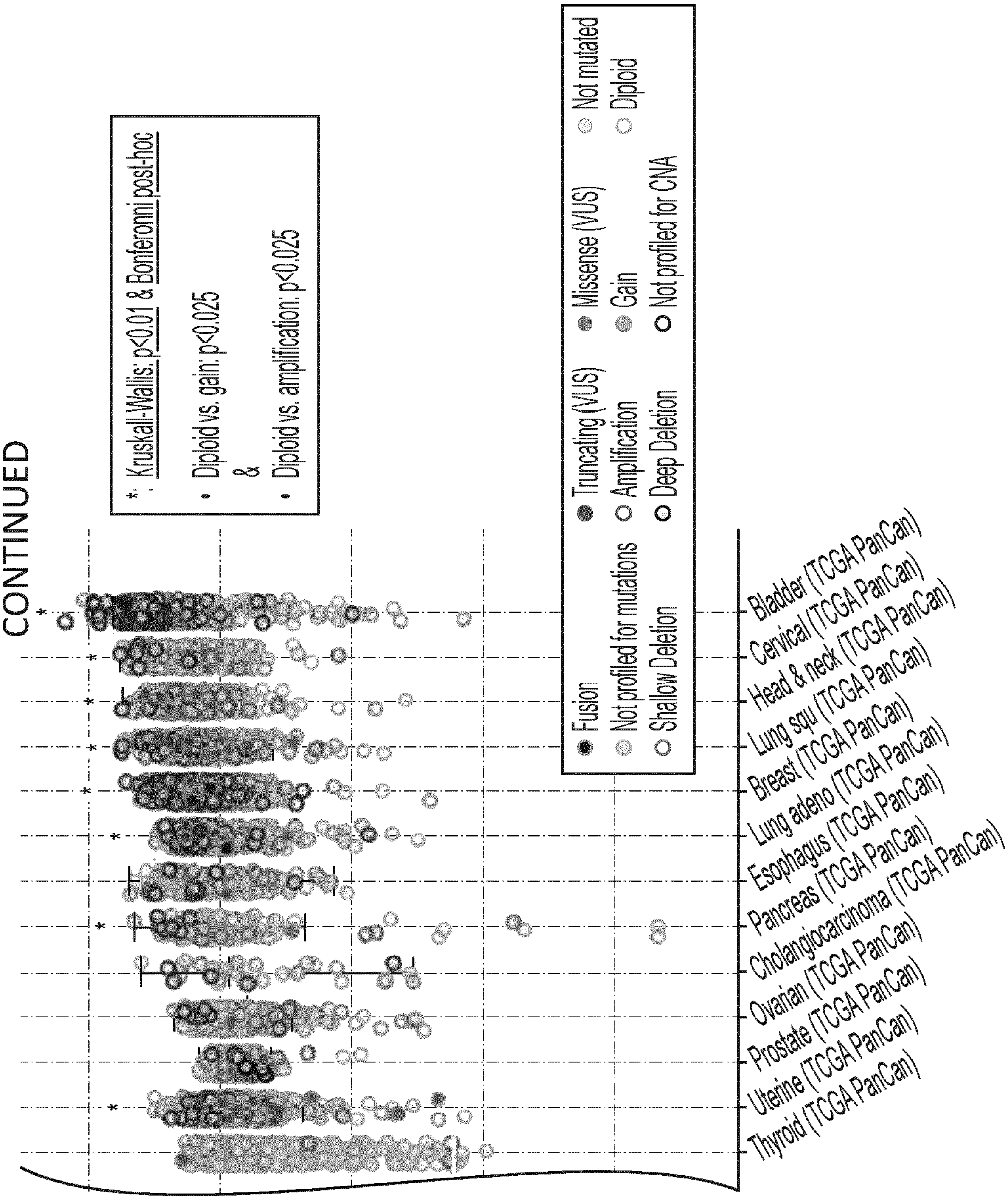

FIG. 2A

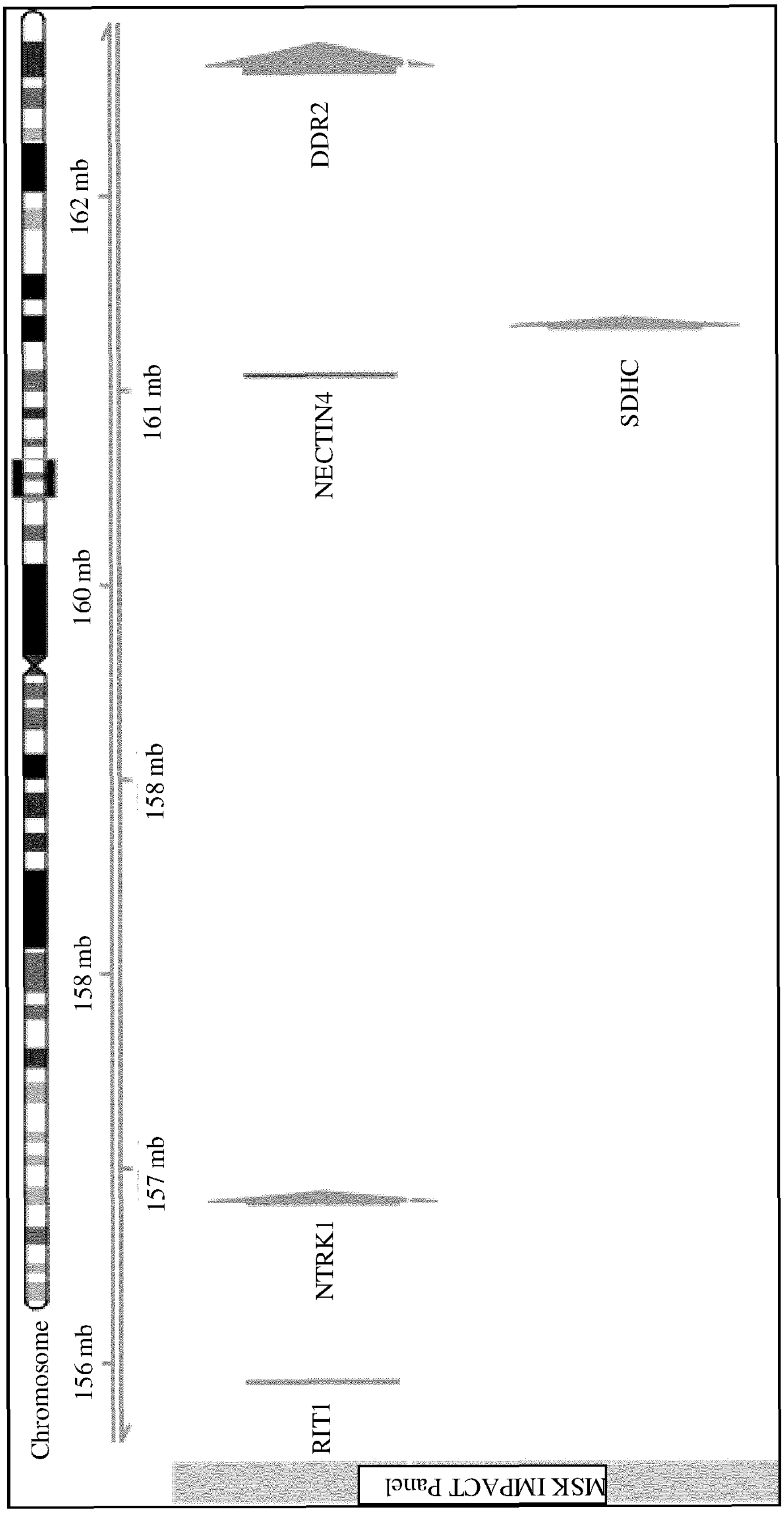
Chromosome
156 mb
157 mb
158 mb
158 mb
160 mb
161 mb
162 mb
RIT1
NTRK1
NECTIN4
DDR2
SDHC
MSK IMPACT Panel

FIG. 2B

| Gene | Description | Genomic Location | Distance to NECTIN4 |
|---|---|---|---|
| NECTIN4 | Nectin cell adhesion molecule 4 | 1:161070995-161089599:-1 | - |
| SDHC | Succinate dehydrogenase complex subunit C | 1:161314257-161375340:1 | 225 kb |
| DDR2 | Discoidin domain receptor tyrosine kinase 2 | 1:162631373-162787400:1 | 1.6 Mb |

FIG. 3A

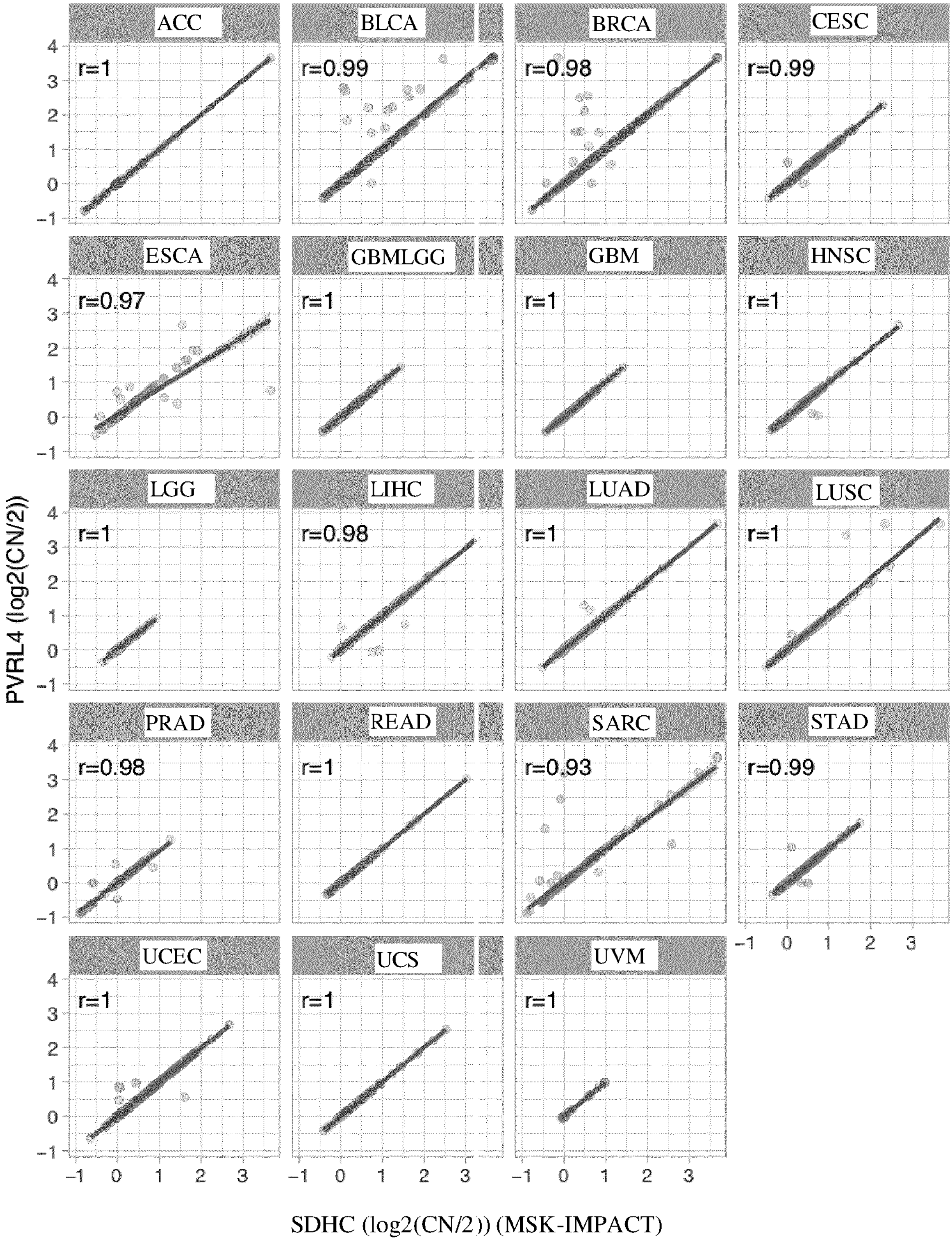
ACC
BLCA
BRCA
CESC
ESCA
GBMLGG
GBM
HNSC
LGG
LIHC
LUAD
LUSC
PRAD
READ
SARC
STAD
UCEC
UCS
UVM
r=1
r=0.99
r=0.98
r=0.99
r=0.97
r=1
r=1
r=1
r=1
r=0.98
r=1
r=1
r=0.98
r=1
r=0.93
r=0.99
r=1
r=1
r=1
PVRL4 (log2(CN/2))
SDHC (log2(CN/2)) (MSK-IMPACT)
4
3
2
1
0
−1
−1
0
1
2
3

FIG. 3A (continued)

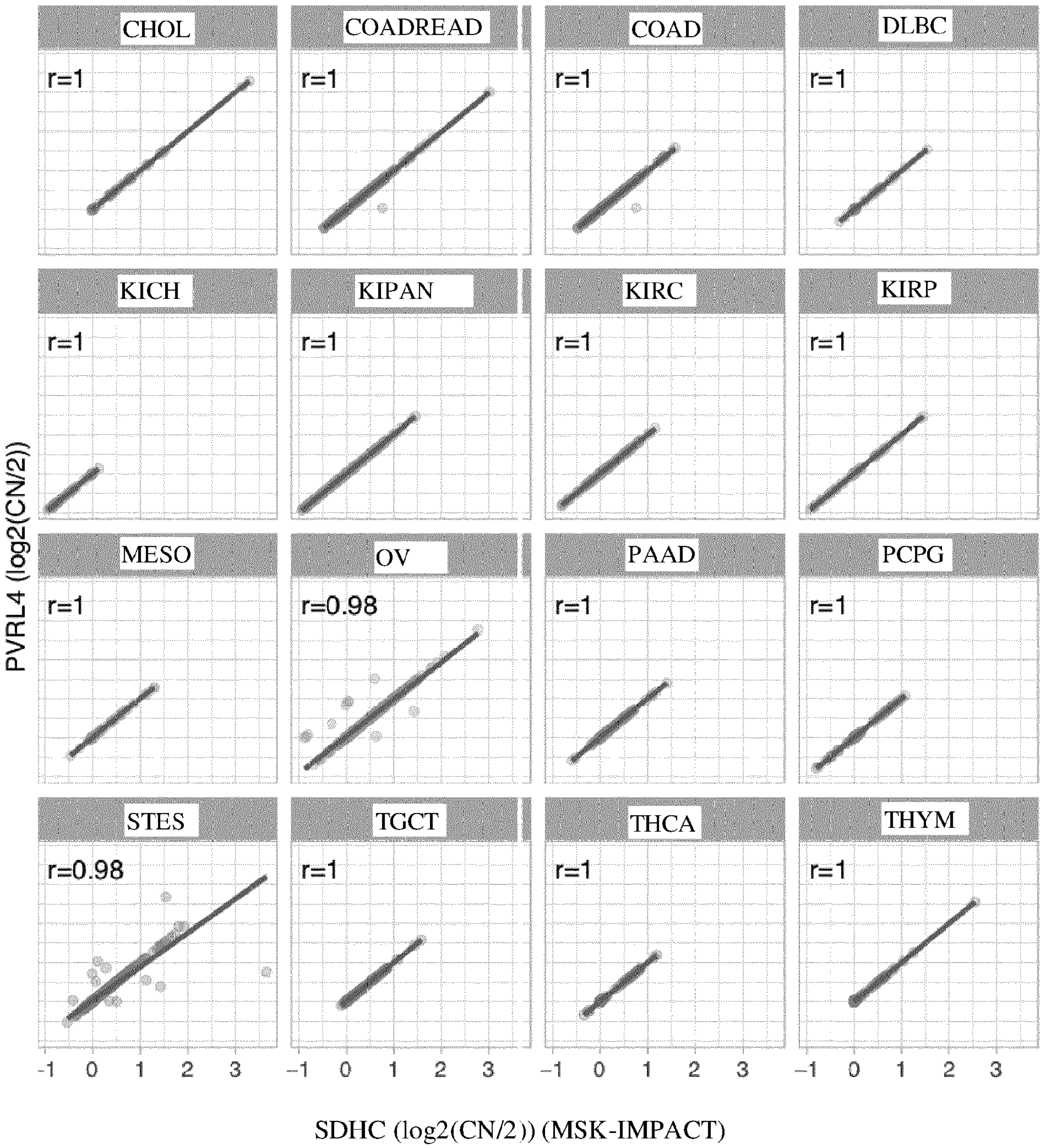

FIG. 3B

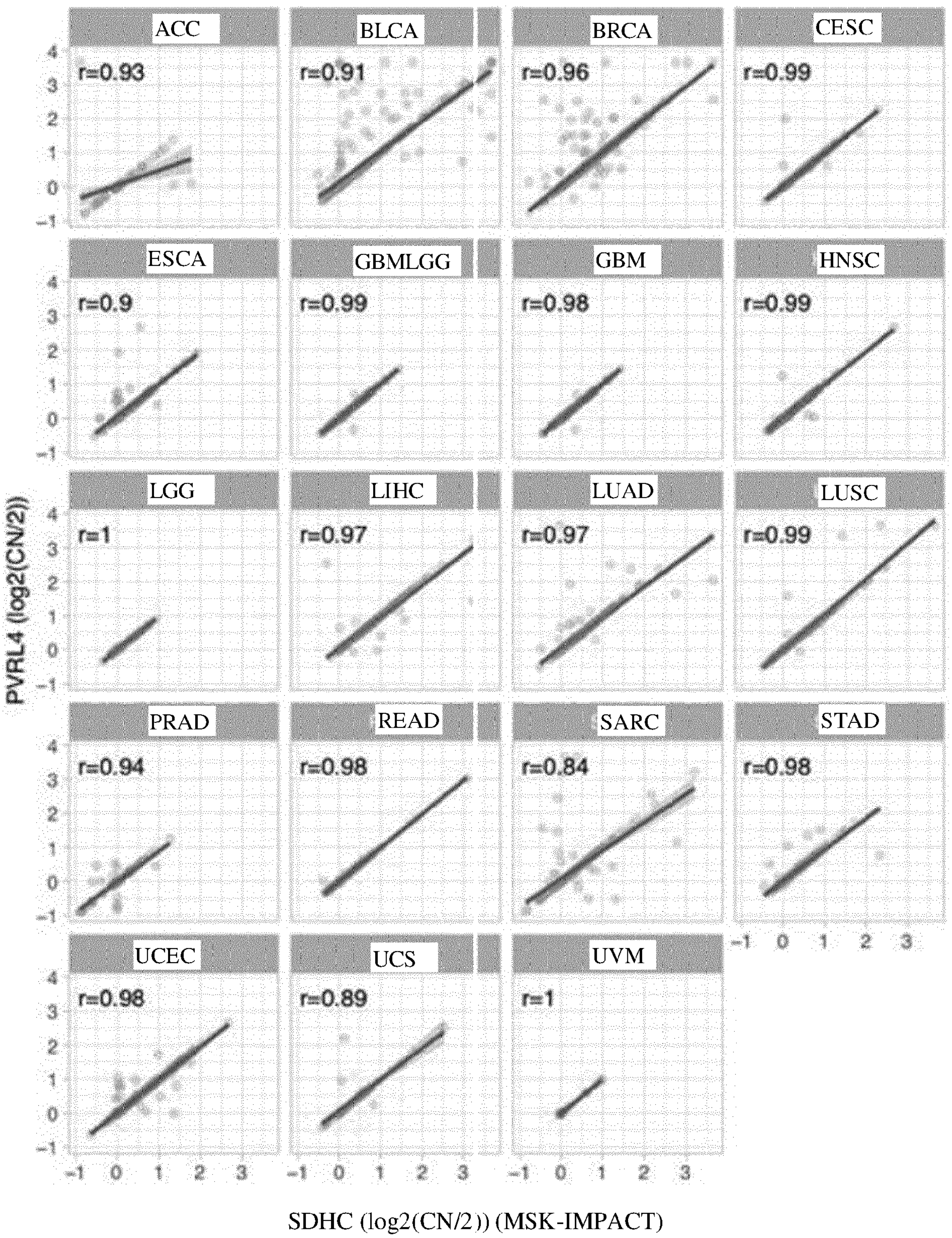
ACC
r=0.93
BLCA
r=0.91
BRCA
r=0.96
CESC
r=0.99
ESCA
r=0.9
GBMLGG
r=0.99
GBM
r=0.98
HNSC
r=0.99
LGG
r=1
LIHC
r=0.97
LUAD
r=0.97
LUSC
r=0.99
PRAD
r=0.94
READ
r=0.98
SARC
r=0.84
STAD
r=0.98
UCEC
r=0.98
UCS
r=0.89
UVM
r=1
PVRL4 (log2(CN/2))
SDHC (log2(CN/2)) (MSK-IMPACT)

FIG. 3B (continued)

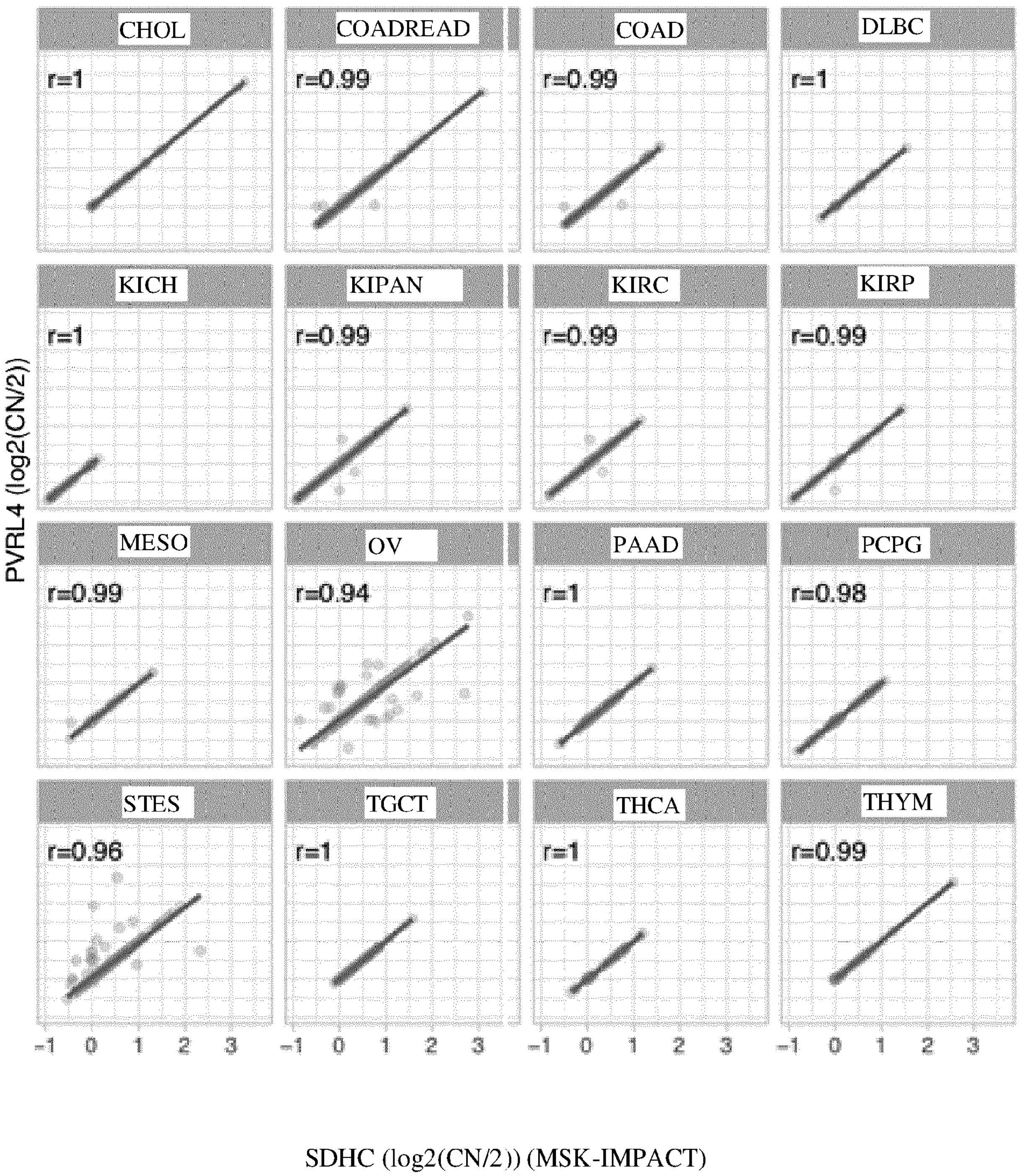

FIG. 4

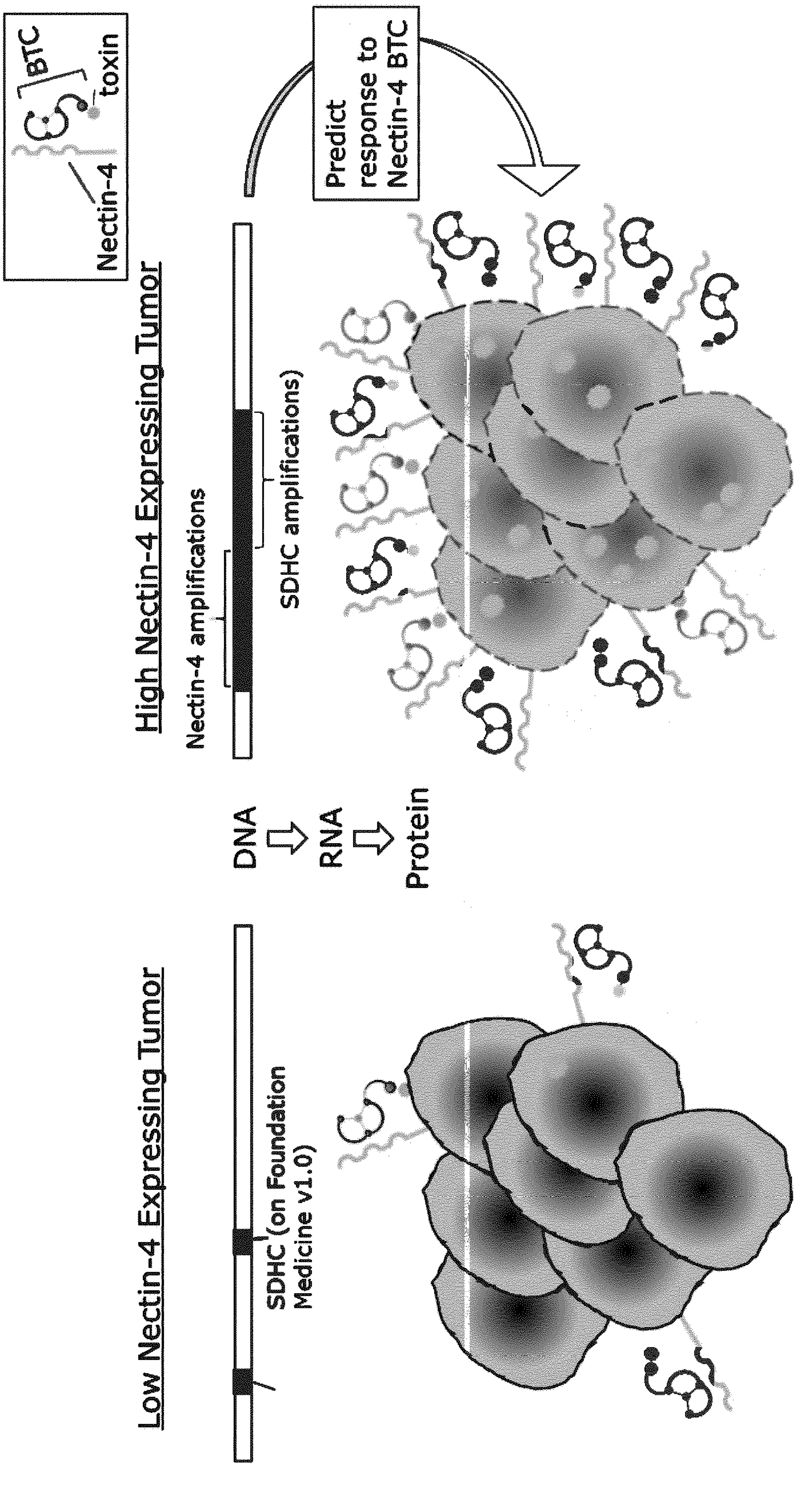
Nectin-4
BTC
toxin
High Nectin-4 Expressing Tumor
Nectin-4 amplifications
SDHC amplifications)
Predict response to Nectin-4 BTC
DNA
RNA
Protein
Low Nectin-4 Expressing Tumor
SDHC (on Foundation Medicine v1.0)

FIG. 5A

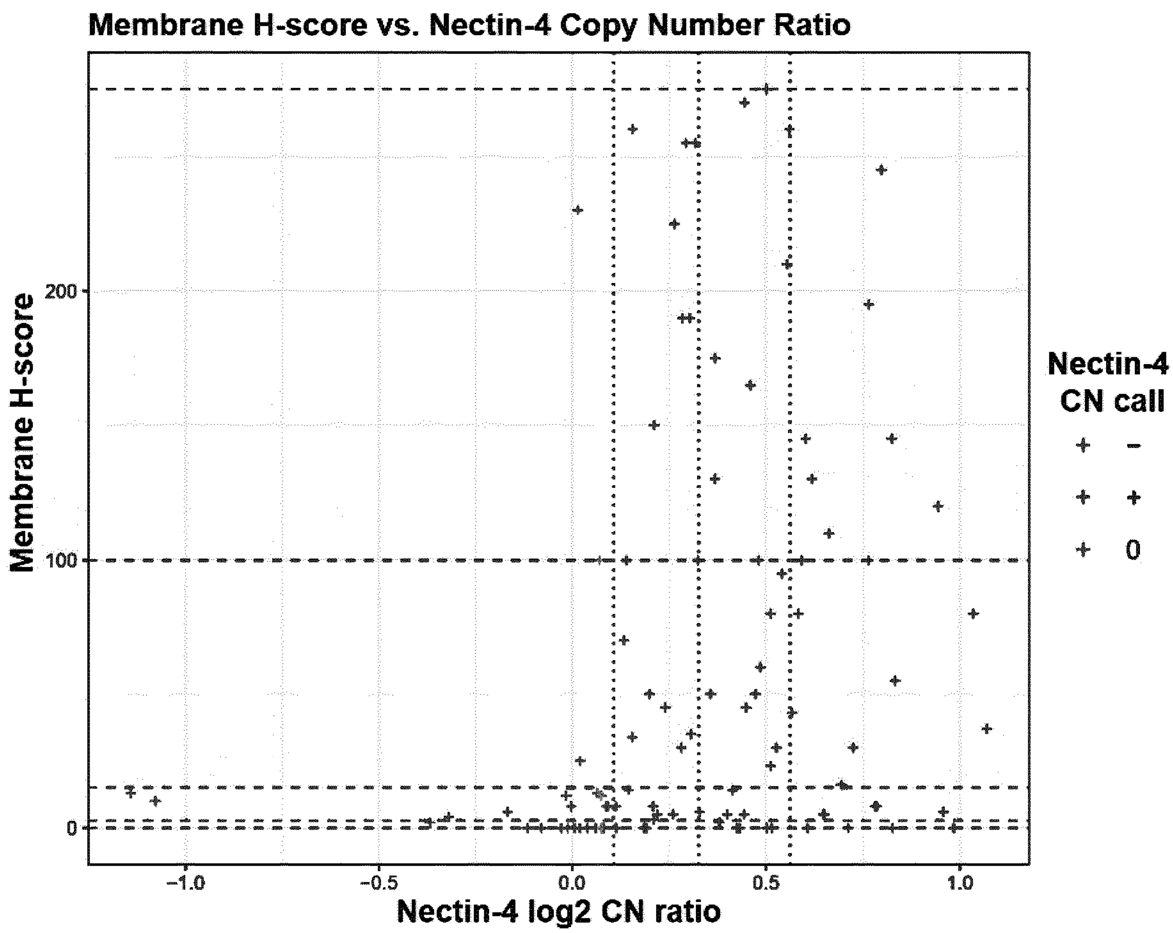
Membrane H-score vs. Nectin-4 Copy Number Ratio
Membrane H-score
200
100
0
-1.0
-0.5
0.0
0.5
1.0
Nectin-4 log2 CN ratio
Nectin-4
CN call
-
+
0

FIG. 5B

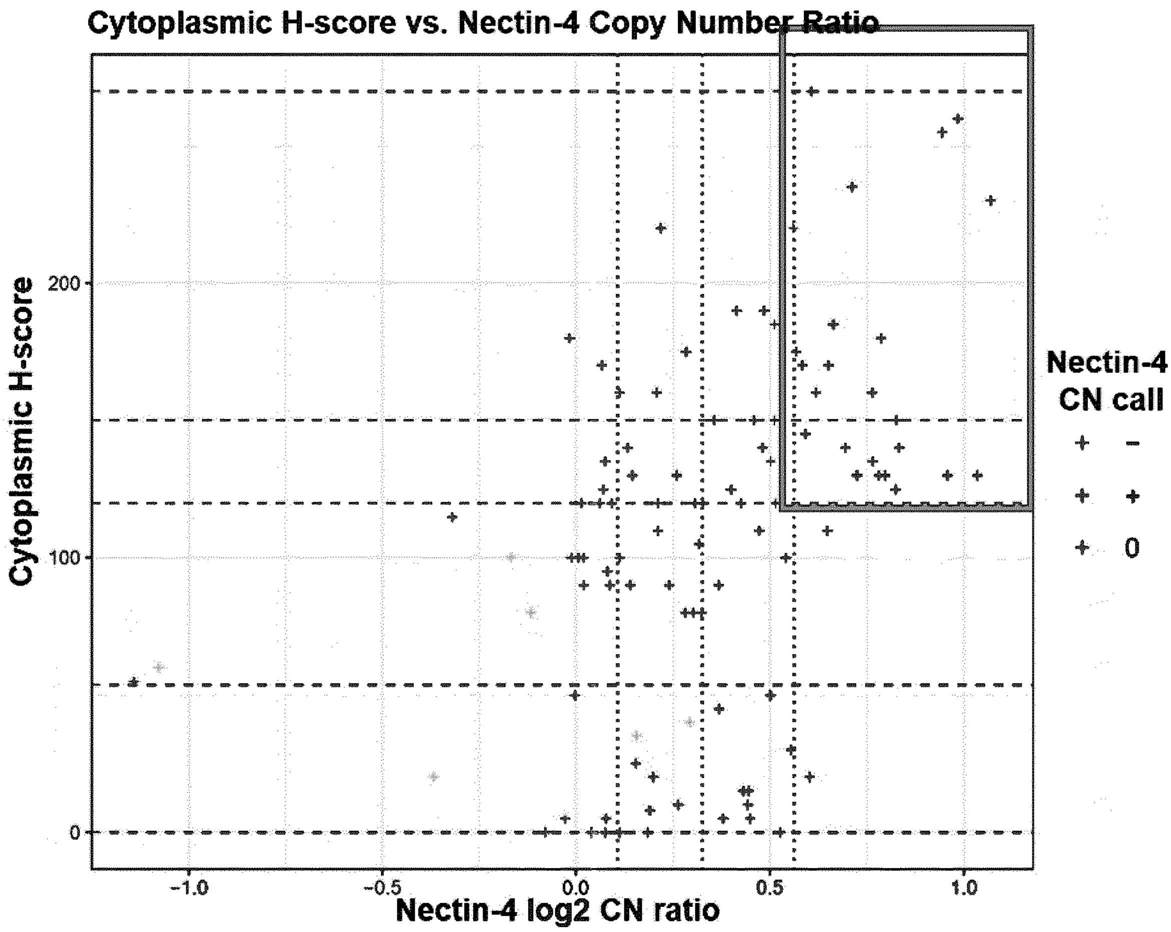

Cytoplasmic H-score vs. Nectin-4 Copy Number Ratio
Cytoplasmic H-score
200
100
0
-1.0
-0.5
0.0
0.5
1.0
Nectin-4 log2 CN ratio
Nectin-4
CN call
-
+
0

FIG. 5C

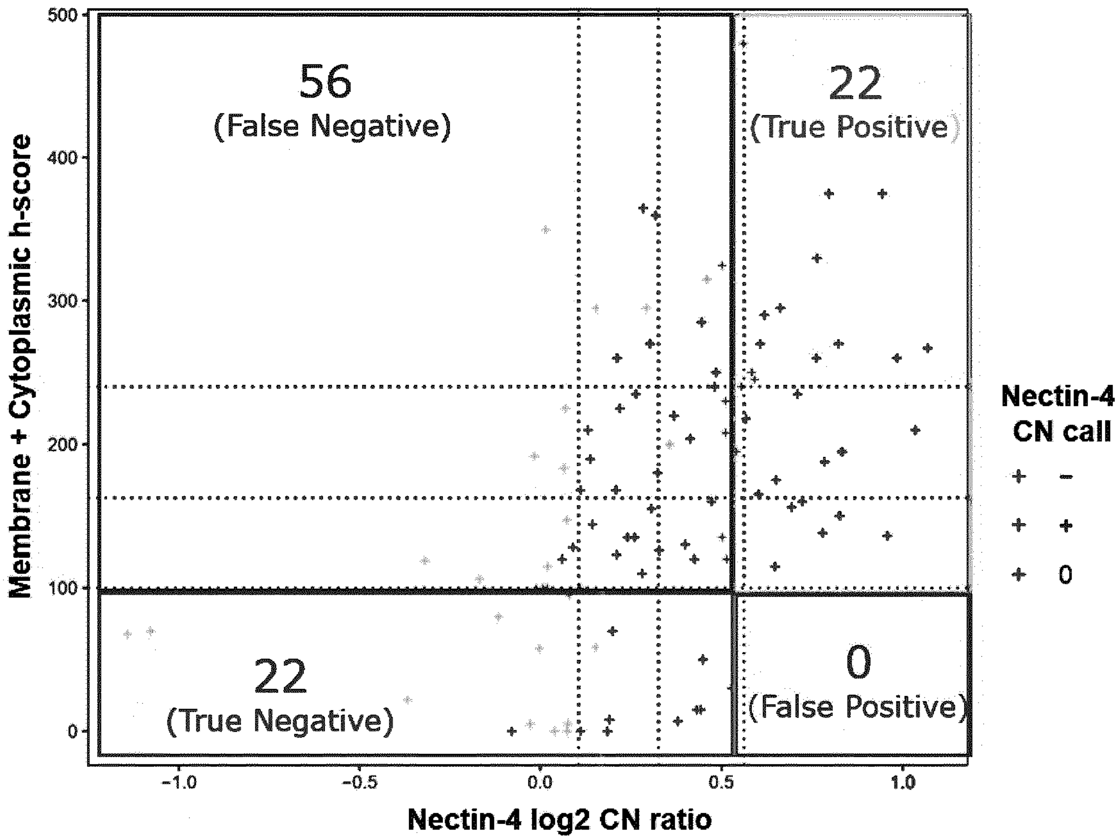
56
(False Negative)
22
(True Positive)
22
(True Negative)
0
(False Positive)
Membrane + Cytoplasmic h-score
500
400
300
200
100
0
-1.0
-0.5
0.0
0.5
1.0
Nectin-4 log2 CN ratio
Nectin-4
CN call
+ −
+ +
+ 0

FIG. 5D

| Test | $H_{TM+TC} \geq 100$ | $H_{TM+TC} < 100$ | Total |
|---|---|---|---|
| CN ≥ 3 (positive) | a=22 (True Positive) | c=0 (False Positive) | a+c=**22** |
| CN < 3 (negative) | b=56 (False Negative) | d=22 (True Negative) | b+d=**78** |
| Total: | a+b=**78** | c+d=**22** | |

FIG 6A

**Analysis of potential association between Nectin-4 membrane + cytoplasmic H-score and copy number**

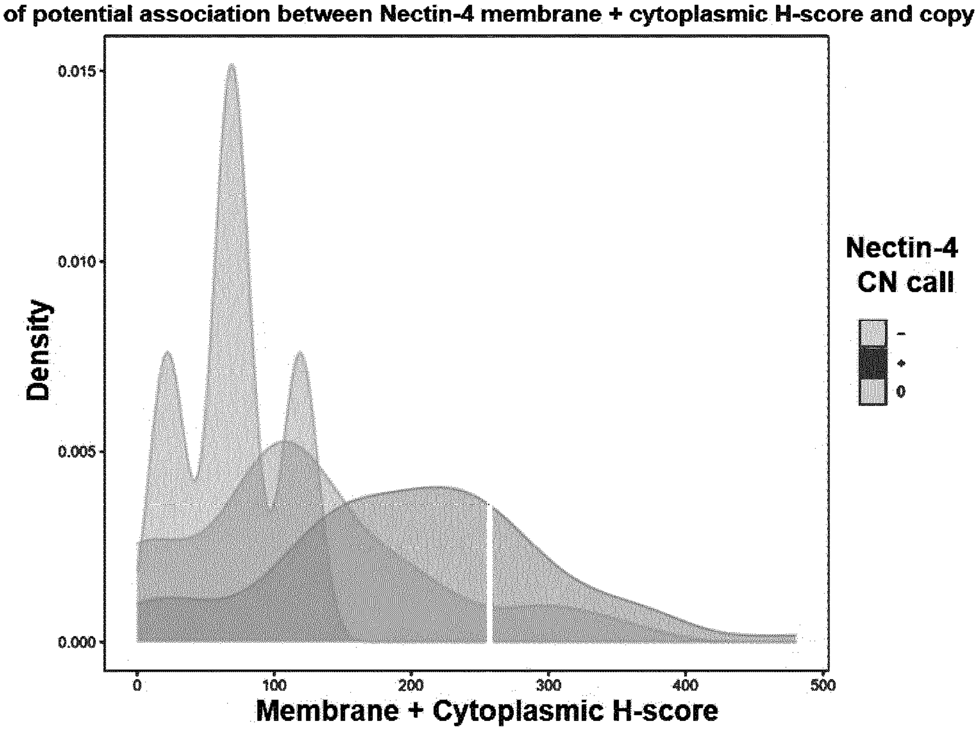


FIG. 6B

| I cutoff | + | + % | 0 | 0 % | - | - % |
|---|---|---|---|---|---|---|
| 0 | 66 | 100 | 30 | 100 | 4 | 100 |
| 50 | 60 | 90.9 | 24 | 80.0 | 3 | 75 |
| 100 | 58 | 87.9 | 19 | 63.3 | 1 | 25 |
| 150 | 47 | 71.2 | 8 | 26.7 | 0 | 0 |
| 200 | 34 | 51.5 | 5 | 16.7 | 0 | 0 |
| 250 | 20 | 30.3 | 3 | 10.0 | 0 | 0 |
| 300 | 8 | 12.1 | 1 | 3.3 | 0 | 0 |
| 350 | 5 | 7.6 | 1 | 3.3 | 0 | 0 |
| 400 | 1 | 1.5 | 0 | 0.0 | 0 | 0 |
| 450 | 1 | 1.5 | 0 | 0.0 | 0 | 0 |
| 500 | 0 | 0.0 | 0 | 0.0 | 0 | 0 |
| 550 | 0 | 0.0 | 0 | 0.0 | 0 | 0 |
| 600 | 0 | 0.0 | 0 | 0.0 | 0 | 0 |

FIG. 7A

## Membrane + Cytoplasmic

| Nectin-4 log2 CN ratio cutoff | | | | | | | | | | | | H-score |
|---|---|---|---|---|---|---|---|---|---|---|---|---|
| All | ≥ 0 | ≥ 0.1 | ≥ 0.2 | ≥ 0.3 | ≥ 0.4 | ≥ 0.5 | ≥ 0.6 | ≥ 0.7 | ≥ 0.8 | ≥ 0.9 | ≥ 1.0 | Cutoff |
| 100 | 89 | 75 | 64 | 54 | 44 | 34 | 22 | 15 | 8 | 5 | 2 | 0 |
| 87 | 79 | 68 | 60 | 50 | 41 | 33 | 22 | 15 | 8 | 5 | 2 | 50 |
| 78 | 74 | 65 | 59 | 49 | 40 | 33 | 22 | 15 | 8 | 5 | 2 | 100 |
| 55 | 54 | 51 | 47 | 41 | 34 | 28 | 19 | 13 | 7 | 4 | 2 | 150 |
| 39 | 39 | 37 | 35 | 30 | 25 | 20 | 12 | 9 | 5 | 4 | 2 | 200 |
| 23 | 23 | 22 | 21 | 18 | 16 | 13 | 10 | 7 | 4 | 3 | 1 | 250 |
| 9 | 9 | 8 | 8 | 7 | 6 | 5 | 3 | 3 | 1 | 1 | 0 | 300 |
| 6 | 6 | 5 | 5 | 4 | 3 | 3 | 2 | 2 | 1 | 1 | 0 | 350 |
| 1 | 1 | 1 | 1 | 1 | 1 | 1 | 0 | 0 | 0 | 0 | 0 | 400 |
| 1 | 1 | 1 | 1 | 1 | 1 | 1 | 0 | 0 | 0 | 0 | 0 | 450 |
| 0 | 0 | 0 | 0 | 0 | 0 | 0 | 0 | 0 | 0 | 0 | 0 | 500 |
| 0 | 0 | 0 | 0 | 0 | 0 | 0 | 0 | 0 | 0 | 0 | 0 | 550 |
| 0 | 0 | 0 | 0 | 0 | 0 | 0 | 0 | 0 | 0 | 0 | 0 | 600 |

FIG. 7B

## Membrane

| Nectin-4 log2 CN ratio cutoff | | | | | | | | | | | | H-score |
|---|---|---|---|---|---|---|---|---|---|---|---|---|
| All | ≥ 0 | ≥ 0.1 | ≥ 0.2 | ≥ 0.3 | ≥ 0.4 | ≥ 0.5 | ≥ 0.6 | ≥ 0.7 | ≥ 0.8 | ≥ 0.9 | ≥ 1.0 | Cutoff |
| 100 | 89 | 75 | 64 | 54 | 44 | 34 | 22 | 15 | 8 | 5 | 2 | 0 |
| 48 | 48 | 45 | 40 | 34 | 27 | 21 | 12 | 9 | 5 | 3 | 2 | 25 |
| 38 | 38 | 36 | 32 | 28 | 22 | 17 | 10 | 7 | 4 | 2 | 1 | 50 |
| 32 | 32 | 30 | 28 | 24 | 19 | 16 | 9 | 6 | 3 | 2 | 1 | 75 |
| 28 | 28 | 26 | 24 | 20 | 15 | 12 | 8 | 5 | 2 | 1 | 0 | 100 |
| 20 | 20 | 19 | 18 | 14 | 10 | 8 | 5 | 3 | 1 | 0 | 0 | 125 |
| 16 | 16 | 15 | 14 | 10 | 7 | 5 | 2 | 2 | 0 | 0 | 0 | 150 |
| 14 | 14 | 13 | 12 | 9 | 6 | 5 | 2 | 2 | 0 | 0 | 0 | 175 |
| 10 | 10 | 9 | 8 | 6 | 5 | 4 | 1 | 1 | 0 | 0 | 0 | 200 |
| 9 | 9 | 8 | 7 | 5 | 4 | 3 | 1 | 1 | 0 | 0 | 0 | 225 |
| 6 | 6 | 6 | 5 | 4 | 3 | 2 | 0 | 0 | 0 | 0 | 0 | 250 |
| 1 | 1 | 1 | 1 | 1 | 1 | 1 | 0 | 0 | 0 | 0 | 0 | 275 |
| 0 | 0 | 0 | 0 | 0 | 0 | 0 | 0 | 0 | 0 | 0 | 0 | 300 |

FIG. 7C

**Cytoplasmic**

| Nectin-4 log2 CN ratio cutoff | | | | | | | | | | | | H-score |
|---|---|---|---|---|---|---|---|---|---|---|---|---|
| All | ≥ 0 | ≥ 0.1 | ≥ 0.2 | ≥ 0.3 | ≥ 0.4 | ≥ 0.5 | ≥ 0.6 | ≥ 0.7 | ≥ 0.8 | ≥ 0.9 | ≥ 1.0 | Cutoff |
| 100 | 89 | 75 | 64 | 54 | 44 | 34 | 22 | 15 | 8 | 5 | 2 | 0 |
| 82 | 74 | 63 | 56 | 47 | 38 | 32 | 21 | 15 | 8 | 5 | 2 | 25 |
| 77 | 69 | 58 | 53 | 45 | 37 | 31 | 21 | 15 | 8 | 5 | 2 | 50 |
| 73 | 68 | 57 | 52 | 44 | 36 | 30 | 21 | 15 | 8 | 5 | 2 | 75 |
| 63 | 59 | 51 | 47 | 41 | 36 | 30 | 21 | 15 | 8 | 5 | 2 | 100 |
| 44 | 43 | 40 | 37 | 33 | 31 | 27 | 20 | 15 | 8 | 5 | 2 | 125 |
| 26 | 25 | 24 | 23 | 20 | 19 | 16 | 11 | 7 | 4 | 3 | 1 | 150 |
| 15 | 14 | 14 | 14 | 12 | 12 | 10 | 7 | 5 | 3 | 3 | 1 | 175 |
| 7 | 7 | 7 | 7 | 6 | 6 | 6 | 5 | 4 | 3 | 3 | 1 | 200 |
| 5 | 5 | 5 | 5 | 5 | 5 | 5 | 5 | 4 | 3 | 3 | 1 | 225 |
| 3 | 3 | 3 | 3 | 3 | 3 | 3 | 3 | 2 | 2 | 2 | 0 | 250 |
| 0 | 0 | 0 | 0 | 0 | 0 | 0 | 0 | 0 | 0 | 0 | 0 | 275 |
| 0 | 0 | 0 | 0 | 0 | 0 | 0 | 0 | 0 | 0 | 0 | 0 | 300 |

FIG. 7D

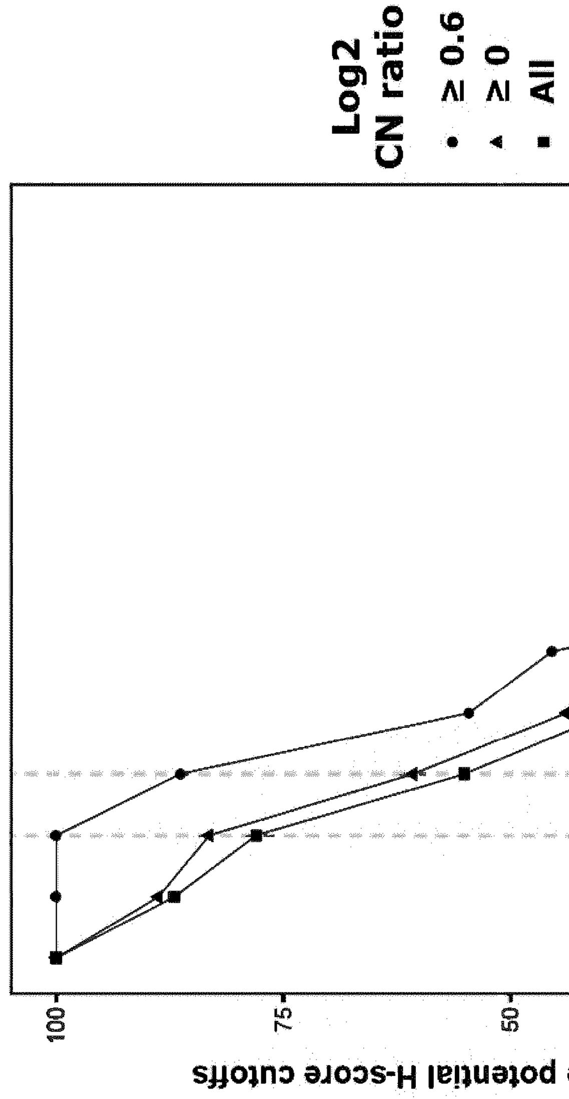
% samples above potential H-score cutoffs
100
75
50
25
0
0
50
100
150
200
250
300
350
400
450
500
550
600
H-Score Cutoff
Log2
CN ratio
≥ 0.6
≥ 0
All

FIG. 8A

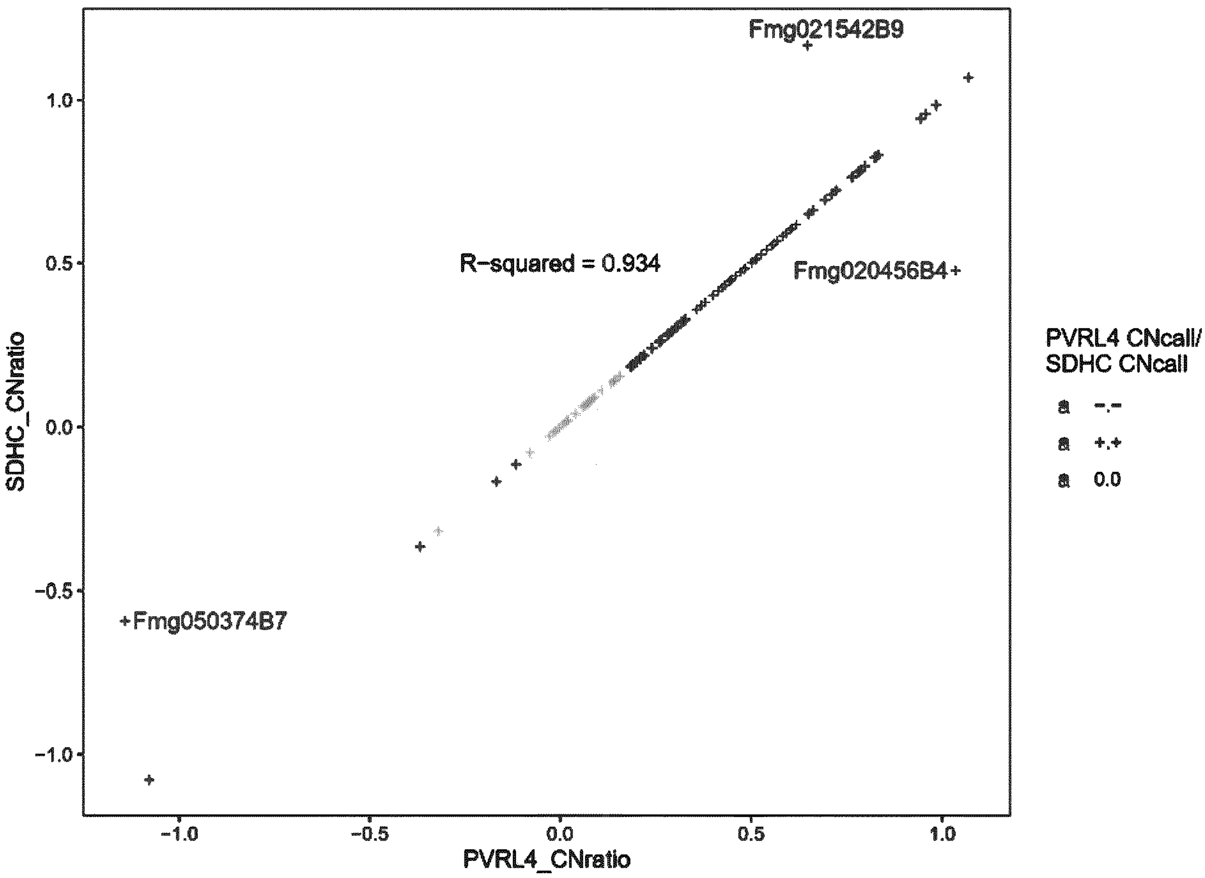
Fmg021542B9
R−squared = 0.934
Fmg020456B4+
+Fmg050374B7
SDHC_CNratio
PVRL4_CNratio
1.0
0.5
0.0
−0.5
−1.0
−1.0
−0.5
0.0
0.5
1.0
PVRL4 CNcall/
SDHC CNcall
a −.−
a +.+
a 0.0

FIG. 8B

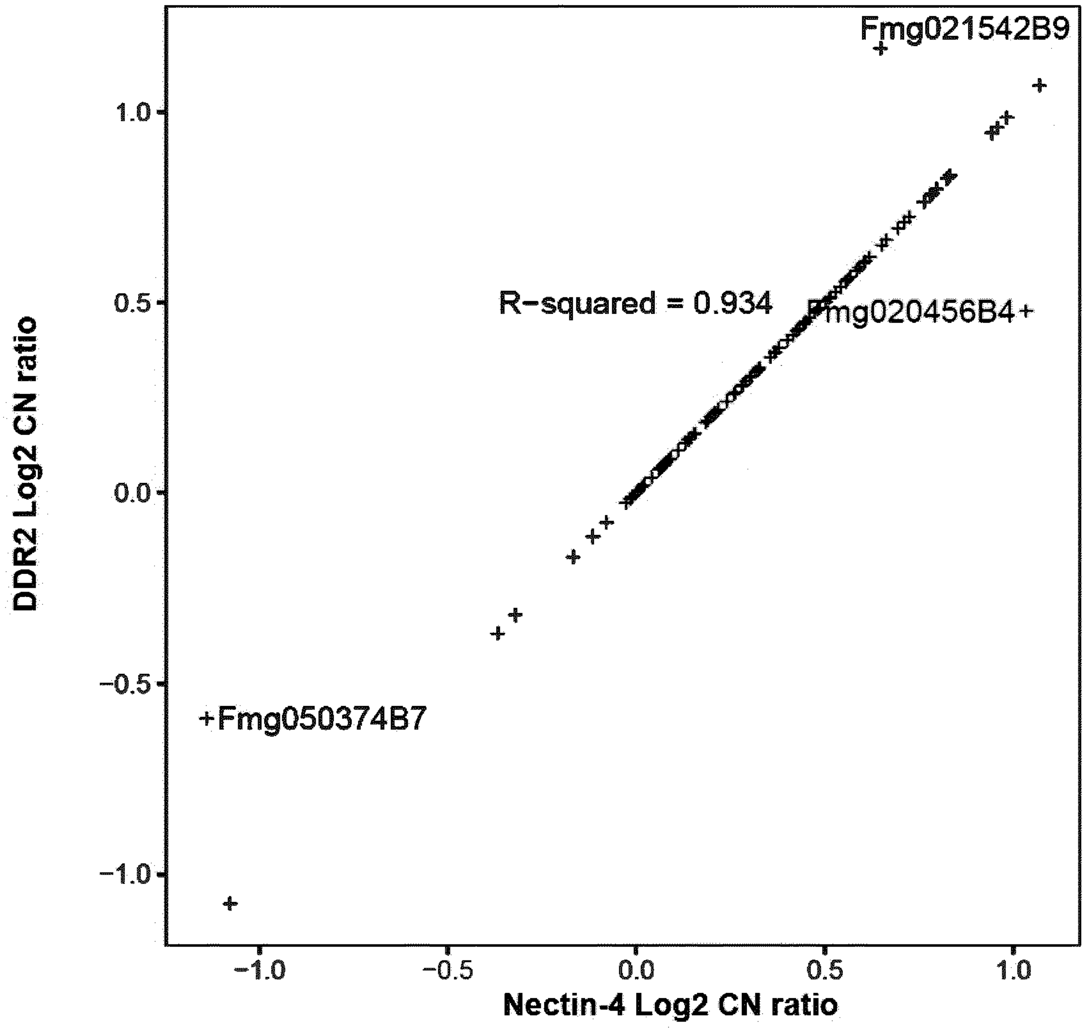
Fmg021542B9
R−squared = 0.934
Fmg020456B4
Fmg050374B7
1.0
0.5
0.0
−0.5
−1.0
DDR2 Log2 CN ratio
−1.0
−0.5
0.0
0.5
1.0
Nectin-4 Log2 CN ratio

FIG. 9

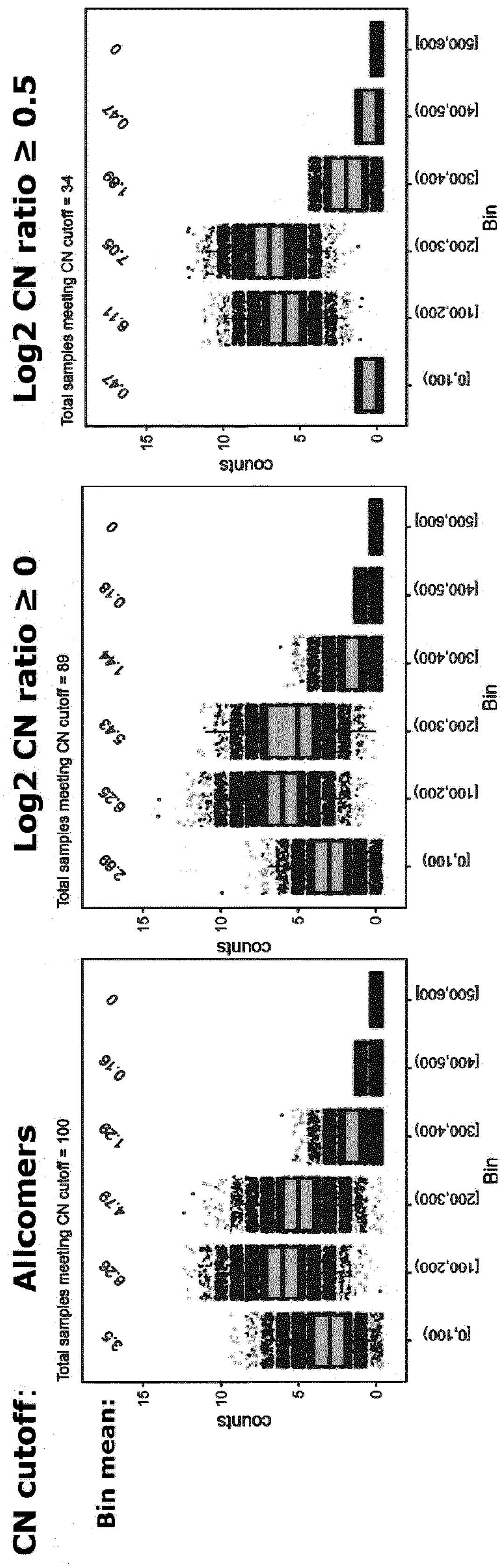
CN cutoff:
Bin mean:
Allcomers
Total samples meeting CN cutoff = 100
3.5
6.26
4.79
1.29
0.16
0
Log2 CN ratio ≥ 0
Total samples meeting CN cutoff = 89
2.89
6.25
5.43
1.44
0.18
0
Log2 CN ratio ≥ 0.5
Total samples meeting CN cutoff = 34
0.47
6.11
7.05
1.89
0.47
0
counts
15
10
5
0
Bin
[0,100)
[100,200)
[200,300)
[300,400)
[400,500)
[500,600]

FIG. 10A

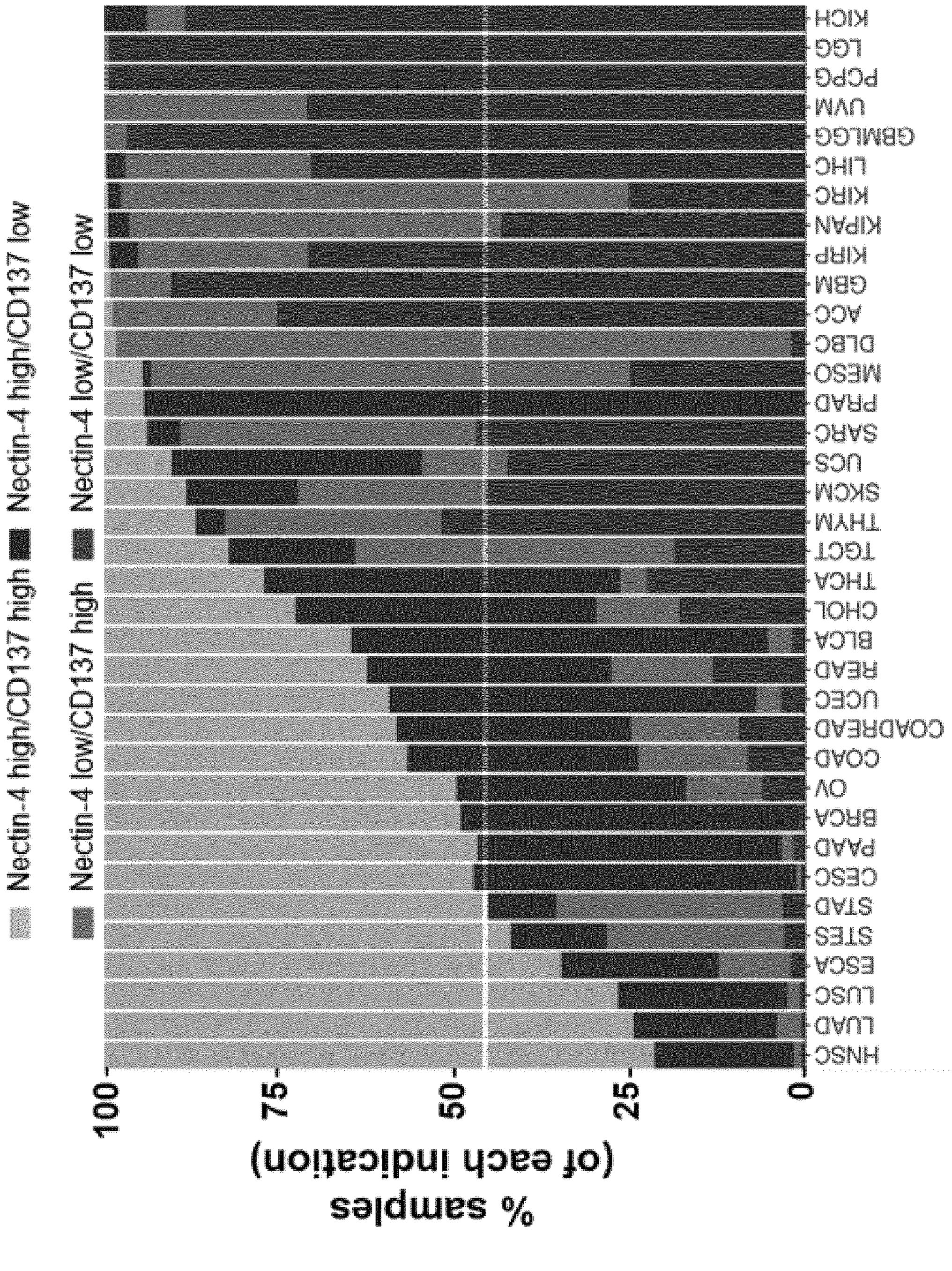
Nectin-4 high/CD137 high
Nectin-4 high/CD137 low
Nectin-4 low/CD137 high
Nectin-4 low/CD137 low
% samples (of each indication)
100
75
50
25
0
HNSC
LUAD
LUSC
ESCA
STES
STAD
CESC
PAAD
BRCA
OV
COAD
COADREAD
UCEC
READ
BLCA
CHOL
THCA
TGCT
THYM
SKCM
UCS
SARC
PRAD
MESO
DLBC
ACC
GBM
KIRP
KIPAN
KIRC
LIHC
GBMLGG
UVM
PCPG
LGG
KICH

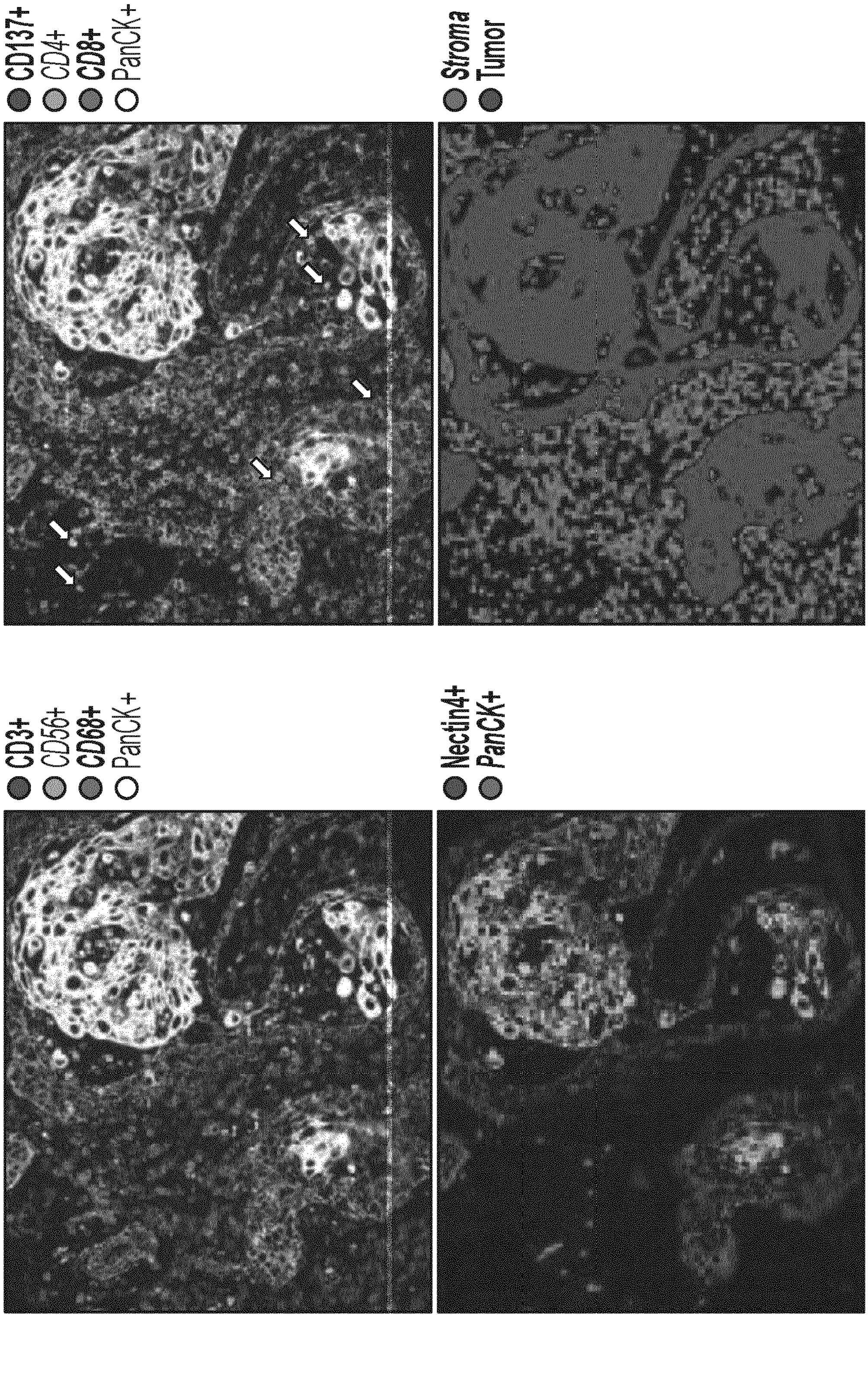
CD137+
CD4+
CD8+
PanCK+
Stroma
Tumor
CD3+
CD56+
CD68+
PanCK+
Nectin4+
PanCK+

FIG. 10B

FIG. 10C

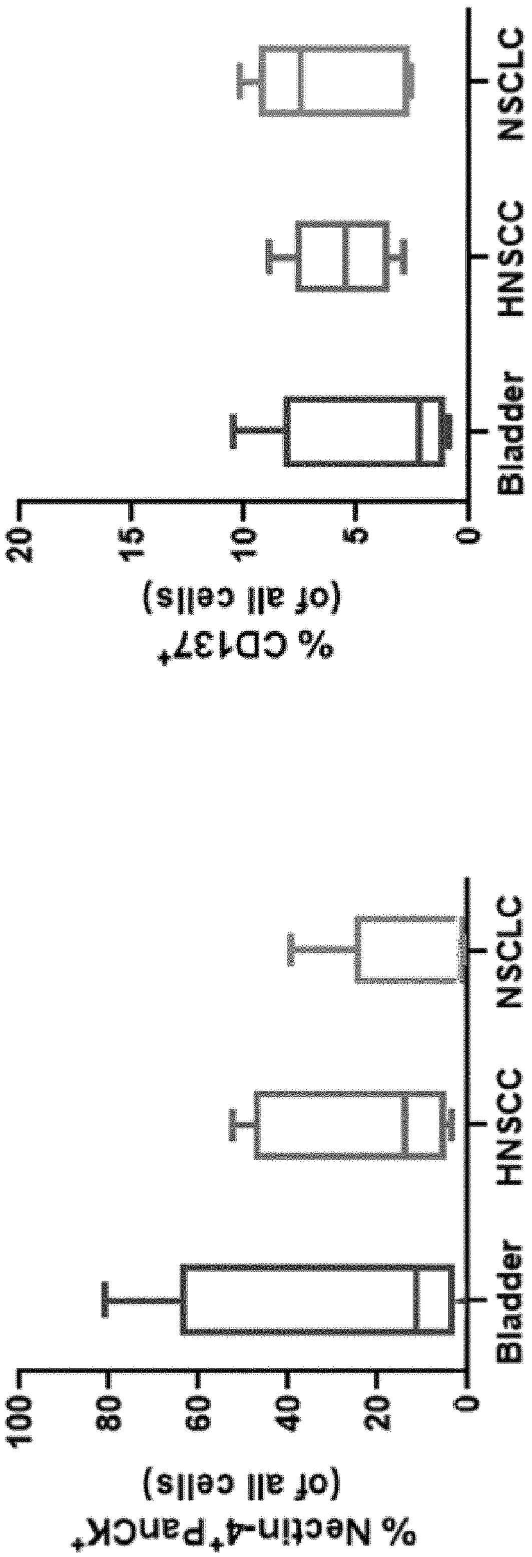
% CD137+
(of all cells)
20
15
10
5
0
Bladder
HNSCC
NSCLC
% Nectin-4+PanCK+
(of all cells)
100
80
60
40
20
0
Bladder
HNSCC
NSCLC

FIG. 10D

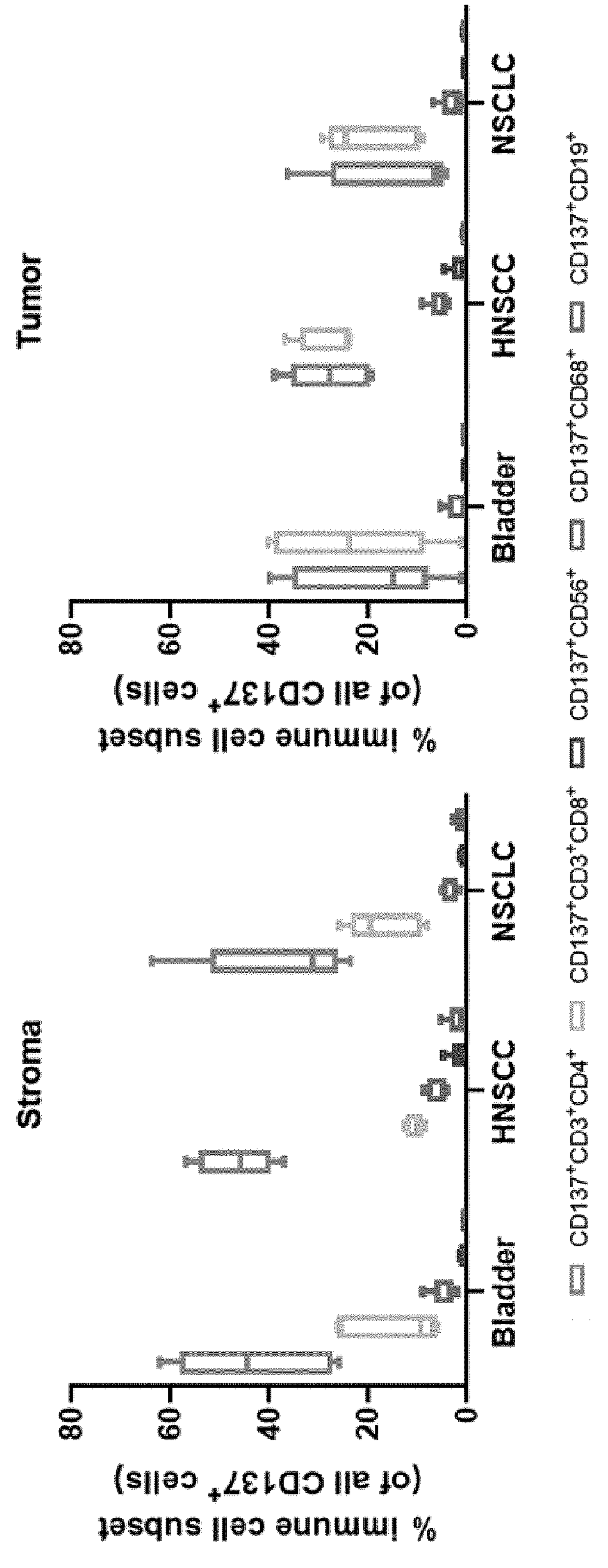
Stroma
Tumor
% immune cell subset
(of all CD137+ cells)
0
20
40
60
80
Bladder
HNSCC
NSCLC
CD137+CD3+CD4+
CD137+CD3+CD8+
CD137+CD56+
CD137+CD68+
CD137+CD19+

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- WO 2009098450 A
- US 20190389906 A
- US 201903889906 A
- WO 2019243832 A
- WO 2019243833 A
- US 20190307836 A
- WO 2019193328 A
- US 20210040154 A
- WO 2021019244 A
- WO 2021019246 A
- WO 2008118802 A
- US 7390799 B
- WO 2004106328 A
- WO 2008039218 A
- WO 2011090760 A
- WO 2003063794 A
- WO 2005007623 A
- WO 2006078846 A
- WO 2004019973 A
- WO 2004089925 A
- WO 2007016176 A
- US 8138347 B
- WO 2002088112 A
- WO 2007084786 A
- WO 2007129161 A
- WO 2006122806 A
- WO 2005113554 A
- WO 2007044729 A
- WO 2009114512 A
- WO 2008109943 A
- WO 2007053452 A
- WO 2000142246 A
- WO 2007070514 A
- US 6552065 B
- WO 1170024 A
- WO 11107553 A
- WO 11131407 A
- WO 1387699 A
- WO 13119716 A
- WO 13132044 A
- WO 11140249 A
- WO 13169264 A
- WO 14036357 A
- WO 2012145493 A
- WO 2010077634 A
- WO 2007005874 A
- WO 201379174 A
- WO 1019570 A
- WO 1408218 A
- WO 08132601 A
- WO 00944273 A
- WO 1232433 A
- WO 006105021 A
- WO 009009116 A
- WO 11028683 A
- WO 0973620 A
- WO 0091156652 A
- WO 1156652 A
- WO 12142237 A
- WO 06029879 A
- WO 11109400 A
- US 8906682 B, June

### Non-patent literature cited in the description

- **ARNOUD et al.** *Clinical Cancer Research*, 2007
- **CUI JJ**. *J of Med Chem*, 2014
- **PEARSON et al.** *Cancer Discovery*, 2016
- **CHALLITA-EID et al.** *Cancer Research*, 2016
- **N PAVLOVA et al.** *Elife*, 2013
- **DRIGGERS et al.** *Nat Rev Drug Discov*, 2008, vol. 7 (7), 608-24
- **WU et al.** *Science*, 2007, vol. 330, 1066-71
- **XIONG et al.** *Science*, 2002, vol. 296 (5565), 151-5
- **ZHAO et al.** *J Struct Biol*, 2007, vol. 160 (1), 1-10
- **CHERNEY et al.** *J Med Chem*, 1998, vol. 41 (11), 1749-51
- **KEMP** ; **MCNAMARA**. *J. Org. Chem*, 1985
- **TIMMERMAN et al.** *ChemBioChem*, 2005
- **HEINIS et al.** *Angew Chem, Int Ed.*, 2014, vol. 53, 1602-1606
- **HEINIS et al.** *Nat Chem Biol*, 2009, vol. 5 (7), 502-7
- **NISHIWADA et al.** *Journal of Experimental & Clinical Cancer Research*, 2015, vol. 34, 30
- **CHENG et al.** *J Molecular Diagnostics*, 2015
- **ROTHWELL et al.** *Nature Medicine*, 2019
- **MILLER et al.** *JCO*, 2013
- **LANMAN et al.** *PLoS One*, 2015

- **S. M. BERGE et al.** *J. Pharmaceutical Sciences*, 1977, vol. 66, 1-19
- Pharmaceutical Salts: Properties, Selection, and Use. August 2002, 388
- **CONNOLLY et al.** *Int'l J. Biological Sciences*, 2012, vol. 8, 964-978
- **HELLMAN et al.** Principles of Radiation Therapy, Cancer, in Principles and Practice of Oncology. 1993, vol. 1, 248-275
- **OKAZAKI, T et al.** *Nat. Immunol.*, 2013, vol. 14, 1212-1218
- **ZOU et al.** *Sci. Transl. Med*, 2016, vol. 8
- **JERRY L. ADAMS et al.** Big opportunities for small molecules in immuno-oncology. *Cancer Therapy*, 2015, vol. 14, 603-622
- **PETER L. TOOGOOD**. Small molecule immuno-oncology therapeutic agents. *Bioorganic & Medicinal Chemistry Letters*, 2018, vol. 28, 319-329
- **SANDRA L. ROSS et al.** Bispecific T cell engager (BITE® ) antibody constructs can mediate bystander tumor cell killing. *PLoS ONE*, vol. 12 (8), e0183390